# EUROPEAN PATENT APPLICATION

(11) **EP 3 381 898 A1**
(43) Date of publication of application: **03.10.2018**
(21) Application number: 17163017.1
(22) Date of filing: 27.03.2017
(51) Int. Cl.: C07D 207/416, C07K 5/02, C07F 5/02, A61K 31/4025, A61K 31/69, A61P 35/00, A61P 17/06, A61P 19/02

(54) **SYMMETRICAL TRIS-ARYL-AMIDE DERIVATIVES AND THEIR USE AS ANTI-HEPARANASE**

(71) Applicant: Leadiant Biosciences SA, 6850 Mendrisio (CH)
(72) Inventor: GIANNINI, Giuseppe, 00071 Pomezia (ROMA) (IT); SIMONI, Daniele, 44123 Ferrara (IT); OLIVA, Paola, 72024 Oria (BR) (IT); MOR, Marco, 25016 Ghedi (BS) (IT); RIVARA, Silvia, 43015 Noceto (PR) (IT)
(74) Representative: Ghirardi, Valeria

(57) **Abstract**

The present invention relates to tris-aryl-amide derivatives having an anti-heparanase activity, in particular it relates to ureido/thioureido/ether tris-aryl-amide derivatives of formula

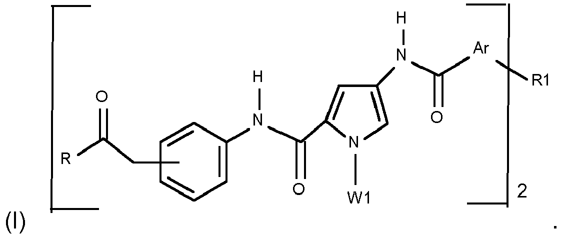

The invention also relates to the use of such compounds as a medicament, in particular for the treatment of diseases and disorders associated with heparanase activity, and to pharmaceutical compositions comprising the same.

## Description

### FIELD OF THE INVENTION

The present invention relates to tris-aryl-amide derivatives having an anti-heparanase activity, in particular it relates to ureido/thioureido/ether tris-aryl-amide derivatives.

The invention also relates to the use of such compounds as a medicament, in particular for the treatment of diseases and disorders associated with heparanase activity, and to pharmaceutical compositions comprising the same.

### BACKGROUND OF THE INVENTION

Heparan sulfate proteoglycans (HSPGs) are major components of the basement membrane and extracellular matrix. They are ubiquitous macromolecules associated with the cell surface and extracellular matrix (ECM) of a wide range of cells of vertebrate and invertebrate tissues (Bernfield et al. Annu. Rev. Biochem. 1999, 68, 729; lozzo R. V., San Antonio J. D. J. Clin. Invest. 2001, 108, 349; Kjellen L., Lindahl U. Annu. Rev. Biochem. 1991, 60, 443). HSPGs are a class of carbohydrate-modified proteins involved in key biological processes, including a role as co-receptors for a number of ligand molecules to regulate their signaling and distribution (Nakato H., Li J.P.- Int. Rev. Cell. Mol. Biol. 2016, 325, 275).

Heparan sulfate (HS) is a component of cell surface and matrix-associated proteoglycans (HSPGs). A key function of HS is to bind and interact with signaling proteins, growth factors, plasma proteins, immune-modulators and other factors. In doing so, the HS chains and HSPGs are able to regulate protein distribution, bio-availability and action on target cells. Moreover, the HS chains ensure that a wide variety of bioactive molecules bind to the cell surface and ECM and thereby function in the control of normal and pathological processes, among which are morphogenesis, tissue repair, inflammation, vascularization and cancer metastasis (Billings P.C., Pacifici M. Connect Tissue Res. 2015, 56(4), 272). HS glycosaminoglycans bind to and assemble a multitude of ECM proteins (i.e., laminin, fibronectin, collagen type IV) and thereby significantly contribute to the ECM self assembly and integrity.

Heparanase is an endoglycosidase, which cleaves heparan sulfate (HS) and hence participates in degradation and remodeling of the ECM, with a release of bioactive saccharide fragments and HS-bound growth factors and cytokines. Heparanase is preferentially expressed in human tumors and its over-expression in tumor cells confers an invasive phenotype in experimental animals. Heparanase upregulation correlates with increased tumor vascularity and poor postoperative survival of cancer patients. Heparanase is synthesized as a 65 kDa inactive precursor that undergoes proteolytic cleavage, yielding 8 kDa and 50 kDa protein subunits that heterodimerize to form an active enzyme.

Heparanase exhibits also non-enzymatic activities, independent of its involvement in ECM degradation.

There is growing evidence that heparanase upregulates expression of genes that participate in cancer metastasis and angiogenesis, glucose metabolism, immune response, inflammation and atherosclerosis, suggesting that heparanase belongs to an emerging class of proteins that play a significant role in regulating transcription in addition to their well-recognized extra-nuclear functions (Pisano C. et al. Biochem. Pharmacol. 2014, 89, 12; Ilan N. et al. Int. J. Biochem. Cell Biol. 2006, 38, 2018; Fux L. et al. Trends Biochem. Sci. 2009, 34, 511; Parish C.R.et al. Matrix Biol. 2013, 32, 228; Ramani V.C. et al. FEBS J. 2013, 280, 2294; Vlodavsky I. et al. Matrix Biol. 2013. 32, 241; Yang Y. et al. Cancer Res. 2010, 70, 8329).

In recent years, heparanase has attracted considerable attention as a promising target for innovative pharmacological applications. Indeed, heparanase appears to be involved in major human diseases, from tumors to chronic inflammation, diabetic nephropathy, bone osteolysis, thrombosis and atherosclerosis, in addition to more recent investigation in various rare diseases (Rivara S. et al. Future Med. Chem. 2016, 8, 647).

In view of the above, compounds able to specifically modulate the activity of this enzyme are highly desired as a useful pharmacological option for many therapeutic indications. Since heparanase is involved in a very wide range of molecular pathways, modulation of the expression or activity of this enzyme is a viable therapeutic option.

Potent and selective heparanase inhibitors are therefore highly searched as therapeutic tools for those clinical indications in which heparanase inhibition is pharmacologically useful.

However, although in the last twenty years numerous efforts have been made in this sense and huge developments have been achieved in the knowledge of heparanase activity and functions, so far no drug able to inhibit or modulate heparanase functions has yet been registered (Rivara S., et al. Future Med. Chem. 2016, 8, 647).

Some compounds are known in the art as heparanase inhibitors.

In particular, three classes of heparanase inhibitors are known: active analogous of endogenous substance, synthetic small molecule compounds and natural products. In the literature, monoclonal antibodies and nucleic acid-based inhibitors are also mentioned as possible heparanase inhibitors.

Among these, only a few heparin derivatives have so far reached the stage of clinical trial (see some review: Ferro V. Mini Rev. Med. Chem. 2004, 4, 693; Jia L., Ma S. Eur. J. Med. Chem. 2016, 121, 209; Rivara S. et al. Future Med. Chem. 2016, 8, 647).

However, the heparin-derivatives macromolecules currently under clinical investigation have a high molecular weight.

Small molecules, instead, are particularly desirable due to their better pharmacokinetic properties. Furthermore, they can be administered orally thus resulting in an improved patient compliance.

Indeed, progress in the development of drugs able to inhibit heparanase activity has been limited also by the lack of efficient small molecule inhibitors.

Therefore, there is still the need of small molecules able to inhibit heparanase activity.

Among synthetic small molecule compounds, some urea derivatives have been designed and their heparanase inhibition activity has been studied.

For example, in Pan et al. (Bioorganic Medicinal Chemistry Letters 2006 Jan 15;16(2):409-12, 1-[4-(1 H-Benzoimidazol-2-yl)-phenyl]-3-[4-(1H-benzoimidazol-2-yl)-phenyl]-urea derivatives as small molecule heparanase inhibitors. Pan W, Miao HQ, Xu YJ, Navarro EC, Tonra JR, Corcoran E, Lahiji A, Kussie P, Kiselyov AS, Wong WC, Liu H.), a class of 1-[4-(1H-benzoimidazol-2-yl)-phenyl]-3-[4-(1H-benzoimidazol-2-yl)-phenyl]-ureas is described and heparanase inhibitory activity of some compounds of this class is investigated. In Xu et al. (Bioorganic Medicinal Chemistry Letters 2006 Jan 15;16(2):404-8, N-(4-{[4-(1H-Benzoimidazol-2-yl)-arylamino]-methyl}-phenyl)-benzamide derivatives as small molecule heparanase inhibitors. Xu YJ, Miao HQ, Pan W, Navarro EC, Tonra JR, Mitelman S, Camara MM, Deevi DS, Kiselyov AS, Kussie P, Wong WC, Liu H) a class of N-(4-{[4-(1H-benzoimidazol-2-yl)-arylamino]-methyl}-phenyl)-benzamides is described and some compounds are tested as heparanase inhibitors.

In "3D QSAR based design of novel substituted urea molecules as heparanase inhibitors" (Raju Bathini, Sabiha Fatima, Sree Kanth Sivan, Vijjulatha Manga; Journal of Pharmacy Research Volume 7, Issue 8, August 2013, Pages 754-761) symmetric urea derivatives are designed and their activity on heparanase inhibition is predicted.

In Courtney et al., 2005 (Bioorg Med Chem Lett. 2005 May 2;15(9):2295-9, Furanyl-1,3-thiazol-2-yl and benzoxazol-5-yl acetic acid derivatives: novel classes of heparanase inhibitor, Courtney SM, Hay PA, Buck RT, Colville CS, Phillips DJ, Scopes DI, Pollard FC, Page MJ, Bennett JM, Hircock ML, McKenzie EA, Bhaman M, Felix R, Stubberfield CR, Turner PR), a series of furanyl-1,3-thiazol-2-yl and benzoxazol-5-yl acetic acids are synthetized and studied for their anti-heparanase activity.

In Courtney et al., 2004 (2,3-Dihydro-1,3-dioxo-1H-isoindole-5-carboxylic acid derivatives: a novel class of small molecule heparanase inhibitors. Courtney SM, Hay PA, Buck RT, Colville CS, Porter DW, Scopes DI, Pollard FC, Page MJ, Bennett JM, Hircock ML, McKenzie EA, Stubberfield CR, Turner PR. Bioorg Med Chem Lett. 2004 Jun 21;14(12):3269-73) a class of 2,3-dihydro-1,3-dioxo-1H-isoindole-5-carboxylic acids is disclosed as heparanase inhibitors.

Also, further compounds have been disclosed in the patent literature as small molecules heparanase inhibitors.

For example, WO03074516 discloses phthalimide carboxylic acid derivatives and their uses as heparanase inhibitors.

All the above mentioned documents show that a big effort has been put in the design of small molecules compounds able to efficiently inhibit heparanase and also endowed with good pharmacokinetic properties. It is therefore evident that such molecules are still highly desired.

### SUMMARY OF THE INVENTION

It has now been found a class of compounds able to inhibit heparanase activity.

It is an object of the present invention a compound having the following formula (I) 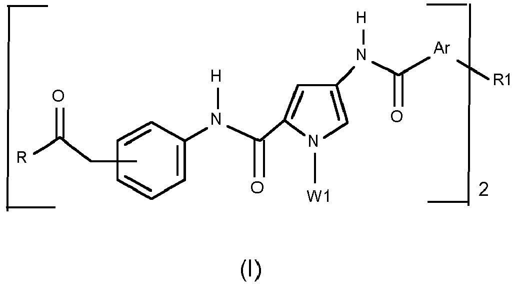
wherein R₁ is selected from the group consisting of NHCONH, O and NHCSNH;
Ar is selected from 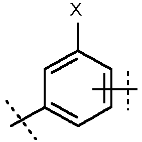 wherein X is selected from the group consisting of H, halogen and C₁-C₄alkyl, linear or branched, optionally substituted with one or more halogens,
   and 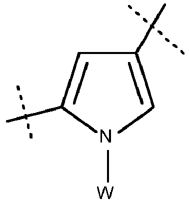 wherein W is selected from the group consisting of H, C₁-C₄ alkyl, linear or branched, optionally substituted with one or more halogens, and cyclopropyl,
W1 is selected from the group consisting of H, C₁-C₄ alkyl, linear or branched, optionally substituted with one or more halogens, and cyclopropyl,
and R is selected from the group consisting of OH, one or more amino acids, NHCH[B(OH)₂]C₁-C₄alkyl, linear or branched, and amino acid-NHCH[B(OH)₂]C₁-C₄alkyl, linear or branched,
the enantiomers, diastereoisomers, and mixtures thereof,
or a pharmaceutically acceptable salt, hydrate or solvate thereof.

Indeed, it has been found that such compounds are able to inhibit heparanase activity.

In the compound of formula (I) R₁ is preferably NHCONH.

In the compound of formula (I) R is preferably an amino acid, more preferably an amino acid selected from glycine and phenylalanine.

In a preferred embodiment of the invention R₁ is NHCONH and Ar is phenyl, optionally substituted with X. In this embodiment, R is preferably an amino acid, more preferably an amino acid selected from glycine and phenylalanine.

In another preferred embodiment of the invention R₁ is NHCONH and Ar is pyrrole, wherein W is preferably CH₃. In this embodiment, R is preferably OH or an amino acid, more preferably an amino acid selected from glycine and phenylalanine.

In another preferred embodiment of the invention R₁ is O and Ar is phenyl. In this embodiment, R is preferably an amino acid, more preferably an amino acid selected from glycine and phenylalanine.

In a preferred embodiment, the compound of formula (I) is in the form of a salt, preferably a sodium salt.

A process for the preparation of the compounds of formula (I), as will be better defined below, is also an object of the present invention.

It is also an object of the invention the compound of formula (I) for use as a medicament.

A further object of the present invention is a compound of formula (I) for the treatment of a disease which can be improved or prevented by an anti-heparanase activity.

In a preferred embodiment, said disease is selected from the group consisting of tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular, if glucose induced following peritoneal dialysis) and aging.

A further object of the invention is a pharmaceutical composition containing as active ingredient a compound of formula (I) and at least one pharmaceutically acceptable vehicle and/or excipient and its use as a medicament.

### Detailed description of the invention

### Definitions

Within the meaning of the present invention, the term "anti-heparanase activity" refers to an activity sufficient to inhibit partially or totally the enzyme heparanase.

Within the meaning of the present invention, the term "substituted by" or "substituted with" means that one or more hydrogen atoms of an hydrocarbon is/are replaced by an atom or a group of atoms (a substituent).

When a halogen is present, it is selected from the group consisting of: fluorine (F), chlorine (Cl), bromine (Br) and iodine (I). Preferably, it is fluorine.

When an amino acid is present it can be any amino acid. In particular, it can be a L or D amino acid, natural or not natural. The structure of any known amino acid is known in the art.

In the compound of formula (I) the amino acid, when present, is linked to the rest of the molecule through its amine group (NH₂), therefore being in the form -NH-CHR_{X}-COOH, wherein the Rₓ group represents the side group of an amino acid. The R_{X} group of any known natural or synthetic amino acid is commonly known in the field. For example, the Rₓ group of glycine is H, the Rₓ group of alanine is CH₃, the Rₓ group of valine is CH(CH₃)₂, and so on.

In the compound of formula (I) more than one amino acid, therefore an amino acid chain, can be present as R group. The amino acid chain, when present, is linked to the rest of the molecule through the amine group (NH₂) of the first amino acid and terminates with the OH group of the last amino acid. In a preferred embodiment, R is glycine-glycine.

When R is amino acid-NHCH[B(OH)₂]C₁-C₄alkyl, the amino acid is linked to the rest of the molecule through its amine group (-NH₂) and it is linked to the boronic group through its carboxyl group (-COOH), therefore being present in the final compound of formula (I) in the form -NH-CHRₓ-CO-, wherein the Rₓ group represents the side group of any natural or synthetic amino acid, as commonly known in the field.

In particular, the amino acid can be any amino acid selected from the group consisting of: alanine (Ala), arginine (Arg), asparagine (Asn), aspartic acid (Asp), cysteine (Cys), glutamic acid (Glu), glutamine (Gin), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr) and valine (Val).

Preferably, it is an amino acid selected from the group consisting of: glycine (Gly) and phenylalanine (Phe).

More preferably, it is glycine (Gly).

According to the present invention, a C₁-C₄alkyl group can be an alkyl with one, two, three or four carbon atoms. It can be linear or branched. For example, it can be a methyl, ethyl, n-propyl, i-propyl, n-butyl, t-butyl, isobutyl, sec-butyl. Preferably, it is methyl.

Optionally, X, W or W1 can be C₁-C₄alkyl substituted with one or more halogens. The halogen can be any of fluorine (F), chlorine (Cl), bromine (Br) and iodine (I), preferably it is fluorine (F). The C₁-C₄alkyl can be substituted, for example, with one, two or three halogens on any position of the alkyl chain; in a preferred embodiment, it is substituted with three halogens. In a preferred embodiment, it is methyl substituted with three halogens. In a more preferred embodiment, it is CF₃.

In an embodiment, W1 is preferably selected from the group consisting of CH₃, CF₃ and cyclopropyl, more preferably it is CH₃.

The group 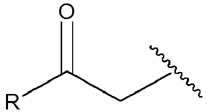 can be present on any position of the phenyl ring. Preferably it is in position 3 (meta) or in position 4 (para) of the phenyl ring. More preferably it is in position 3 (meta).

When Ar is a phenyl, the R₁ group is preferably linked to said phenyl in position 3 (meta) or 4 (para), as herein represented:

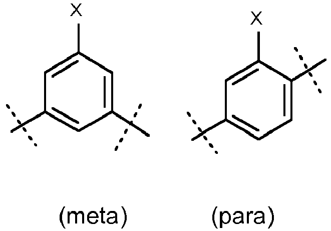

Preferably, it is in position 3 (meta).

In an embodiment, R is NHCH[B(OH)₂]C₁-C₄alkyl, linear or branched, or amino acid-NHCH[B(OH)₂]C₁-C₄alkyl. In this embodiment, C₁-C₄alkyl can be an alkyl with one, two, three or four carbon atoms, linear or branched. In a preferred embodiment, it is a C₄alkyl, preferably branched, more preferably it is isobutyl (CH₂CH(CH₃)₂). Therefore, in a preferred embodiment, R is NHCH[B(OH)₂]CH₂CH(CH₃)₂ or amino acid-NHCH[B(OH)₂]CH₂CH(CH₃)₂.

In an embodiment, X is a methyl substituted with one or more halogens; the halogen can be any of fluorine (F), chlorine (Cl), bromine (Br) and iodine (I). The methyl can be substituted with one, two or three halogens; in a preferred embodiment, it is substituted with three halogens. In a more preferred embodiment, X is CF₃.

In a preferred embodiment, X is selected from the group consisting of H, F and CF₃.

In an embodiment, R₁ is NHCONH, Ar is 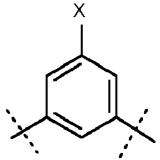 wherein X, W1 and R have the meanings above defined.

In this embodiment, X is preferably selected from H and CF₃, more preferably it is H.

In this embodiment, R₁ is preferably linked to the phenyl ring in position meta.

In this embodiment, R is preferably selected from OH and one or more amino acids, preferably it is an amino acid. Said amino acid can be any amino acid, for example it can be leucine, glycine, L-lysine, alanine, phenylalanine or L-glutammate. It can also be more than one amino acid, for example glycine-glycine. More preferably it is selected from Gly and Phe. Even more preferably it is Gly.

R can also be NHCH[B(OH)₂]C₁-C₄alkyl or amino acid-NHCH[B(OH)₂]C₁-C₄alkyl, wherein saia amino acid is preferably a glycine.

In this embodiment, W1 is preferably selected from the group consisting of CH₃, CF₃ and cyclopropyl, more preferably it is CH₃.

In another embodiment, R₁ is NHCONH, Ar is 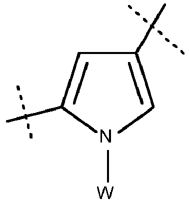 and R, W and W1 have the meanings above defined.

In this embodiment, W and W1 are preferably independently selected from the group consisting of CH₃, CF₃ and cyclopropyl, more preferably it is CH₃.

In this embodiment, R is preferably selected from OH and an amino acid, preferably it is an amino acid. Said amino acid can be any amino acid, for example it can be leucine, glycine, alanine, phenylalanine or L-glutammate. More preferably, it is an amino acid selected from Gly and Phe.

In another embodiment, R₁ is O and Ar is 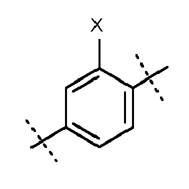 wherein X, W1 and R can have any of the meanings above defined.

In this embodiment, R₁ is preferably linked to the phenyl ring in position para.

In this embodiment, X is preferably H.

In this embodiment, W1 is preferably selected from the group consisting of CH₃, CF₃ and cyclopropyl, more preferably it is CH₃.

In this embodiment, R is preferably selected from OH and one or more amino acids, preferably it is an amino acid. Said amino acid can be any amino acid, for example it can be leucine, glycine, L-lysine, alanine, phenylalanine or L-glutammate. More preferably, it is Phe. It can also be more than one amino acid, for example glycine-glycine.

R can also be NHCH[B(OH)₂]C₁-C₄alkyl or amino acid-NHCH[B(OH)₂]C₁-C₄alkyl, wherein saia amino acid is preferably a glycine.

In another embodiment, R₁ is O, Ar is 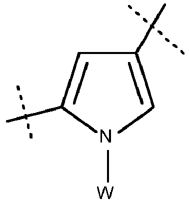 and R, W1 and W can have any of the meanings above defined.

In another embodiment, R₁ is NHCSNH and Ar is 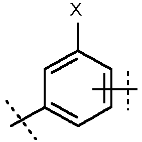 wherein X, W1 and R can have any of the meanings above defined.

In another embodiment, R₁ is NHCSNH, Ar is 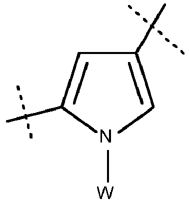 and R, W1 and W can have any of the meanings above defined.

Any nitrogen group can be protected. The protective group is preferably selected from tert-butoxycarbonyl, benzyloxycarbonyl and 9-fluorenylmethyloxycarbonyl.

When one or more chiral centers are present in the compounds of the present invention, the individual isomers, enantiomers and diastereoisomers, and mixtures thereof (e.g., racemates) are intended to be encompassed by the present invention.

The pharmaceutical acceptable salts of the compound of formula (I) are included in the scope of the invention.

Pharmaceutical acceptable salts are salts which retain the biological activity of the compound and are derived from known pharmacologically acceptable basis. Examples of pharmaceutically acceptable salts are, for example, those from compounds of formula (I) and inorganic bases, such as sodium, potassium, calcium and aluminum hydroxides, as well as those with organic bases such as, for example, lysine, arginine, N-methyl-glucamine, triethylaminetrimethylamine triethanolamine, dibenzyilamine, methylbenzyilamine, di-(2-ethylhexyl)-amine, piperidine, N-ethylpiperidine, N,N-di-ethylaminoethylamine, N-ethylmorpholine, 3-phenethylamine, N-benzyil-3-phenethylamine, N-benzyil-N,N-dimethylamine. A preferred salt is sodium salt. Pharmaceutical acceptable salts can also be those from compounds of formula (I) and known inorganic or organic acids, for example trifluoroacetate salt.

In some embodiments, the compound of the invention is preferably in the form of a sodium salt.

Also, pharmaceutically acceptable hydrates or solvates of the compounds of formula (I) are included in the scope of the present invention. In particular, they can be any hydrate or solvate commonly used in the art.

Pharmaceutically acceptable salts and their preparation are within the common knowledge of the skilled person and general reference is made to the relevant literature, such as, for example Pharmaceutical Salts and Co-crystals, Johan Wouters, Luc Quéré, Royal Society of Chemistry, 2011.

Preferred compounds according to the present invention are the followings:
disodium 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}diethanoate salt (SST0755NA1),
2,2'-((2,2'-(((4,4'-((3,3'-(Carbonylbis(azanediyl))bis(benzoyl))bis(azanediyl))bis(1-methyl-1*H-*pyrrole-4,2-diyl-2-carbonyl))bis(azanediyl))bis(3,1-phenylene))bis(acetyl))bis(azanediyl))diacetic acid (SST0755AA1),
disodium 2,2'-{carbonylbis[imino[5-(trifluoromethyl)-3,1-phenylene]carbonylimino(1-methyl-1H-pyrrole-4,2-diyl)carbonylimino-3,1-phenylene(1-oxoethane-2,1-diyl)imino]}diethanoate salt (SST0899NA1),
2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}bis(3-phenylpropanoic acid) (SST0881AA1),
2,2'-{carbonylbis[imino(1-methyl-1H-pyrrole-4,2-diyl)carbonylimino(1-methyl-1H-pyrrole-4,2-diyl)carbonylimino-3,1-phenylene]}diacetic acid (SST0760AA1),
disodium 2,2'-{carbonylbis[imino(1-methyl-1H-pyrrole-4,2-diyl)carbonylimino(1-methyl-1H-pyrrole-4,2-diyl)carbonylimino-3,1-phenylene(1-oxoethane-2,1-diyl)imino]}diethanoate salt (SST0820NA1),
2,2'-{carbonylbis[imino(1-methyl-1H-pyrrole-4,2-diyl)carbonylimino(1-methyl-1H-pyrrole-4,2-diyl)carbonylimino-3,1-phenylene(1-oxoethane-2,1-diyl)imino]}bis(3-phenylpropanoic acid) (SST0837AA1) and
(2S,2'S)-2,2'-((2,2'-(((4,4'-((4,4'-Oxybis(benzoyl))bis(azanediyl))bis(1-methyl-1H-pyrrole-2,2'-carbonyl))bis(azanediyl))bis(3,1-phenylene))bis(acetyl))bis(azanediyl))bis(3-phenylpropanoic acid) (SST0832AA1).

Further compounds according to the present invention are the followings:
2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}bis(4-methylpentanoic acid) (SST0884AA1)
5,5'-{carbonylbis[imino-3,1-phenylenecarbonylimino(1-methyl-1H-pyrrole-4,2-diyl)carbonylimino-3,1-phenylene(1-oxoethane-2,1-diyl)imino]}bis(1-aminium-6-hexanoic acid) trifluoroacetate salt (SST0900TF1)
2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}dipropanoic acid (SST0883AA1)
2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}dipentandioic acid (SST0882AA1)
Disodium 2,2'-{carbonylbis[imino-3,1-phenylenecarbonylimino(1-methyl-1H-pyrrole-4,2-diyl)carbonylimino-3,1-phenylene(1-oxoethane-2,1-diyl)imino(1-oxoethane-2,1-diyl)imino]}diethanoate salt (SST0898NA1)
2,2'-{carbonylbis[imino-3,1-phenylenecarbonylimino(1-methyl-1H-pyrrole-4,2-diyl)carbonylimino-4,1-phenylene]}diacetic acid (SST0756AA1)
2,2'-{carbonylbis[imino(1-methyl-1H-pyrrole-4,2-diyl)carbonylimino(1-methyl-1H-pyrrole-4,2-diyl)carbonylimino-3,1-phenylene(1-oxoethane-2,1-diyl)imino]}bis(4-methylpentanoic acid) (SST0936AA1)
2,2'-{carbonylbis[imino(1-methyl-1H-pyrrole-4,2-diyl)carbonylimino(1-methyl-1H-pyrrole-4,2-diyl)carbonylimino-3,1-phenylene(1-oxoethane-2,1-diyl)imino]}dipropanoic acid (SST0886AA1)
2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}dipentandioic acid (SST0885AA1)
2,2'-(((4,4'-((3,3'-(Carbonylbis(azanediyl))bis(benzoyl))bis(azanediyl))bis(1-methyl-1H-pyrrole-4,2-diyl-2-carbonyl))bis(azanediyl))bis(3,1-phenylene))diacetic acid (SST0754AA1)
(2S,2'S)-2,2'-((2,2'-(((4,4'-((4,4'-(Carbonylbis(azanediyl))bis(1-methyl-1H-pyrrole-4,2-diyl-2-carbonyl))bis(azanediyl))bis(1-methyl-1H-pyrrole-4,2-diyl-2-carbonyl))bis(azanediyl))bis(3,1-phenylene))bis(acetyl))bis(azanediyl))bis(3-phenylpropanoic acid) (SST0837AA1)
(2S,2'S)-2,2'-((2,2'-(((4,4'-((4,4'-(Carbonylbis(azanediyl))bis(1-methyl-1H-pyrrole-4,2-diyl-2-carbonyl))bis(azanediyl))bis(1-methyl-1H-pyrrole-4,2-diyl-2-carbonyl))bis(azanediyl))bis(3,1-phenylene))bis(acetyl))bis(azanediyl))bis(5-(tert-butoxy)-5-oxopentanoic acid) (SST0885AA1)
(2S,2'S)-2,2'-((2,2'-(((4,4'-((4,4'-(carbonylbis(azanediyl))bis(1-methyl-1H-pyrrole-4,2-diyl-2-carbonyl))bis(azanediyl))bis(1-methyl-1H-pyrrole-4,2-diyl-2-carbonyl))bis(azanediyl))bis(3,1-phenylene))bis(acetyl))bis(azanediyl))bis(6-aminohexanoic acid) dihydrochloride (SST0902AA1)
2,2'-((2,2'-(((4,4'-((4,4'-Oxybis(benzoyl))bis(azanediyl))bis(1-methyl-1H-pyrrole-2,2'-carbonyl))bis(azanediyl))bis(3,1-phenylene))bis(acetyl))bis(azanediyl))diacetic acid (SST0907AA1)
(2S,2'S)-2,2'-((2,2'-(((4,4'-((4,4'-Oxybis(benzoyl))bis(azanediyl))bis(1-methyl-1H-pyrrole-2,2'-carbonyl))bis(azanediyl))bis(3,1-phenylene))bis(acetyl))bis(azanediyl))dipropanoic acid (SST0830AA1)
(2S,2'S)-2,2'-((2,2'-(((4,4'-((4,4'-Oxybis(benzoyl))bis(azanediyl))bis(1-methyl-1H-pyrrole-2,2'-carbonyl))bis(azanediyl))bis(3,1-phenylene))bis(acetyl))bis(azanediyl))bis(4-methylpentanoic acid) (SST0831AA1)
(2S,2'S)-2,2'-((2,2'-(((4,4'-((4,4'-Oxybis(benzoyl))bis(azanediyl))bis(1-methyl-1H-pyrrole-2,2'-carbonyl))bis(azanediyl))bis(3,1-phenylene))bis(acetyl))bis(azanediyl))dipentanedioic acid (SST0833AA1)
(2S,2'S)-2,2'-((2,2'-(((4,4'-((4,4'-oxybis(benzoyl))bis(azanediyl))bis(1-methyl-1H-pyrrole-4,2-diyl-2-carbonyl))bis(azanediyl))bis(3,1-phenylene))bis(acetyl))bis(azanediyl))bis(6-aminohexanoic acid) dihydrochloride (SST0834AA1)
((1R,1'R)-((2,2'-(((4,4'-((4,4'-(carbonylbis(azanediyl))bis(1-methyl-1H-pyrrole-4,2-diyl-2-carbonyl))bis(azanediyl))bis(1-methyl-1H-pyrrole-4,2-diyl-2-carbonyl))bis(azanediyl))bis(3,1-phenylene))bis(acetyl))bis(azanediyl))bis(3-methylbutane-1,1-diyl))diboronic acid (SST0905AA1)
((1R,1'R)-((2,2'-(((4,4'-((4,4'-Oxybis(benzoyl))bis(azanediyl))bis(1-methyl-1H-pyrrole-2,2'-carbonyl))bis(azanediyl))bis(3,1-phenylene))bis(acetyl))bis(azanediyl))bis(3-methylbutane-1,1-diyl))diboronic acid (SST0838AA1)
((1R,1'R)-((2,2'-(((4,4'-((3,3'-(Carbonylbis(azanediyl))bis(benzoyl))bis(azanediyl))bis(1-methyl-1 H-pyrrole-4,2-diyl-2-carbonyl))bis(azanediyl))bis(3,1-phenylene))bis(acetyl))bis(azanediyl))bis(3-methylbutane-1,1-diyl))diboronic acid (SST0828AA1)
((1R,1'R)-((2,2'-((2,2'-(((4,4'-((4,4'-(carbonylbis(azanediyl))bis(1-methyl-1H-pyrrole-4,2-diyl-2-carbonyl))bis(azanediyl))bis(1-methyl-1H-pyrrole-4,2-diyl-2-carbonyl))bis(azanediyl))bis(3,1-phenylene))bis(acetyl))bis(azanediyl))bis(acetyl))bis(azanediyl))bis(3-methylbutane-1,1-diyl))diboronic acid (SST0906AA1)
((1R,1'R)-((2,2'-((2,2'-(((4,4'-((3,3'-(Carbonylbis(azanediyl))bis(benzoyl))bis(azanediyl))bis(1-methyl-1 H-pyrrole-4,2-diyl-2-carbonyl))bis(azanediyl))bis(3,1-phenylene))bis(acetyl))bis(azanediyl))bis(acetyl))bis(azanediyl))bis(3-methylbutane-1,1-diyl))diboronic acid (SST0829AA1),
2,2'-((2,2'-(((4,4'-((4,4'-oxybis(benzoyl))bis(azanediyl))bis(1-methyl-1H-pyrrole-2,2'-carbonyl))bis(azanediyl))bis(3,1-phenylene))bis(acetyl))bis(azanediyl))diacetic acid (49b) and 2,2'-((2,2'-(((4,4'-((3,3'-(thiocarbonylbis(azanediyl))bis(benzoyl))bis(azanediyl))bis(1-methyl-1 H-pyrrole-2,2'-carbonyl))bis(azanediyl))bis(3,1-phenylene))bis(acetyl))bis(azanediyl))diacetic acid (52b).

A process for the preparation of the compounds of formula (I) is also within the scope of the present invention.

The compounds of the present invention can be synthesized by many methods available to those skilled in the art of organic chemistry. General and exemplary synthetic schemes for preparing compounds of the present invention are described below. These schemes are illustrative and are not meant to limit the possible techniques one skilled in the art may use to prepare the compounds disclosed herein. Different methods to prepare the compounds of the present invention will be evident to those skilled in the art. Additionally, the various steps in the synthesis may be performed in an alternate sequence in order to give the desired compound or compounds.

Examples of compounds of the present invention prepared according to methods described in the general schemes are given in the intermediates and examples section set out hereinafter.

The compounds of the present invention can be synthesized using the methods described below, together with synthetic methods known in the art of synthetic organic chemistry, or by variations thereon as appreciated by those skilled in the art. Preferred methods include, but are not limited to, those described below. The reactions are performed in a solvent or solvent mixture appropriate to the reagents and materials employed and suitable for the transformations being effected. It will be understood by those skilled in the art of organic synthesis that the functionality present on the molecule should be consistent with the transformations proposed.

It will also be recognized that another major consideration in the planning of any synthetic route in this field is the judicious choice of the protecting groups used for protection of the reactive functional groups present in the compounds described in this invention. An authoritative account describing the many alternatives to the trained practitioner is Greene et al. (Protective Groups in Organic Synthesis, 4th Edition, Wiley- Interscience (2006)).

Also, the skilled in the art can easily alter the reagents and reaction conditions exemplified in the schemes below to include any combination of substituents as defined above. Also, the skilled artisan can easily use interchangeable steps for each synthetic process and incorporate isolation and/or purification steps as deemed necessary.

Starting materials and intermediates useful for preparing compounds of the invention are commercially available or can be prepared by well known synthetic procedures.

The final products obtained by the synthesis described below may be purified using techniques commonly known to one skilled in the art such as preparatory chromatography, thin-layer chromatography, HPLC, or crystallization.

Exemplary processes for the synthesis of the compounds of the invention are herein described.

### Synthesis of diphenyl urea

An exemplary process for obtainment of compounds of formula (I) wherein R₁ is NHCONH and Ar is 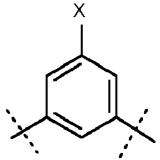 wherein X and W1 have the meanings above defined and R is OH or an amino acid is herein disclosed.

An exemplary process wherein R is an amino acid is represented in the following Scheme 1.

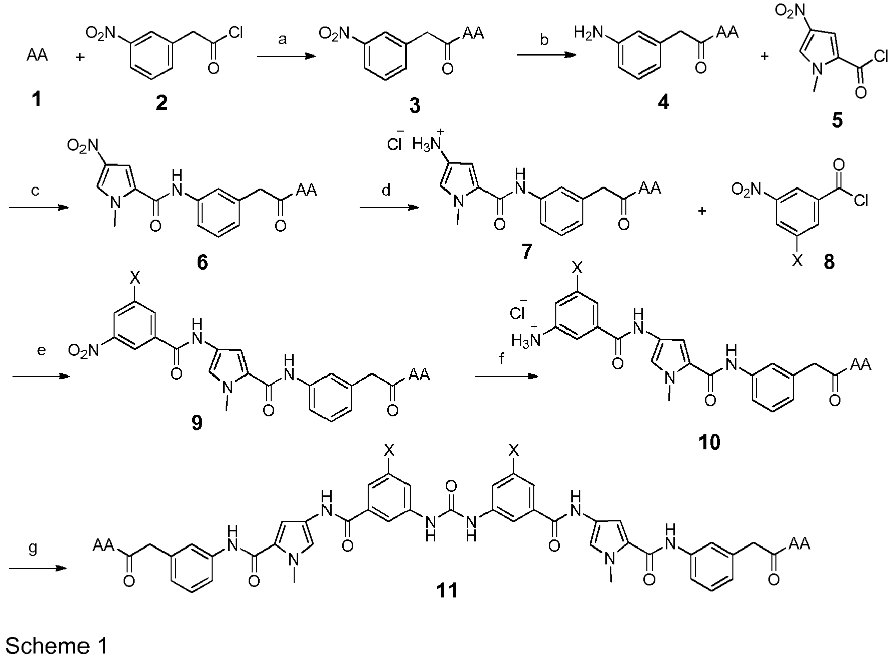

In the first step (a), condensation of commercially available amino acid 1 with commercially available 2-(3-nitrophenyl)acetyl chloride 2 is carried out, to afford the intermediate 3, wherein the carbonyl group acyl chloride of 2 reacts with the amine group of the amino acid (AA) with consequent formation of the amide function. The acid function of the amino acid is optionally protected, for example in the form of an alkyl ester. The nitro derivative **3** is then transformed into the corresponding amine analogue **4** by catalytic hydrogenation (b) and subsequently condensed with commercially available compound **5** affording (c) the nitro compound **6**. The latter nitro compound is then reduced (d) to compound **7** by catalytic hydrogenation and the resulting amino derivate coupled with the suitable 3-nitrobenzoyl chloride **8** to afford (e) the nitro derivate **9**. Nitro compound **9** is in turn reduced by catalytic hydrogenation (f) to furnish the amino analogue **10**. A typical reaction of formation of symmetrical ureas is then carried out (g), for example using carbonyldiimidazole (CDI), to synthesize the ureidic compound **11**. Finally, compounds having the acid function of AA protected as alkyl ester are hydrolysed, for example with LiOH in tetrahydrofuran (THF), while optional protective groups of the amino and carboxylic acid moieties of the AA, for example Boc and t-butyl functions, are removed with suitable agents, for example trifluoroacetic acid (TFA).

Optionally, when a salt form of the compound is desired, the obtained compounds can be treated with stoichiometric amount of a base, for example NaOH, or of an acid, for example trifluoroacetic acid.

When R is OH, the same procedure above described can be used with the difference that commercially available ethyl 2-(3-nitrophenyl)acetate is used as starting material instead of compound **3**.

Compounds wherein the 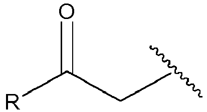 group is in position para can be obtained with the same procedure above descrived with the difference that commercially available 2-(3-nitrophenyl)acetyl chloride is used as starting material instead of compound **2** when R is an amino acid or ethyl 2-(3-nitrophenyl)acetate is used as starting material instead of compound **3** when R is OH.

Compound 3-nitrobenzoyl chloride (8) wherein X can have any of the meanings above defined is commercially available or can be easily obtained from commercially available compounds by well known procedures. For example, 3-nitrobenzoyl chloride (X=H) and 3-nitro-5-(trifluoromethyl)benzoyl chloride (X=CF₃) are commercially available.

Catalytic hydrogenation can be carried out according to the common knowledge and practice in the field, for example using palladium on carbon (Pd/C) as a catalyst.

Suitable solvents and reagents, such as triethylamine (TEA), dimethylformamide (DMF) and dichloromethane can be used throughout the process according to the general knowledge in the field.

The procedure above described is only an exemplary procedure to obtain diphenyl ureas according to the present invention. The skilled person can easily modify the procedure, for example the reagents or the reaction conditions, according to the specific compound to be obtained using the general knowledge in the field.

### Synthesis of dipyrrole ureas

An exemplary process for obtainment of compounds of formula (I) wherein R₁ is NHCONH, W1 can have any of the meanings above defined, Ar is 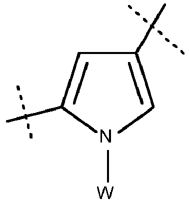 and R is OH or an amino acid, is herein disclosed.

An exemplary process is represented in the following Scheme 2.

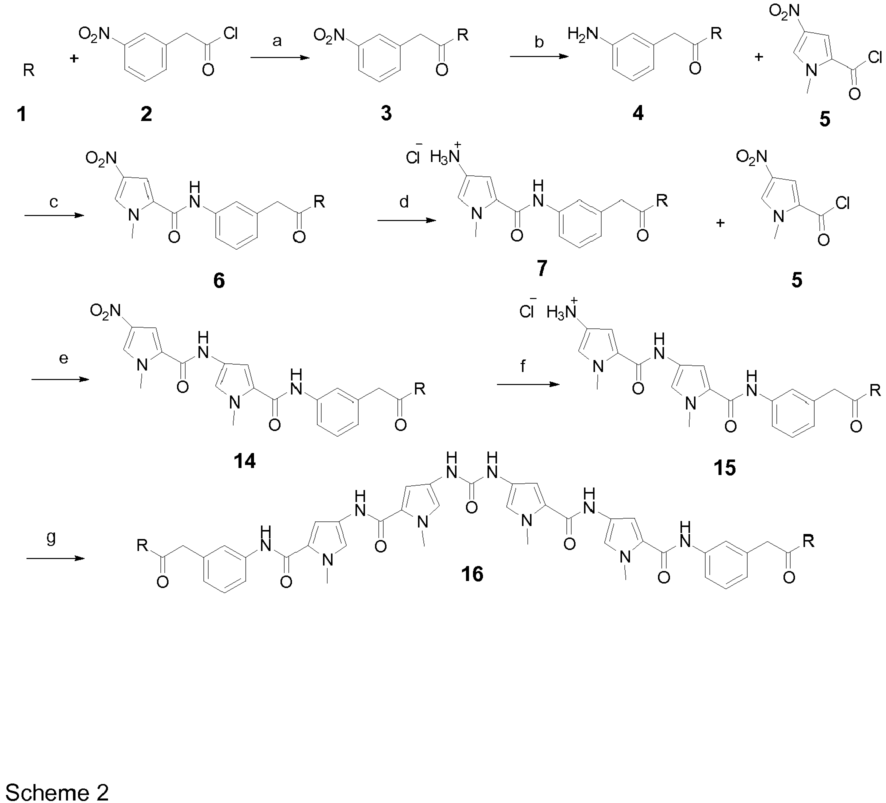

In a first step (a), condensation of commercially available aminoacid **1** with commercially available 2-(3-nitrophenyl)acetyl chloride **2** is carried out, to afford the intermediate **3**, wherein the carbonyl group acyl chloride of 2 reacts with the amine group of the amino acid (R) with consequent formation of the amide function. The acid function of the amino acid is optionally protected, for example in the form of an alkyl ester. The latter nitro derivative **3** is transformed (b) into the corresponding amine analogue **4** by catalytic hydrogenation; the latter amine is condensed (c) with commercially available 1-methyl-4-nitro-1H-pyrrole-2-carbonyl chloride **5** affording the nitro derivate **6** in turn reduced to **7** by catalytic hydrogenation (d); the resulting amino derivate **7** is coupled with 1-methyl-4-nitro-1 H-pyrrole-2-carbonyl chloride **5** to afford (e) the nitro compound **14** subsequently reduced by catalytic hydrogenation (f) to furnish the amino analogue **15**. A typical reaction of formation of symmetrical ureas is then carried out (g), for example with CDI, allowing to obtain the desired ureidic compound **16**.

Finally, compounds having the acid function of the amino acid protected as alkyl ester are hydrolysed, for example with LiOH in THF, while optional protective groups of the amino and carboxylic acid moieties of the amino acid, for example Boc and t-butyl functions, if present, are removed with suitable agents, for example TFA.

Optionally, when a salt form of the compound is desired, final compounds can be treated with stoichiometric amount of a base, for example NaOH, or of an acid, for example trifluoroacetic acid.

When R is OH, the same procedure above described can be used with the difference that ethyl 2-(3-nitrophenyl)acetate is used as starting material instead of compound **3**.

Catalytic hydrogenation can be carried out according to the common knowledge and practice in the field, for example using palladium on carbon (Pd/C) as a catalyst.

Suitable solvents and reagents, such as triethylamine (TEA), dimethylformamide (DMF) and dichloromethane can be used throughout the process according to the general knowledge in the field.

The procedure above described is only an exemplary procedure to obtain dipyrrole ureas according to the present invention. The skilled person can easily modify the procedure according to the specific compound to be obtained using the general knowledge in the field. For example compounds wherein W or W1 are different from CH₃ can be easily obtained based on the above process using a properly modified compound 5 according to the general knowledge in the field.

### Synthesis of diphenyl ethers

An exemplary procedure for the obtainment of compounds of formula (I) wherein R₁ is O, Ar is 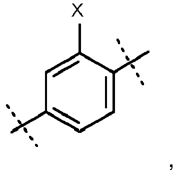 wherein X has any of the meanings above defined, and R is OH or an amino acid, is herein described.

Exemplary procedures are represented in the following Schemes 3 and 4.

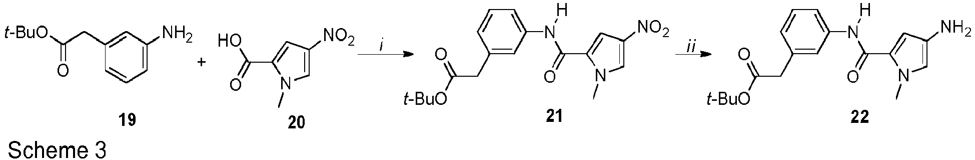

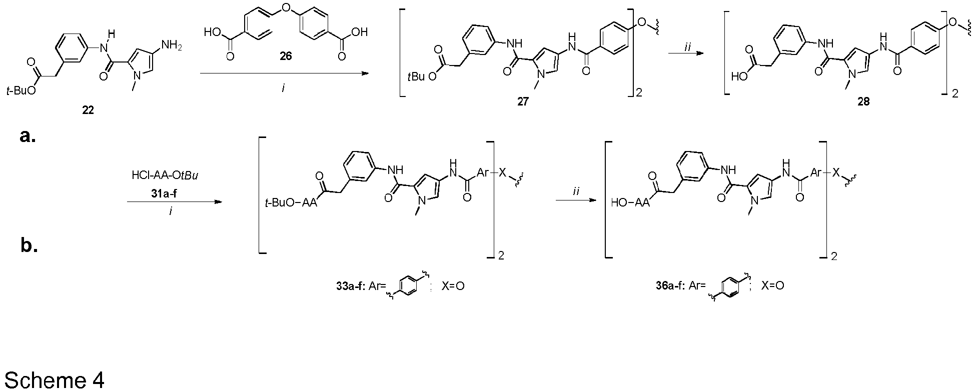

As represented in Scheme 3, 3-aminopyrrole intermediate 22 is obtained by condensation (i) of commercially available tert-butyl 2-(3-aminophenyl)acetate (19) with 1-methyl-4-nitropyrrole-2-carboxylic acid (20) (Ong, C. W.; Yang, Y.-T.; Liu, M.-C.; Fox, K. R.; Liu, P. H.; Tung, H.-W. Org. Biomol. Chem. 2012, 10, 1040-1046) and subsequent hydrogenation of the nitro group (ii).

As represented in Scheme 4a, compound **28** (R=OH) is obtained by coupling reactions between the commercially available simmetrical dicarboxylic acid **26** and the 3-aminopyrrole intermediate **22** above mentioned (i), and subsequent tert-butyl ester deprotection (ii), for example with HCl in dioxane.

When R is an amino acid, coupling reactions between the suitable tert-butyl ester aminoacid (**31a-f**), commercially available, with properly activated dicarboxylic acid **28** (i), followed by tert-butyl ester deprotection (ii) give the desired target compound **36a-f** (Scheme 4b).

Although the phenyl (Ar) is not substituted (X=H) in the schemes above, one of ordinary skill in the art can easily obtain compounds according to the present invention wherein X has any of the meanings above defined by choosing the suitable compound **26** in step (i) of scheme 4a above and according to the general knowledge in the field.

Deprotection can be carried out according to the general knowledge in the field.

### Alternative approach for synthesis of diphenyl ureas

Another exemplary process for obtainment of compounds of formula (I) wherein R₁ is NHCONH and Ar is 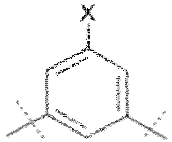 wherein X has the meanings above defined and R is OH or an amino acid, is represented in the following Scheme 5.

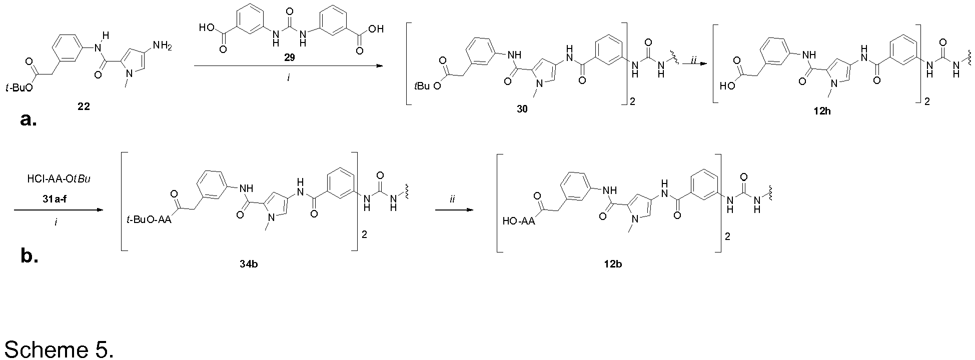

3-aminopyrrole intermediate **22** is obtained as above described (scheme 3).

Compound **12h** (R=OH) is obtained by coupling reactions between the corresponding symmetrical dicarboxylic acid **29** (Drewe, W. C.; Nanjunda, R.; Gunaratnam, M.; Beltran, M.; Parkinson, G. N.; Reszka, A. P.; Wilson, W. D.; Neidle, S. J. Med. Chem. 2008, 51, 7751-7767) and the 3-aminopyrrole intermediate **22** (i) and subsequent tert-butyl ester deprotection (ii) (Scheme 5a above).

When R is an amino acid, coupling reactions between the suitable tert-butyl ester amino acids (**31a-f**) with properly activated dicarboxylic acid **12h** (i), followed by tert-butyl ester deprotection (ii) give the desired target compound 12b (scheme 5b above).

Although the phenyl (Ar) is not substituted (X=H) in the schemes above, one of ordinary skill in the art can easily obtain compounds according to the present invention wherein X has any of the meanings above defined by choosing the suitable compound **29** in step (i) of scheme 5a and according to the general knowledge in the field.

Deprotection can be carried out according to the general knowledge in the field.

### Alternative synthesis of dipyrrole ureas

An alternative exemplary process for obtainment of compounds of formula (I) wherein R₁ is NHCONH, Ar is 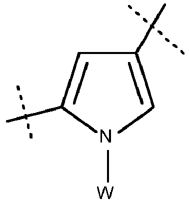 and R is OH or an amino acid, is represented in the following Schemes 6 and 7.

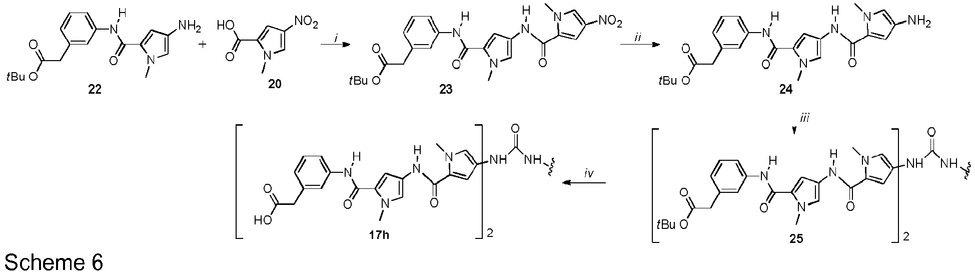

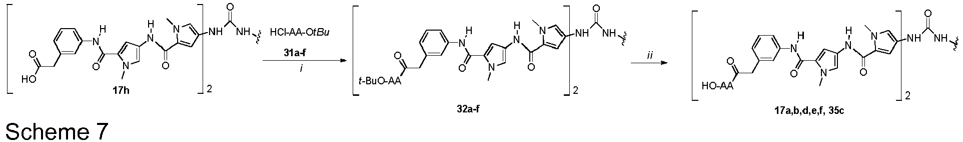

As represented in the above scheme 6, coupling of 3-aminopyrrole intermediate **22** with 1-methyl-4-nitropyrrole-2-carboxylic acid (**20**) (i), preferably in the presence of 1-ethyl-3-(3-dimethylaminopropyl)carbodiimide hydrochloride (EDC·HCl), hydroxybenzotriazole (HOBt), and triethylamine (TEA), gives the 3-nitropyrrole derivative **23** that is reduced (ii) to the corresponding amine **24** by catalytic hydrogenation. Condensation of two equivalents of the 3-amino derivative **24** with carbonyldiimidazole (iii), followed by tert-butyl ester deprotection (iv), for example with HCl in dioxane, gives the dicarboxylic acid 17h (R = OH).

When R is an amino acid, coupling reactions between the suitable tert-butyl ester aminoacids (**31a-f**) with properly activated dicarboxylic acid **17h** (i), followed by tert-butyl ester deprotection (ii) give the desired target compounds **17a-f** (scheme 7).

Deprotection can be carried out according to the general knowledge in the field.

Catalytic hydrogenation can be carried out according to the common knowledge and practice in the field, for example using palladium on carbon (Pd/C) as a catalyst.

Suitable solvents and reagents, such as triethylamine (TEA), dimethylformamide (DMF) and dichloromethane can be used throughout the process according to the general knowledge in the field.

### Synthesis of dipyrrole ethers

An exemplary procedure for the obtainment of compounds of formula (I) wherein R₁ is O, Ar is 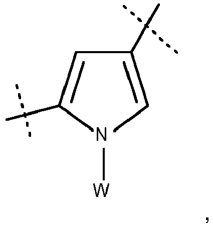 wherein X has any of the meanings above defined, and R is OH or an amino acid, is herein disclosed.

An exemplary process is represented in the following Scheme 8

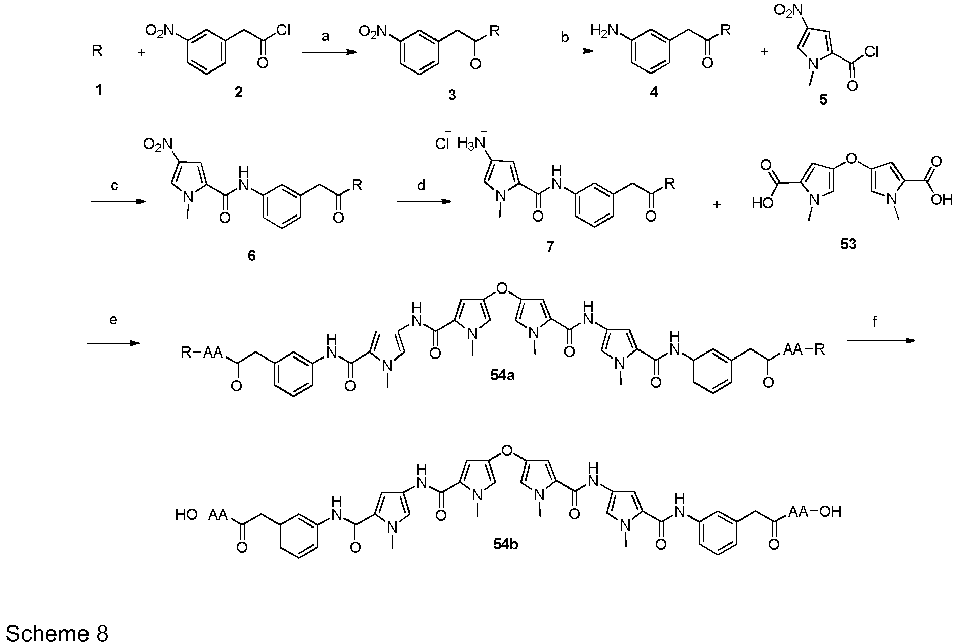

In the first step (a), condensation of commercially available amino acid **1** with commercially available 2-(3-nitrophenyl)acetyl chloride **2** is carried out, to afford the intermediate **3**, wherein the carbonyl group acyl chloride of 2 reacts with the amine group of the amino acid (AA) with consequent formation of the amide function. The acid function of the amino acid is optionally protected, for example in the form of an alkyl ester. The nitro derivative **3** is then transformed into the corresponding amine analogue **4** by catalytic hydrogenation (b) and subsequently condensed with commercially available compound **5** affording (c) the nitro compound **6**. The latter nitro compound is then reduced (d) to compound **7** by catalytic hydrogenation and the resulting amino derivate coupled with 4,4'-oxybis(1-methyl-1 H-pyrrole-2-carboxylic acid) **53** to afford (e) the compound **54a**. Compound 53 can be obtained by Williamson ether synthesis between commercially availables methyl 4-hydroxy-1-methyl-1 H-pyrrole-2-carboxylate and methyl 4-bromo-1-methyl-1 H-pyrrole-2-carboxylate and subsequent saponification of dimethyl 4,4'-oxybis(1-methyl-1 H-pyrrole-2-carboxylate), for example with LiOH in a mixture of methanol and water. Finally, compounds having the acid function of AA protected as alkyl ester are hydrolysed (f), for example with LiOH in tetrahydrofuran (THF), while optional protective groups of the amino and carboxylic acid moieties of the AA, for example Boc and t-butyl functions, are removed with suitable agents, for example trifluoroacetic acid (TFA).

Deprotection can be carried out according to the general knowledge in the field.

Catalytic hydrogenation can be carried out according to the common knowledge and practice in the field, for example using palladium on carbon (Pd/C) as a catalyst.

Suitable solvents and reagents, such as triethylamine (TEA), dimethylformamide (DMF) and dichloromethane can be used throughout the process according to the general knowledge in the field.

### Synthesis of boronic derivatives

An exemplary process for the obtainment of compounds of formula (I) wherein R is NHCH[B(OH)₂]C₁-C₄alkyl or aminoacid-NHCH[B(OH)₂]C₁-C₄alkyl is herein disclosed.

An exemplary process is represented in the following schemes 9 and 10.

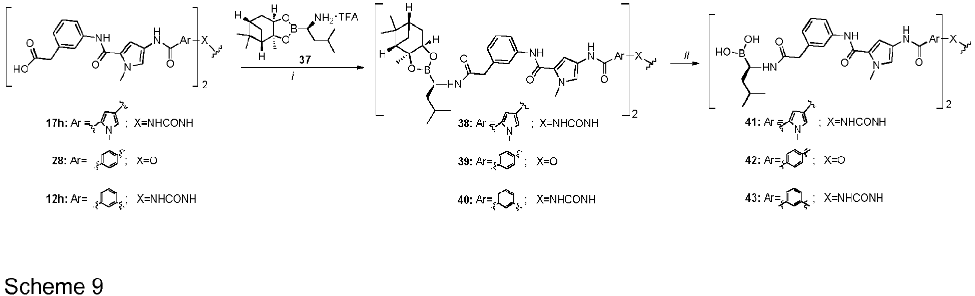

The intermediates **17h**, **28** and **12h** obtained with the processes above described can be used to obtain boronic derivatives of dipyrrole ureas, diphenyl ethers and diphenyl ureas, respectively.

As represented in the above scheme 9, said intermediates are coupled (i) with commercially available pinanediol leucine boronate (**37**) to provide the pinanediol ester derivatives **38-40**. Then, the pinanediol intermediates undergo a trans-esterification reaction (ii) with phenylboronic acid under acidic conditions, to afford the desired boronic acid derivatives **41-43**.

In a similar way amino acid boronates can be prepared, as represented in scheme 10 below.

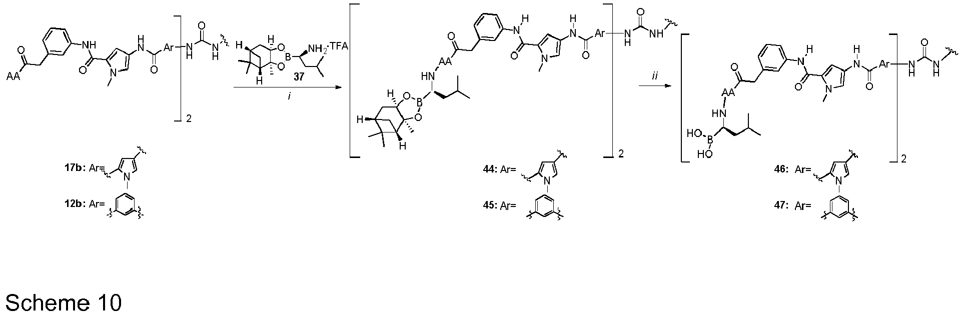

The desired amino acid derivative **17b** or **12b** of the above mentioned intermediates **17h** and **12h**, which can be obtained as shown above, see for example scheme 7, is coupled (i) with pinanediol leucine boronate (**37**) to provide the pinanediol ester derivatives **44-45**. Then, the pinanediol intermediates undergo a trans-esterification reaction (ii) with phenylboronic acid under acidic conditions, to afford the desired boronic acid derivatives **46-47**.

In schemes 9 and 10 above, R is NHCH[B(OH)₂]CH₂CH(CH₃)₂ or amino acid-NHCH[B(OH)₂]CH₂CH(CH₃)₂. However, the same procedures can be applied with different lengths or ramifications of the terminal alkyl chain by coupling the starting compound with the suitable boronate compound, according to the general knowledge in the field.

The same or similar procedures can be applied for obtaining boronic or amino acid boronic derivatives of compounds according to formula (I) with different meanings of R₁, R, W and X. In particular, the same procedures, with minor modifications, can be used to obtain boronic derivatives wherein R₁ is NHCSNH. Minor modifications required to the process in order to obtain the desired compounds are well within the knowledge and the abilities of the skilled person.

All the transformations above described are only exemplary synthetic procedures already known in organic chemistry and well known to the experts in the field

### Synthesis of diphenyl thioureas

An exemplary procedure for the obtainment of compounds of formula (I) wherein R₁ is NHCSNH, Ar is 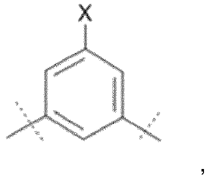 and X and R have any of the meanings above defined is represented in the following Scheme 11 and described below.

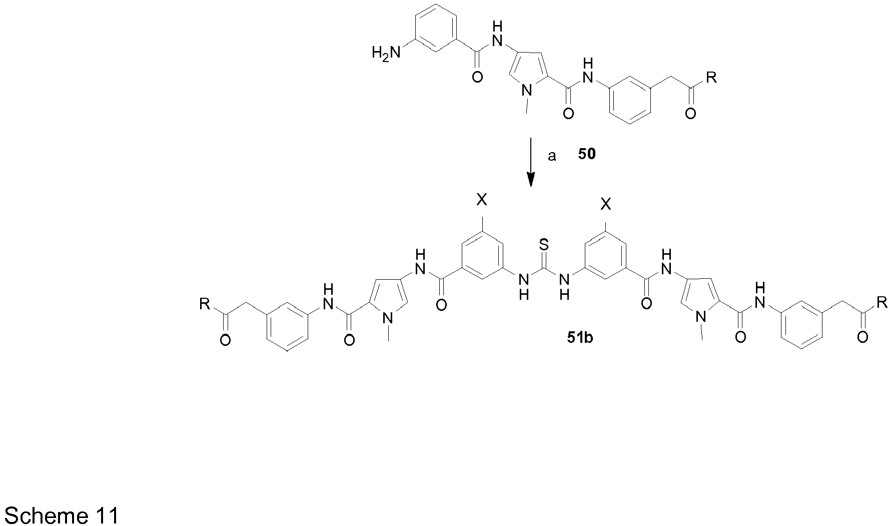

To compound **50**, which can be obtained according to procedures disclosed above, see for example scheme 1, for example in solution with CH₂Cl₂, commercial N,N'-Thiocarbonyldiimidazole is added to obtain compound **51b**. The reaction can be carried out, for example, at 40°C for 12 hours.

When R is an amino acid, compounds having the acid function of the amino acid protected as alkyl ester are subsequently hydrolysed, for example with LiOH in THF, while optional protective groups of the amino and carboxylic acid moieties of the amino acid, for example Boc and t-butyl functions, if present, are removed with suitable agents, for example TFA.

When R is a boronic derivative, the procedures described above can be applied by using the suitable starting compound.

### Synthesis of dipyrrole thioureas

Compounds of formula (I) wherein R₁ is NHCSNH, Ar is 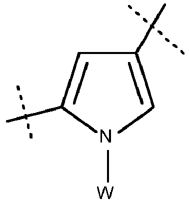 and R is OH or an amino acid, can be prepared following the same procedure reported above for the synthesis of dipyrrole ureas and using a suitable condensing agent, for example commercial N,N'-Thiocarbonyldiimidazole.

In the above schemes, the group 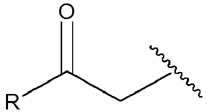 is always shown in position 3 of the phenyl ring. However, compounds of formula (I) wherein such group is on different positions of the phenyl ring can be easily obtained by choosing the suitable starting compounds, as exemplified in the description of scheme 1 above.

All the transformations above described are only exemplary synthetic procedures already known in organic chemistry and well known to the experts in the field.

Any variation or combination of substitutents of compounds of formula (I) which is within the scope of the present invention and is not explicitly shown or described in the above processes, can be easily obtained starting from the exemplary processes described above with suitable modifications, for example in the starting compounds or reagents, that are well within the knowledge of the skilled person in the field of organic chemistry. Reference can be made, for example, to the book "March's Advanced Organic Chemistry, Reactions, Mechanism and structures.", Michael B. Smith, Jerry March, last edition.

### Medical uses

According to the present invention, the compounds of formula (I) can be used as medicaments.

In particular, according to the present invention, the compounds of formula (I) are endowed with anti-heparanase activity. Therefore, they can be advantageously used for the treatment of diseases which can be improved or prevented by the inhibition of heparanase activity.

The heparanase has a critical role in modulating the microenvironment via the ECM (extracellular matrix) remodeling and thus influencing a multitude of both normal and disease-related processes, as inflammation, blood coagulation, wound healing, angiogenesis, fibrosis and tumor cell growth, invasion and metastasis (for a review of the roles and activities of heparanase see for example "Heparanase: From basic research to therapeutic applications in cancer and inflammation", Vlodavsky I., Singh P., Boyango I., Gutter-Kapon L., Elkin M., Sanderson RD, Ilan N., Drug Resist. Updat. 2016, 29, 54). Heparanase has also been recently reported to play a fundamental role in many other pathologies, such as nephropathies of different origin, severe infective disorders, gastrointestinal diseases, osteolysis in bone metastases as well as in other bone tissue related pathologies, atherosclerosis, cardiovascular disorders and cutaneous aging. The panorama of diseases affected by heparanase expression and activity also includes the so-called rare diseases, such as amyloidosis, hereditary multiple exostoses and idiopathic avascular necrosis of bone as well as pathologies that are not rare in terms of incidence, but are rarely diagnosed, such as vulvodynia (Rivara et al., 2016).

Therefore, the compounds of formula (I) can be advantageously used in a broad range of diseases.

In particular, heparanase expression is enhanced in cancers, including various carcinomas, sarcomas and hematological malignancies, and it has been demonstrated that it correlates with increased tumor size, tumor angiogenesis, enhanced metastasis and poor prognosis. Indeed, treatments of tumor-bearing mice with heparanase-inhibiting compounds has been shown to markedly attenuate tumor progression further supporting an anti-heparanase therapy for multiple types of cancer (Vlodavski et al., 2016).

Therefore, the compounds of the invention, thanks to their demonstrated activity as heparanase inhibitors, can be advantageously used in the treatment of tumors and metastasis.

In particular, they can be used in the treatment of any kind of tumor, both solid and liquid. For example they can be used in the treatment of solid tumors, such as for example glioma and sarcomas, such as fibrosarcoma and osteosarcoma, and in the treatment of hematological malignancies.

The involvement of heparanase in inflammatory reactions has also been shown (Vlodavski et al., 2016) thus supporting the use of inhibitors of heparanase also in diseases characterized by or related with acute and chronic inflammation, such as colitis, atherosclerosis, pancreatitis, Chron's disease, psoriasis and others.

More recently, heparanase overexpression has been documented to be related to the endothelial mesenchymal transition (EMT; Specific heparanase inhibition reverses glucose-induced mesothelial-to-mesenchymal transition Masola .V et al Nephrol. Dial. Transplant 2017; 1-9) and thus heparanase inhibition may be also beneficial in those pathologic processes involving EMT, as fibrosis occurring at different organs.

Examples of diseases which can be treated by compounds of formula (I) are tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular, peritoneal fibrosis glucose induced following peritoneal dialysis) and aging.

The skilled in the art, for example a physician, can identify and recognize if a disease can be ameliorated by an anti-heparanase activity on the basis of the common knowledge in the field. For example, for a broad, even though not exhaustive, overview, on possible clinical applications of heparanase inhibitors, reference can be made to the previously mentioned reviews of Rivara et al., 2016 and Vlodavski et al., 2016.

Also, tests are known in the field and available to the skilled person to evaluate if a compound is endowed with an anti-heparanase activity, for example the AGAIA assay (Hammond E. et al. Anal. Biochem. 2010, 396, 112).

Compounds of the invention are particularly advantageous also for non-tumor applications since they are endowed with a strong anti-heparanase activity and therefore they can already be efficiently used at very low doses at which they are not cytotoxic. Indeed, in some embodiments they are selective heparanase inhibitors already at concentrations in which they do not interfere significantly with cell proliferation. This renders them particularly useful for therapeutic applications wherein a cytotoxic effect is undesired. Also, it has been found that such compounds at not cytotoxic concentrations are already able to inhibit invasion of tumor cells thus being particularly useful in the treatment of tumors and metastasis, also as adjuvants of chemotherapeutic agents. The compounds of the invention are also able to interfere with adhesion properties of cancer cells and they have an inhibitory effect against tumor transcription genes encoding for proangiogenic factors thus further supporting their therapeutic use in cancer diseases and metastasis.

The skilled person in the field knows how to determine the suitable dose in view of the desired application and his general knowledge in the medical and pharmaceutical field and by carrying out routine experimentation.

Another object of the present invention is a pharmaceutical composition containing at least one compound of formula (I) as an active ingredient, in an amount such as to produce a significant therapeutic effect, together with pharmaceutically acceptable vehicles and/or excipients.

Such pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

The compositions according to the present invention contain, along with the active ingredient, at least one pharmaceutically acceptable vehicle or excipient. These may be particularly useful formulation coadjuvants, e.g. solubilising agents, dispersing agents, suspension agents, and emulsifying agents.

The pharmaceutical compositions according to the invention comprising the compound of formula (I) can also contain one or more further active ingredients, for example chemotherapeutic agents. In particular, when the pharmaceutical composition is used for the treatment of a tumor, it preferably comprises also a chemotherapeutic agent. Indeed in some embodiments, the compounds of the invention act by modifying the tumor microenvironment to affect cell growth and spread as well as to facilitate the tumor killing action of other agents. Therefore, the combination of the compounds of the invention with any chemotherapeutic agent can be particularly useful. The chemotherapeutic agent can be chosen among the known agents according to the general knowledge of the skilled person in the field. For example, said chemotherapeutic agent can be selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens. When said chemotherapeutic agent is a cytotoxic agent, it is preferably selected from the group consisting of melphalan, irinotecan, rituximab and lapatinib. When said chemotherapeutic agent is a proteasome inhibitor, it is preferably selected from the group consisting of bortezomib, carfilzomib and ixazomib. When said chemotherapeutic agent is an immunomodulatory agent, it is preferably selected from the group consisting of thalidomide, lenalidomide and pomalidomide.

For example, when the disease to be treated is multiple myeloma (MM), the compounds of the invention can be administered in conjunction with chemotherapy drugs known to be effective in MM, such as for example cytotoxic agents, for example melphalan, proteasome inhibitors, immunomodulatory drugs (IMIDs) and monoclonal antibodies towards highly expressed myeloma associated antigens.

According to the administration route chosen, the compositions will be in solid or liquid form, suitable for oral, parenteral or topical administration.

Generally, the compounds of this invention are administered in a "therapeutically effective amount". The amount of the compound actually administered will typically be determined by a physician, in the light of the relevant circumstances, including the condition to be treated, the chosen route of administration, the actual compound administered, drug combination, the age, body weight, and response of the individual patient, the severity of the patient's symptoms, and the like. For any compound, the therapeutically effective dose can be estimated initially either in cell culture assays or in animal models, usually mice, rats, guinea pigs, rabbits, dogs, or pigs. The animal model may also be used to determine the appropriate concentration range and route of administration. Such information can then be used to determine useful doses and routes for administration in humans. In calculating the Human Equivalent Dose (HED) it is recommended to use the conversion table provided in Guidance for Industry and Reviewers document (2002, U.S. Food and Drug Administration, Rockville, Maryland, USA).

Generally, an effective dose will be from 0.01 mg/kg to 100 mg/kg, preferably 0.05 mg/kg to 50 mg/kg. For any compound, the therapeutically effective dose can be estimated initially either in cell culture assays or in animal models, usually mice, rats, guinea pigs, rabbits, dogs, or pigs. The precise effective dose for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

Compositions may be administered individually to a patient or may be administered in combination with other agents, drugs or hormones. For example they can be used in combination with further active ingredients, such as for example anticancer drugs.

The medicament may also contain a pharmaceutically acceptable carrier, for administration of a therapeutic agent. Such carriers include antibodies and other polypeptides, genes and other therapeutic agents such as liposomes, provided that the carrier does not induce the production of antibodies harmful to the individual receiving the composition, and which may be administered without undue toxicity.

Suitable carriers may be large, slowly metabolised macromolecules such as proteins, polysaccharides, polylactic acids, polyglycolic acids, polymeric amino acids, amino acid copolymers and inactive virus particles.

A thorough discussion of pharmaceutically acceptable carriers is available in Remington's Pharmaceutical Sciences (Mack Pub. Co., N.J. 1991).

Pharmaceutically acceptable carriers in therapeutic compositions may additionally contain liquids such as water, saline, glycerol and ethanol.

Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The medicament of this invention may be administered by any number of routes including, but not limited to, oral, intravenous, intramuscular, intra-arterial, intramedullary, intrathecal, intraventricular, transdermal or transcutaneous applications, subcutaneous, intraperitoneal, intranasal, enteral, topical, sublingual, intravaginal or rectal means.

The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include refilled, pre-measured ampoules or syringes of the liquid compositions or pills, tablets, capsules or the like in the case of solid compositions. In such compositions, the compound of the invention is usually a minor component (from about 0.1 to about 50% by weight or preferably from about 1 to about 40% by weight) with the remainder being various vehicles or carriers and processing aids helpful for forming the desired dosing form. Dosage treatment may be a single dose schedule or a multiple dose schedule.

A further object of the invention is a process for the preparation of pharmaceutical compositions characterized by mixing one or more compounds of formula (I) with suitable excipients, stabilizers and/or pharmaceutically acceptable diluents.

It is also an object of the invention the use of such pharmaceutical compositions as a medicament, in particular in the treatment of a disease which can be improved or prevented by an anti-heparanase activity. More in particular, the above mentioned diseases can be treated by such pharmaceutical compositions.

Pharmaceutical compositions and preparations thereof are within the general knowledge and reference can be made to conventional manuals and national and European Pharmacopoeias.

In an embodiment, the compounds of the invention can also be used for cosmetic applications thanks to their activity of inhibition of heparanase. Indeed, heparanase is produced in the skin by epidermal keratinocytes composing the epidermis and fibroblasts or vascular endothelial cells of the dermis and it decomposes heparan sulfate proteoglycan, which regulates differentiation and growth of the epidermis. It is known that heparanase can promote the formation of wrinkles; indeed, inhibition of heparanase activity suppresses the release of growth factors that accompanies decomposition of heparan sulfate, and allows migration of growth factors between the epidermis and dermis to be controlled, thereby aiding in anti-aging and anti-wrinkles effects of the skin (see for example EP2484349, US2014080878 and the work "Hyperpigmentation in human solar lentigo is promoted by heparanase-induced loss of heparan sulfate chains at the dermal-epidermal junction." Iriyama S., Ono T., Aoki H., Amano S. Dermatol Sci. 2011, 64(3), 223).

In view of the above and of the heparanase inhibition activity of the compounds of the invention, it is also an object of the present invention the cosmetic use of the compounds of formula (I) for prevention or treatment of skin aging. In particular, they can be used for preventing or suppressing the formation of wrinkles, in particular wrinkles caused by reduced level of heparanase sulfate in the epidermal basal membrane.

The compounds of the invention are particularly suitable for such cosmetic applications due to their low cytotoxicity. In particular, due to their potent anti-heparanase activity, they can already exert their effect at very low doses, at which they are not cytotoxic and thus suitable for cosmetic uses.

It is also an object of the invention, a cosmetic composition comprising the compound of formula (I) and cosmetically acceptable excipients and/or vehicles. Such excipients or vehicles can be chosen by the skilled person among the cosmetic ingredients commonly used in the field. In particular, they are components commonly used in external preparations. Such cosmetic composition can be used in the prevention or suppression of wrinkles or in any other anti-aging applications. It is preferably applied and administered topically.

The following examples further illustrate the invention.

### EXAMPLES

### Abbreviations:

- Boc: *t*-butoxycarbonyl
- CDI: carbonyldiimidazole
- CH₂Cl₂: dichloromethane
- DMF: dimethylformamide
- LiOH·H₂O: lithium hydroxide monohydrate
- MeOH: methanol
- on: over night
- rt: room temperature
- TEA: triethylamine
- TFA: trifluoroacetic acid
- THF: tetrahydrofuran

### General Remarks

¹H and ¹³C NMR data were obtained with a Varian VXR 200 spectrometer and a Varian Mercury Plus 400 spectrometer, respectively. Peak positions are given in parts per million (δ) downfield, and J values are given in hertz. Positive-ion electrospray ionization (ESI) mass spectra were recorded on a double-focusing Finnigan MAT 95 instrument with BE geometry. TLC was carried out using glass plates coated with silica gel 60 F₂₅₄ by Merck, and compounds were visualized by UV detection or with aqueous KMnO₄. Flash column chromatography was performed using 230-400 mesh silica gel and the indicated solvent system. Organic solutions were dried over anhydrous Na₂SO₄. Solvents and reagents that are commercially available were purchased from Aldrich (Sigma-Aldrich) or Alfa Aesar (Johnson Matthey Company) and were used without further purification unless otherwise noted.

### Starting Materials

L-leucine methyl ester hydrochloride (**1a**), L-glycine methyl ester hydrochloride (**1b**), N(epsilon)-Boc-L-lysine *tert*-butyl ester hydrochloride (**1c**), L-alanine methyl ester hydrochloride (**1d**), L-phenylalanine methyl ester hydrochloride (**1e**), L-glutamic acid di methyl ester hydrochloride (**1f**), L-glycin-glycine methyl ester hydrochloride (**1g**), ethyl 2-(3-nitrophenyl)acetate (**3h**), ethyl 2-(4-nitrophenyl)acetate (**3j**), 1-methyl-4-nitro-1H-pyrrole-2-carbonyl chloride (**5**), 3-nitrobenzoyl chloride (**8a**), 3-nitro-5-(trifluoromethyl)benzoyl chloride (**8b**), 3,3'-Oxydibenzoic acid (**26**), glycine *tert*-butyl ester hydrochloride (**31b**), L-alanine tert-butyl ester hydrochloride (**31d**), L-leucine *tert-butyl* ester hydrochloride (**31a**), L-phenylalanine *tert*-butyl ester hydrochloride (**31e**), L-glutamic acid di-*tert*-butyl ester hydrochloride (**31f**), N(epsilon)-Boc-L-lysine *tert*-butyl ester hydrochloride (**31c**) and (*R*)-boroleucine-(+)-pinanediol trifluoroacetate (**37**) are commercially available. 1-Methyl-4-nitro-1*H*-pyrrole-2-carboxylic acid (Ong et al., 2012) (**20**) and 3,3'-(carbonylbis(azanediyl))dibenzoic acid (Drewe et al., 2008) (**29**) were prepared as previously reported (Ong, C. W.; Yang, Y.-T.; Liu, M.-C.; Fox, K. R.; Liu, P. H.; Tung, H.-W. Org. Biomol. Chem. 2012, 10, 1040-1046).

### EXAMPLES 1-11

### Synthesis of diphenyl ureas

The preparation of new compounds (Scheme 13a) entails the condensation of aminoacids **1a-h** with 2-(3-nitrophenyl)acetyl chloride **2**, to afford the intermediate **3a-h** in good yields. The nitro derivatives **3a-h** were transformed into the corresponding amine analogues **4a-h** in quantitative yield by catalytic hydrogenation (Pd on charcoal, 10%) and subsequently condensed with **5** affording the nitro compounds **6a-h** in 70-89% yields. The latter nitro compounds were then reduced to **7a-h** by catalytic hydrogenation and the resulting amino derivates coupled with 3-nitrobenzoyl chloride (**8a**) to afford the nitro derivate **9a-h**. Compound **7b** was further condensed with **8b** to yield the nitro derivate **9i**. Nitro compounds **9a-9i** were in turn reduced by catalytic hydrogenation to furnish the amino analogues **10a-i** which were further treated with CDI to synthesize the ureidic compounds **11a-i** in moderate yield (15%) Compound **12c** was obtained by treatment of derivative **11c** with TFA, while compounds **12a-b**, **12d-f** and **12i** were obtained after hydrolysis of the esters **11a-b, 11d-f** and **11i,** respectively, with LiOH in THF. Finally compounds **13a**, **13b** and **13c** were prepared by treatment of **12b**,**12g** and **12i**, respectively, with stoichiometric amount of NaOH (0.1 N).

The compound 12j was obtained following the same synthetic way described above, using ethyl 2-(4-nitrophenyl)acetate as starting compound (Scheme 13 b).

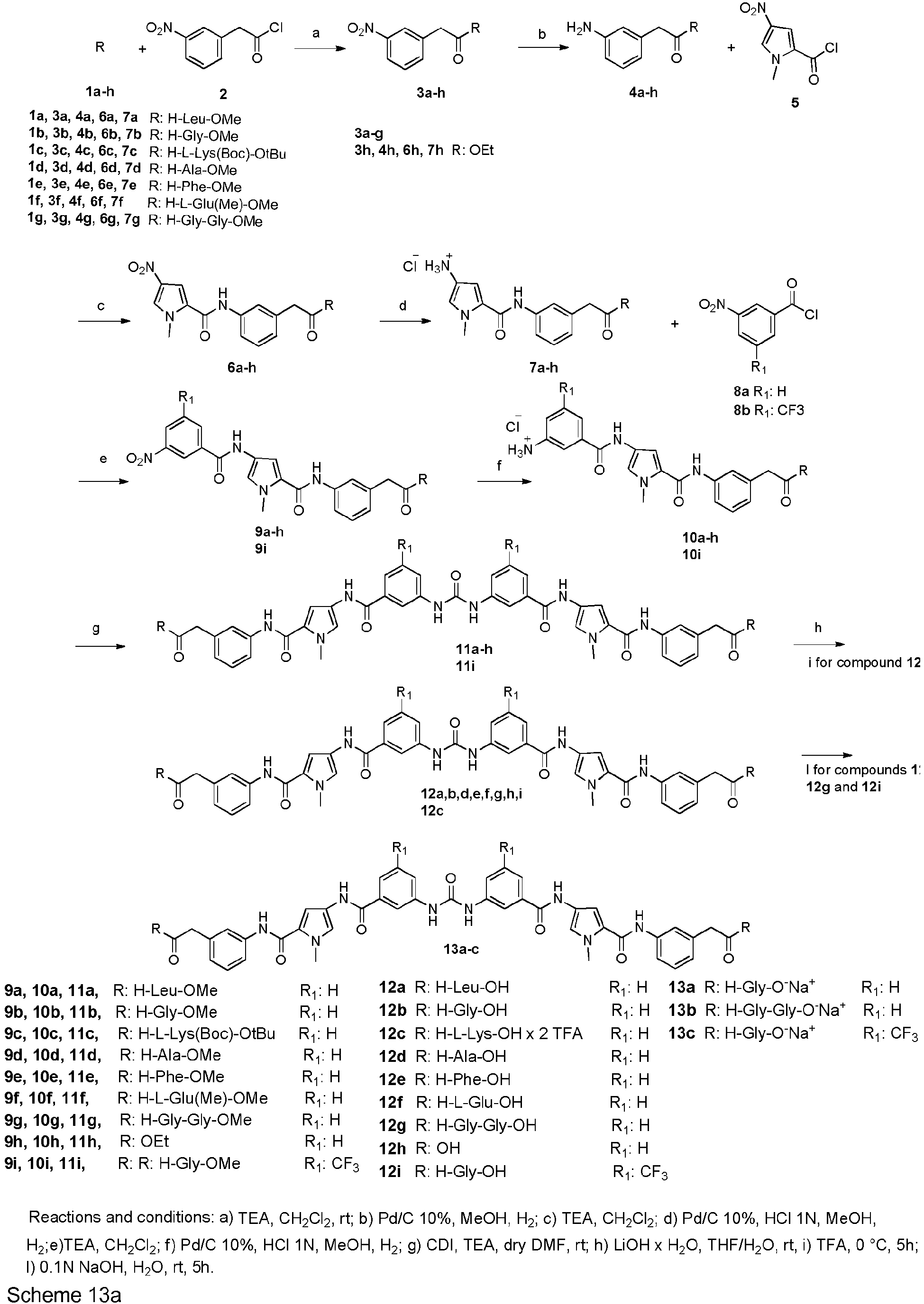

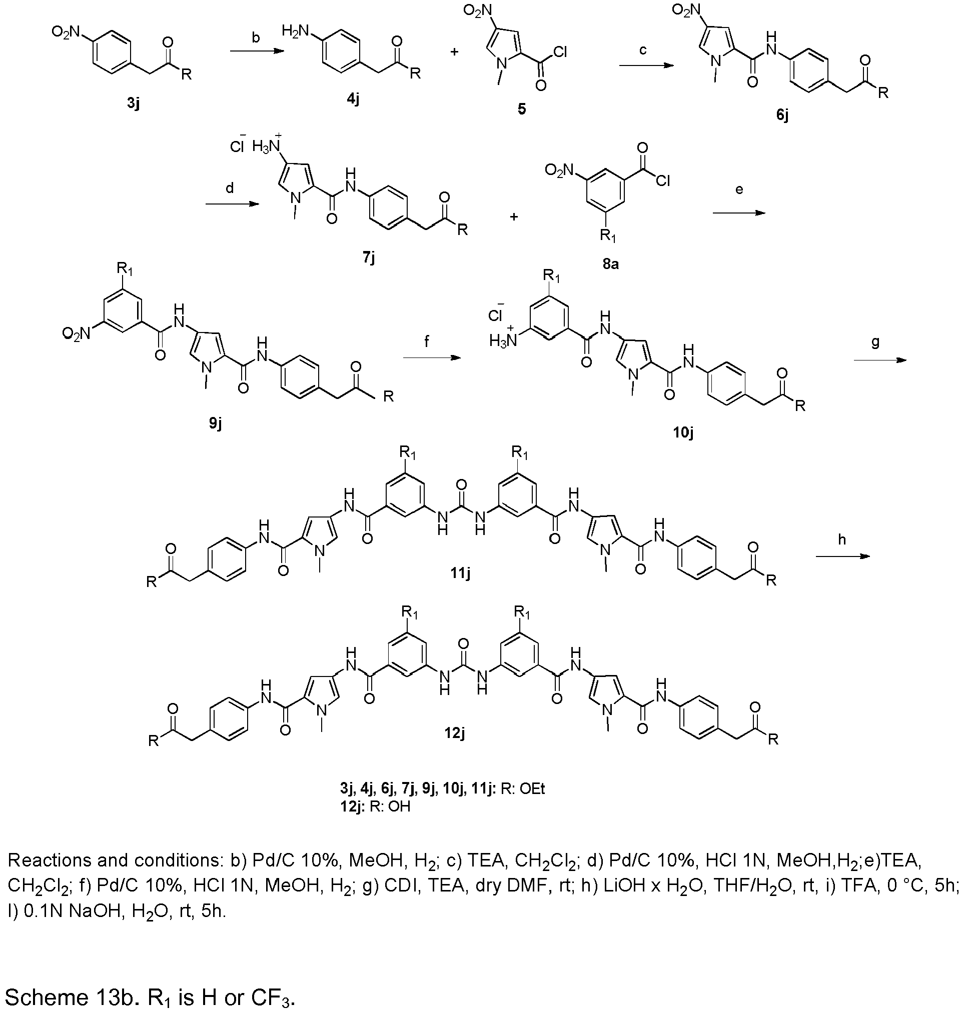

Preparation of each compound is disclosed more in details in the following.

### Example 1

### Preparation of compound SST0884AA1 (12a)

**Methyl *N*-[(3-nitrophenyl)acetyl]leucinate (3a)**. To a solution of L-leucine methyl ester hydrochloride (**1a**) (300 mg, 1.65 mmol) and TEA (0.46 mL, 3.30 mmol) in CH₂Cl₂ (10 mL), was added slowly to a cold solution (5 °C) of 2-(3-nitrophenyl)acetyl chloride (**2**) (494 mg, 2.48 mmol) in CH₂Cl₂ (5 mL). After stirring over night at 25 °C, the reaction mixture was diluted with CH₂Cl₂ and water. The organic phase was washed for three times with 1 N HCl, saturated aqueous NaHCO₃, brine, dried over Na₂SO₄ and concentrated under reduced pressure. The residue was purified by column chromatography on silica gel column with EtOAc:Petroleum ether (30:70 v/v) as eluent to furnish the desired product methyl methyl *N-*[(3-nitrophenyl)acetyl]leucinate (**3a**) (450 mg, 88%). ¹H-NMR 200 MHz (DMSO-*d*₆) δ: 0.81 (d, 3H, *J*=6.2 Hz), 0.88 (d, 3H, *J*=6.0 Hz), 1.52-1.58 (m, 3H), 3.60 (s, 3H), 3.66 (s, 2H), 4.15 (m, 1H), 7.61-7.69 (m, 2H), 8.09-8.15 (m, 2H), 8.62 (d, 1H, *J*=7.4 Hz). MS (ESI): [M+1]⁺ = 308.13.

**Methyl *N*-[(3-aminophenyl)acetyl]leucinate (4a).** A solution of methyl *N*-[(3-nitrophenyl)acetyl]leucinate (**3a**) (1.05 g, 3.40 mmol) in MeOH (25 mL) was hydrogenated over 10% Pd/C (catalytic amount) for 4h. The catalyst was filtered off and the resulting solution was concentrated to afford methyl *N*-[(3-aminophenyl)acetyl]leucinate (**4a**) (800 mg, 84 %). ¹H-NMR 200 MHz (DMSO-*d*₆) δ: 0.81 (d, 3H, *J*=6.2 Hz), 0.88 (d, 3H, *J*=6.0 Hz), 1.52-1.58 (m, 3H), 3.60 (s, 3H), 3.66 (s, 2H), 4.15 (m, 1H), 5.47 (b, 2H), 7.61-7.69 (m, 2H), 8.09-8.15 (m, 2H), 8.62 (d, 1H, *J*=7.4 Hz). MS (ESI): [M+1]⁺ = 278.13.

**Methyl *N*-[(3-{[(1-methyl-4-nitro-1*H*-pyrrol-2-yl)carbonyl]amino}phenyl)acetyl]leucinate (6a)**. To a solution of methyl ***N*-[(3-aminophenyl)acetyl]leucinate** (**4a**) (944 mg, 3.39 mmol) and TEA (0.47 mL, 3.39 mmol) in CH₂Cl₂ (20 mL), was added slowly to a cold solution (5 °C) of 1-methyl-4-nitro-1 H-pyrrole-2-carbonyl chloride (**5**) (959 mg, 5.08 mmol) in CH₂Cl₂ (5 mL). After stirring over night at 25 °C, the reaction mixture was diluted with CH₂Cl₂ and water. The organic phase was washed for three times with 1 N HCl, saturated aqueous NaHCO₃, brine, dried over Na₂SO₄ and concentrated under reduced pressure. The residue was purified by column chromatography on silica gel using EtOAc:Petroleum ether (30:70 v/v) as eluent to furnish the desired product methyl *N*-[(3-{[(1-methyl-4-nitro-1*H*-pyrrol-2-yl)carbonyl]amino}phenyl)acetyl]leucinate (**6a**) (889 mg, 61%).
¹H-NMR 200 MHz (DMSO-*d*₆) δ: 0.82 (d, 3H, *J*=6.4 Hz), 0.88 (d, 3H, *J*=6.2 Hz), 1.52-1.62 (m, 3H), 3.46 (s, 2H), 3.61 (s, 3H), 3.95 (s, 3H), 4.26-4.30 (m, 1H), 6.99 (d, 1H, *J*=7.8 Hz), 7.26 (t, 1H, *J*=7.8 Hz), 7.55 (d, 1H, *J*=8.0 Hz), 7.65 (s, 1 H), 7.71 (d, 1 H, *J*=2.0 Hz), 8.22 (d, 1 H, *J*=2.0 Hz), 8.50 (d, 1 H, *J*=7.6 Hz), 10.11 (s, 1 H). MS (ESI): [M+1]⁺ = 431.07.

**5-({[3-(2-{[1-(methoxycarbonyl)-3-methylbutyl]amino}-2-oxoethyl)phenyl]amino}carbonyl)-1-methyl-1H-pyrrol-3-aminium chloride (7a)**. To a solution of methyl *N*-[(3-{[(1-methyl-4-nitro-1*H*-pyrrol-2-yl)carbonyl]amino}phenyl)acetyl]leucinate (**6a**) (300 mg, 0.69 mmol) in MeOH (20 ml) was added 1N HCl (0.69 mL, 0.69mmol). The mixture of reaction was hydrogenated over 10% Pd/C (catalytic amount) for 4h. The catalyst was filtered off and the resulting solution was concentrated to afford 5-({[3-(2-{[1-(methoxycarbonyl)-3-methylbutyl]amino}-2-oxoethyl)phenyl]amino}carbonyl)-1-methyl-1*H*-pyrrol-3-aminium chloride (**7a**) (283 mg, 93 %).
¹H-NMR 200 MHz (DMSO-*d*₆) δ: 0.79 (d, 3H, *J*=6.8 Hz), 0.86 (d, 3H, *J*=6.4 Hz), 1.43-1.63 (m, 3H), 3.45 (s, 2H), 3.58 (s, 3H), 3.83 (s, 3H), 4.21-4.27 (m, 1H), 6.95 (d, 1H, *J*=7.6 Hz), 7.08 (d, 1H, *J*=2.0 Hz), 7.14 (d, 1H, *J*=2.0 Hz), 7.16-7.26 (m, 1H), 7.52 (d, 1H, *J*=8.0 Hz), 7.61 (s, 1H), 8.50 (d, 1H, *J*=7.6 Hz), 9.92-10.01 (m, 4H). MS (ESI): [M+1]⁺ = 401.28.

**Methyl *N*-({3-[({1-methyl-4-[(3-nitrobenzoyl)amino]-1*H*-pyrrol-2-yl}carbonyl)amino]phenyl}acetyl)leucinate (9a)**. To a solution of 5-({[3-(2-{[1-(methoxycarbonyl)-3-methylbutyl]amino}-2-oxoethyl)phenyl]amino}carbonyl)-1-methyl-1*H-*pyrrol-3-aminium chloride (**7a**) (304 mg, 0.69 mmol) and TEA (0.20 mL, 1.39 mmol) in CH₂Cl₂ (35 mL), was added slowly to a cold solution (5 °C) of 3-nitrobenzoyl chloride (**8a**) (194 mg, 1.05 mmol) in CH₂Cl₂ (5 mL). After stirring over night at 25 °C, the reaction mixture was diluted with CH₂Cl₂ and water. The organic phase was washed for three times with 1 N HCl, saturated aqueous NaHCO₃, brine, dried over Na₂SO₄ and concentrated under reduced pressure. The residue was purified by column chromatography on silica gel column using EtOAc:light petroleum (50:50 v/v) as eluent to furnish the desired product methyl *N*-({3-[({1-methyl-4-[(3-nitrobenzoyl)amino]-1*H*-pyrrol-2-yl}carbonyl)amino]phenyl}acetyl)leucinate (**9a**) (310 mg, 82%). ¹H-NMR 200 MHz (DMSO-*d*₆) δ: 0.82 (d, 3H, *J*=6.4 Hz), 0.89 (d, 3H, *J*=6.4 Hz), 1.43-1.63 (m, 3H), 3.45 (s, 2H), 3.61 (s, 3H), 3.89 (s, 3H), 4.21-4.27 (m, 1 H), 6.97 (d, 1H, *J*=7.6 Hz), 7.19-7.23 (m, 2H), 7.43 (d, 1 H, *J*=1.6 Hz), 7.57 (m, 1 H), 7.69 (s, 1 H), 7.86 (t, 1H, *J*=7.8 Hz), 8.38-8.47 (m, 3H), 8.80 (d, 1 H, *J*=3.6 Hz), 9.91 (s, 1 H), 10.75 (s, 1 H). MS (ESI): [M+1]⁺ = 550.36.

**3-({[5-({[3-(2-{[1-(methoxycarbonyl)-3-methylbutyl]amino}-2-oxoethyl)phenyl]amino)carbonyl)-1-methyl-1*H*-pyrrol-3-yl]amino}carbonyl)benzenaminium chloride (10a)**. To a solution of methyl *N*-({3-[({1-methyl-4-[(3-nitrobenzoyl)amino]-1*H*-pyrrol-2-yl}carbonyl)amino]phenyl}acetyl)leucinate (**9a**) (194 mg, 0.35 mmol) in MeOH (30 mL) was added 1N HCl (0.35 mL, 0.35 mmol). The mixture reaction was hydrogenated over 10% Pd/C (catalytic amount) for 4h. The catalyst was filtered off and the resulting solution was concentrated to afford 3-({[5-({[3-(2-{[1-(methoxycarbonyl)-3-methylbutyl]amino}-2-oxoethyl)phenyl]amino}carbonyl)-1-methyl-1*H-*pyrrol-3-yl]amino}carbonyl)benzenaminium chloride (**10a**) (130 mg, 67%). ¹H-NMR 200 MHz (DMSO-*d*₆) δ: 0.82 (d, 3H, *J*=6.2 Hz), 0.89 (d, 3H, *J*=6.0 Hz), 1.51-1.58 (m, 3H), 3.44 (s, 2H), 3.61 (s, 3H), 3.87 (s, 3H), 4.21-4.26 (m, 1 H), 6.95 (d, 1H, *J*=7.8 Hz), 7.18-7.69 (m, 9H), 8.49 (d, 1H, *J*=7.8 Hz), 9.89 (s, 1H), 10.43 (s, 1H). MS (ESI): [M+1]⁺ = 520.36.

**Dimethyl 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}bis(4-methylpentanoate) (11a).** To a solution of 3-({[5-({[3-(2-{[1-(methoxycarbonyl)-3-methylbutyl]amino}-2-oxoethyl)phenyl]amino}carbonyl)-1-methyl-1*H*-pyrrol-3-yl]amino}carbonyl)benzenaminium chloride (**10a**) (130 mg, 0.23 mmol) in dry DMF (2 mL), was added TEA (0.06 mL, 0.47 mmol) and 0.25 equiv. of CDI for 4 times every 30 min. The reaction mixture was stirred for 4 h at room temperature and then heated at 70 °C under Ar for 36 h. After being cooled at room temperature, the solvent was evaporated under vacuum and the residue was purified by column chromatography on silica gel column EtOAc:MeOH (90:10 v/v) as eluent to furnish the desired product dimethyl 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}bis(4-methylpentanoate) (**11a**) (24.45 mg, 10%). ¹H-NMR 400 MHz (DMSO-*d*₆) δ: 0.82 (d, 3H, *J*=6.4 Hz), 0.89 (d, 3H, *J*=6.4 Hz), 1.51-1.58 (m, 3H), 3.45 (s, 2H), 3.61 (s, 3H), 3.87 (s, 3H), 4.21-4.26 (m, 1H), 6.95 (d, 1H, *J*=7.8 Hz), 7.17-7.22 (m, 2H), 7.40-7.41 (m, 2H), 7.52 (m, 2H), 7.63 (s, 1 H), 7.70 (m, 2H), 8.12 (s, 1 H), 8.45 (d, 2H, *J*=7.8 Hz ), 9.90 (s, 1 H), 10.43 (s, 1 H). MS (ESI): [M+1]⁺ = 1066.82.

**2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}bis(4-methylpentanoic acid) SST0884AA1 (12a)**. To a suspension of dimethyl 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}bis(4-methylpentanoate) (**11a**) (17.10 mg, 0.02 mmol) in THF (5 mL), was added LiOH·H₂O (2.7 mg, 0.06 mmol) and water (1.6 mL). After stirring over night at 25 °C, the solvent was concentrated under reduced pressure, the reaction mixture was diluted with water and then acidified with 1 N HCl. The precipitate was filtered and wash with water to give 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}bis(4-methylpentanoic acid) **SST0884AA1 (12a)** (6.97 mg, 42%). ¹H-NMR 400 MHz (DMSO-*d*₆) δ: 0.82 (d, 6H, *J*=6.4 Hz), 0.88 (d, 6H, *J*=6.4 Hz), 1.51-1.53 (m, 6H), 3.45 (s, 4H), 3.87 (s, 6H), 4.21-4.26 (m, 2H), 6.96 (d, 2H, *J*=7.6 Hz), 7.17-7.21 (m, 4H), 7.40-7.43 (m, 4H), 7.53 (m, 4H), 7.68 (s, 4H), 8.01 (s, 2H), 8.25 (s, 2H), 9.19 (b, 2H), 9.88 (s, 2H), 10.36 (s, 2H), 10.45 (b, 2H).
¹³C-NMR (400 MHz, DMSO-*d*₆) δ: 21.28 (2), 22.78, 24.22, 36.15, 41.99, 50.37, 105.61, 117.51, 118.12, 119.38, 120.56, 120.84 (2), 122.04, 122.77, 123.64, 128.07, 128.67, 135.39, 136.51, 139.12, 139.80, 159.65, 163.77, 169.89, 174.17. HPLC: 16.20 min. MS (ESI): [M+1]⁺ = 1037.80.

### Example 2

### Preparation of compound SST0755NA1 (13a)

**Methyl *N*-[(3-nitrophenyl)acetyl]glycinate (3b)** was synthesized according to the procedure described for compound (**3a**), using L-glycine methyl ester hydrochloride (**1b**) as aminoacid. ¹H-NMR 200 MHz (DMSO-*d*₆) δ: 3.62 (s, 3H), 3.68 (s, 2H), 3.87 (d, 2H, *J*=5.8 Hz), 7.57-7.75 (m, 2H), 8.08-8.17 (m, 2H), 8.65 (b, 1H). Yield 60%. MS (ESI): [M+1]⁺ = 253.07.

**Methyl *N*-[(3-aminophenyl)acetyl]glycinate (4b)** was synthesized according to the procedure described for compound (**4a**). ¹H-NMR 200 MHz (DMSO-*d*₆) δ: 3.62 (s, 3H), 3.68 (s, 2H), 3.87 (d, 2H, *J*=5.8 Hz), 7.32 (b, 2H), 7.57-7.75 (m, 2H), 8.08-8.17 (m, 2H), 8.65 (b, 1H). Yield 91%. MS (ESI): [M+1]⁺ = 223.10.

**Methyl *N*-[(3-{[(1-methyl-4-nitro-1*H*-pyrrol-2-yl)carbonyl]amino}phenyl)acetyl]glycinate** (6b) was synthesized according to the procedure described for compound (**6a**). ¹H-NMR 200 MHz (DMSO-*d*₆) δ: 3.48 (s, 2H), 3.62 (s, 3H), 3.85 (d, 2H, *J*=5.8 Hz), 3.95 (s, 3H), 7.02 (d, 1H. *J*=7.6 Hz), 7.26 (t, 1 H, *J*=7.8 Hz), 7.56-7.63 (m, 2H), 7.72 (d, 1H, *J*=1.8 Hz), 8.21 (d, 1H, *J*=1.4 Hz), 8.49 (b, 1 H), 10.11 (s, 1H). Yield 85%. MS (ESI): [M+1]⁺ = 374.35.

**5-{[(3-{2-[(2-methoxy-2-oxoethyl)amino]-2-oxoethyl}phenyl)amino]carbonyl}-1-methyl-1*H*-pyrrol-3-aminium chloride** (**7b**) was synthesized according to the procedure described for compound (**7a**). ¹H-NMR 200 MHz (DMSO-*d*₆) δ: 3.48 (s, 2H), 3.62 (s, 3H), 3.85 (d, 2H, *J*=5.8 Hz), 3.95 (s, 3H), 7.02 (d, 1H, *J*=7.6 Hz), 7.26 (t, 1H, *J*=7.8 Hz), 7.56-7.63 (m, 2H), 7.72 (d, 1H, *J*=1.8 Hz), 8.21 (d, 1 H, *J*=1.4 Hz), 8.49 (b, 1 H), 10.11 (s, 1H), 10.45 (b, 3H). Yield 82%. MS (ESI): [M+1]⁺ = 345.15.

**Methyl *N*-({3-[({1-methyl-4-[(3-nitrobenzoyl)amino]-1*H*-pyrrol-2-yl}carbonyl)amino]phenyl}acetyl)glycinate** (**9b**) was synthesized according to the procedure described for compound (**9a**). ¹H-NMR 200 MHz (DMSO-*d*₆) δ: 3.47 (s, 2H), 3.62 (s, 3H), 3.84-3.87 (m, 5H), 6.99 (s, 1H), 7.21-7.24 (m, 2H), 7.43 (d, 1H, *J*=1.6 Hz), 7.56 (s, 1 H), 7.68 (s, 1 H), 7.86 (t, 1H, *J*=8.0 Hz), 8.39-8.44 (m, 3H), 8.80 (b, 1 H), 9.93 (s, 1 H), 10.76 (s, 1 H). Yield 72%. MS (ESI): [M+1]⁺ = 493.46.

**3-{[(5-{[(3-{2-[(2-methoxy-2-oxoethyl)amino]-2-oxoethyl}phenyl)amino]carbonyl}-1-methyl-1*H*-pyrrol-3-yl)amino]carbonyl}benzenaminium chloride** (**10b**) was synthesized according to the procedure described for compound (**10a**). ¹H-NMR 200 MHz (DMSO-*d*₆) δ: 3.45 (s, 2H), 3.64 (s, 3H), 3.84-3.87 (m, 5H), 6.99 (s, 1H), 7.21-7.24 (m, 2H), 7.43 (d, 1H, *J*=1.6 Hz), 7.56 (s, 1 H), 7.68 (s, 1 H), 7.86 (t, 1H, *J*=8 Hz), 8.36-8.42 (m, 3H), 8.78 (b, 1 H), 9.97 (s, 1H), 10.74 (s, 1H). Yield 85%. MS (ESI): [M+1]⁺ = 464.18.

**Dimethyl 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}diethanoate (11b)** was synthesized according to the procedure described for compound (**11a**). ¹H-NMR 200 MHz (DMSO-*d*₆) δ: 3.48 (s, 2H), 3.62 (s, 3H), 3.84-3.87 (m, 5H), 6.97 (d, 1H, *J*=7.6 Hz), 7.17-7.27 (m, 2H), 7.39-7.47 (m, 2H), 7.54-7.58 (m, 2H), 7.67 (s, 2H), 8.00 (s, 1 H), 8.48 (s, 1 H), 9.01 (s, 1 H), 9.89 (s, 1 H), 10.35 (s, 1 H). Yield 15%. MS (ESI): [M+1]⁺ = 953.33.

**2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}diethanoic acid** (**12b**) was synthesized according to the procedure described for compound (**12a**). ¹H-NMR 200 MHz (DMSO-*d*₆) δ: 3.52 (s, 4H), 3.76 (d, 4H, *J*=6.0 Hz), 3.87 (s, 6H), 6.97 (d, 2H, *J*=7.6 Hz), 7.17-7.22 (m, 4H), 7.40-7.43 (m, 4H), 7.55-7.56 (m, 4H), 7.62-7.70 (m, 4H), 8.00 (s, 2H), 8.37 (s, 2H), 9.00 (s, 2H), 9.90 (s, 2H), 10.37 (s, 2H), 12.58 (b, 2H). Yield 90%. ¹³C-NMR (400 MHz, DMSO-*d*₆) δ: 36.18, 40.65, 42.01, 105.65, 117.52, 118.21, 119.43, 120.62, 120.83 (2), 122.05, 122.79, 123.80, 128.15, 128.71, 135.40, 136.29, 139.17, 139.72, 152.50, 159.68, 163.73, 170.35, 171.22. MS (ESI): [M+1]⁺ = 925.32.

**Disodium 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}diethanoate salt SST0755NA1 (13a)**. To a suspension of 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}diethanoic acid (**12b**) (36.50 mg, 0.04 mmol) in water (2 mL) was added dropwise 0.1 N NaOH (0.87 mL, 0.08 mmol) at 0 °C. After stirring 5 h at 25 °C, the solvent was concentrated under reduced pressure to give disodium 2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}diethanoate salt SST0755NA1 (**13a**) (31.30 mg, 83%). HPLC: 11.62 min.

### Example 3

### Preparation of compound SST0900TF1 (12c).

***Tert*-butyl *N*⁶-(*tert*-butoxycarbonyl)-*N²*-[(3-nitrophenyl)acetyl]lysinate (3c)** was synthesized according to the procedure described for compound (**3a**), using Nepsilon-Boc-L-lysine *tert*-butyl ester hydrochloride (**1c**) as aminoacid. ¹H-NMR 200 MHz (DMSO-*d*₆) δ: 1.34-1.36 (m, 22H), 1.60-1.68 (m, 2H), 2.86-2.89 (m, 2H), 3.65 (s, 2H), 4.04-4.07 (m, 1 H), 6.76 (b, 1H), 7.57-7.74 (m, 2H), 8.08-8.17 (m, 2H), 8.48 (d, 1H, *J*=7.2 Hz). Yield 59%. MS (ESI): [M+1]⁺ = 466.28.

***Tert-butyl N*⁶-(*tert-*butoxycarbonyl)-*N*²-[(3-aminophenyl)acetyl]lysinate (4c)** was synthesized according to the procedure described for compound (**4a**). ¹H-NMR 200 MHz (DMSO-*d*₆) δ: 1.34-1.36 (m, 22H), 1.60-1.68 (m, 2H), 2.86-2.89 (m, 2H), 3.65 (s, 2H), 4.04-4.07 (m, 1H), 6.78 (b, 1H), 7.15 (s, 2H), 7.57-7.72 (m, 2H), 8.08-8.17 (m, 2H), 8.42 (d, 1H, *J*=7.2 Hz). Yield 79%. MS (ESI): [M+1]⁺ = 435.28.

***Tert*-butyl *N*⁶-(*tert*-butoxycarbonyl)-*N*²-[(3-{[(1-methyl-4-nitro-1*H*-pyrrol-2-yl)carbonyl]amino}phenyl)acetyl]lysinate (6c)** was synthesized according to the procedure described for compound (**6a**). ¹H-NMR 200 MHz (DMSO-*d*₆) δ: 1.36 (m, 22H), 1.55-1.79 (m, 2H), 2.86-2.89 (m, 2H), 3.46 (s, 2H), 3.95 (s, 3H), 3.97-4.07 (m, 1 H), 6.79 (b, 1H), 7.03 (d, 1H), 7.25 (m, 1H), 7.55-7.72 (m, 3H), 8.22 (d, 1H, *J*=1.6 Hz), 8.39 (d, 1 H), 10.11 (s, 1H). MS (ESI): [M+1]⁺ = 586.68.

**5-[({3-[2-({1-(*tert*-butoxycarbonyl)-5-[(*tert*-butoxycarbonyl)amino]pentyl}amino)-2-oxoethyl**]**phenyl**}**amino**)**carbonyl**]-**1**-**methyl**-**1*H***-**pyrrol**-**3**-**aminium chloride (7c)** was synthesized according to the procedure described for compound (**7a**). ¹H-NMR 200 MHz (DMSO-*d*₆) δ: 1.36 (m, 22H), 1.55-1.79 (m, 2H), 2.86-2.89 (m, 2H), 3.46 (s, 2H), 3.94 (s, 3H), 3.97-4.09 (m, 1H), 6.77 (b, 1H), 7.03 (d, 1H), 7.25 (m, 1H), 7.52-7.72 (m, 3H), 8.22 (d, 1H, *J*=1.6 Hz), 8.39 (d, 1H), 9.53 (b, 3 H), 10.15 (s, 1H). Yield 80%. MS (ESI): [M+1]⁺ = 558.63.

**Isopropyl *N*⁶-(*tert*-butoxycarbonyl)-*N*²-({3-[({1-methyl-4-[(4-nitrobenzoyl)amino]-1*H-*pyrrol-2-yl}carbonyl)amino]phenyl}acetyl)lysinate (9c)** was synthesized according to the procedure described for compound (**9a**). ¹H-NMR 200 MHz (DMSO-*d*₆) δ: 1.36 (m, 22H), 1.52-1.74 (m, 2H), 2.89-2.91 (m, 2H), 3.45 (s, 2H), 3.89 (s, 3H), 3.97-4.04 (m, 1 H), 6.79 (b, 1 H), 6.99 (d, 1 H), 7.20-7.23 (m, 2H), 7.43 (d, 1 H, J=1.8 Hz), 7.59 (m, 1 H), 7.69 (s, 1 H), 7.84 (m, 1H), 8.22-8.43 (m, 3H), 8.80 (s, 1H), 9.91 (s, 1H), 10.74 (s, 1H). Yield 61%. MS (ESI): [M+1]⁺ = 705.79.

**4-[({5-[({3-[2-({1-(*tert*-butoxycarbonyl)-5-[(*tert*-butoxycarbonyl)amino]pentyl}amino)-2-oxoethyl]phenyl}amino)carbonyl]-1-methyl-1*H*-pyrrol-3-yl}amino)carbonyl]benzenaminium chloride (10c)** was synthesized according to the procedure described for compound (**10a**). ¹H-NMR 200 MHz (DMSO-*d*₆) δ: 1.34 (m, 22H), 1.50-1.75 (m, 2H), 2.89-2.90 (m, 2H), 3.45 (s, 2H), 3.89 (s, 3H), 3.97-4.04 (m, 1 H), 6.77 (b, 1 H), 6.99 (d, 1 H), 7.20-7.23 (m, 2H), 7.43 (d, 1 H, *J*=1.8 Hz), 7.59 (m, 1 H), 7.69 (s, 1 H), 7.84 (m, 1H), 8.22-8.43 (m, 3H), 8.80 (s, 1H), 9.91 (s, 1H), 10.24 (b, 3H), 10.74 (s, 1H). Yield 61%. MS (ESI): [M+1]⁺ = 677.72.

**Di-*tert*-butyl 2**,**2**'-{**carbonylbis**[**imino**-**3**,**1**-**phenylenecarbonylimino**(**1**-**methyl**-**1*H***-**pyrrole-4,2-diyl)carbonylimino-3,1-phenylene(1-oxoethane-2,1-diyl)imino]}bis{6-[(*tert-*butoxycarbonothioyl)amino]hexanoate} (11c)** was synthesized according to the procedure described for compound (**11a**). ¹H-NMR 200 MHz (DMSO-*d*₆) δ: 1.36 (m, 22 H), 1.56-1.72 (m, 2H), 2.86-2.90 (m, 2H), 3.45 (s, 2H), 3.87 (s, 3H), 4.01- 4.12 (m, 1 H), 6.79 (b, 1H), 6.98 (d, 1H), 7.17-7.22 (m, 2H), 7.39-7.43 (m, 2H), 7.52-7.54 (m, 2H), 7.64-7.69 (m, 2H), 8.00 (s, 1 H), 8.33 (d, 1H. *J*=7.6 Hz), 9.00 (s, 1 H), 9.89 (s, 1 H), 10.36 (s, 1 H). Yield 20%. MS (ESI): [M+1]⁺ = 1380.37.

**5,5'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}bis(1-aminium-6-hexanoic acid) trifluoroacetate salt SST0900TF1 (12c).** In 0.50 mL of TFA was added di-*tert*-butyl 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}bis{6-[(*tert-*butoxycarbonothioyl)amino]hexanoate} (**11c**) (10.5 mg, 0.01 mmol) at 0 °C. After stirring 5 h at 25 °C, the solvent was concentrated under reduced pressure to give 5,5'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}bis(1-aminium-6-hexanoic acid) trifluoroacetate salt SST0900TF1 (**12c**) (6.12 mg, 65%). ¹H-NMR 200 MHz (DMSO-*d*₆) δ: 1.33-1.76 (m, 12H), 2.86-2.90 (m, 4H), 3.47 (s, 4H), 3.66 (s, 6H), 3.94-4.02 (m, 2H), 7.02-7.14 (m, 6H), 7.52-7.57 (m, 6H), 7.79-7.97 (m, 10H), 8.24 (d, 2H, *J*=7.6 Hz), 9.65 (s, 2H), 10.19 (s, 2H), 12.86 (b, 6H), 14.52 (b, 2H). ¹³C-NMR (400 MHz, DMSO-*d*₆) δ: 15.08, 22.26, 26.44, 30.54, 41.96, 51.52, 64.83, 105.69, 116.93, 118.16, 119.24 (2), 120.93, 122.07, 123.67 (2), 128.10, 128.67, 134.30, 136.43, 139.15, 140.72, 151.43, 157.68, 157.99, 168.10, 170.10, 173.51. MS (ESI): [M+1]⁺ = 1067.52. HPLC: 10.86 min.

### Example 4

### Preparation of compound SST0883AA1 (12d)

**Methyl *N*-[(3-nitrophenyl)acetyl]alaninate (3d)** was synthesized according to the procedure described for compound (**3a**), using L-alanine methyl ester hydrochloride (**1d**) as aminoacid. ¹H-NMR 200 MHz (DMSO-*d*₆) δ: 1.29 (d, 3H, *J*=7.2 Hz), 3.60 (s, 3H), 3.65 (s, 2H), 4.23-4.30 (m, 1H), 7.61-7.73 (m, 2H), 8.09-8.15 (m, 2H), 8.67 (d, 1H, *J*=6.6 Hz). Yield 79%.
MS (ESI): [M+1]⁺= 267.09.

**Methyl *N*-[(3-aminophenyl)acetyl]alaninate (4d)** was synthesized according to the procedure described for compound (**4a**). ¹H-NMR 200 MHz (DMSO-*d*₆) δ= 1.27 (d, 3H, *J*=7.4 Hz), 3.26 (s, 2H), 3.61 (s, 3H), 4.20-4.27 (m, 1H), 4.97 (s, 2H), 6.37-6.44 (m, 3 H), 6.90 (t, 1 H, *J*=7.6 Hz), 8.41 (d, 1H, *J*=7 Hz). Yield 93%. MS (ESI): [M+1]⁺= 237.23.

**Methyl *N*-[(3-{[(1-methyl-4-nitro-1*H*-pyrrol-2-yl)carbonyl]amino}phenyl)acetyl]alaninate (6d)** was synthesized according to the procedure described for compound (**6a**). ¹H-NMR 200 MHz (DMSO-*d*₆) δ: 1.28 (d, 3H, *J*=7.4 Hz), 3.45 (s, 2H), 3.61 (s, 3H), 3.95 (s, 3H), 4.22-4.29 (m, 1 H), 7.00 (d, 1 H, *J*=7.6 Hz), 7.26 (t, 1H, *J*=7.8 Hz), 7.55-7.63 (m, 2H), 7.72 (d, 1H, *J*=1.6 Hz), 8.22 (d, 1H, *J*=1.6 Hz), 8.55 (d, 1H, *J*=7.0 Hz), 10.12 (s, 1H). Yield 64%. MS (ESI): [M+1]⁺= 389.44.

**5-{[(3-{2-[(2-methoxy-1-methyl-2-oxoethyl)amino]-2-oxoethyl}phenyl)amino]carbonyl}-1-methyl-1*H*-pyrrol-3-aminium chloride (7d)** was synthesized according to the procedure described for compound (**7a**). ¹H-NMR 200 MHz (DMSO-*d*₆) δ: 1.28 (d, 3H, *J*=7.4 Hz), 3.44 (s, 2H), 3.61 (s, 3H), 3.88 (s, 3H), 4.22-4.29 (m, 1H), 6.96-7.27 (m, 4H), 7.55-7.62 (m, 2H), 8.56 (d, 1H, *J*=6.6 Hz), 9.94 (s, 1H), 10.02 (s, 3H). Quantitative yield. MS (ESI): [M+1]⁺= 359.46.

**Methyl *N*-({3-[({1-methyl-4-[(3-nitrobenzoyl)amino]-1*H*-pyrrol-2-yl}carbonyl)amino]phenyl}acetyl)alaninate (9d)** was synthesized according to the procedure described for compound (**9a**). ¹H-NMR 200 MHz (DMSO-*d*₆) δ: 1.29 (d, 3H, *J*=7.4 Hz), 3.44 (s, 2H), 3.61 (s, 3H), 3.89 (s, 3H), 4.23-4.30 (m, 1 H), 6.96 (d, 1H, *J*=7.4 Hz), 7.21-7.27 (m, 2H), 7.43 (d, 1 H, *J*= 2), 7.54-7.67 (m, 2H), 7.84 (b, 1 H), 8.38-8.53 (m, 2H), 8.55 (d, 1H, *J*=7.4 Hz), 8.80 (s, 1H), 9.92 (s, 1H), 10.75 (s, 1H). Yield 78%. MS (ESI): [M+1]⁺= 508.41.

**3-{[(5-{[(3-{2-[(2-methoxy-1-methyl-2-oxoethyl)amino]-2-oxoethyl}phenyl)amino]carbonyl}-1-methyl-1*H*-pyrrol-3-yl)amino]carbonyl}benzenaminium chloride (10d)** was synthesized according to the procedure described for compound (**10a**). ¹H-NMR 200 MHz (DMSO-*d*₆) δ: 1.29 (d, 3H, *J*=7.4 Hz), 3.43 (s, 2H), 3.61 (s, 3H), 3.87 (s, 3H), 4.22-4.29 (m, 1 H), 6.96 (d, 1H, *J*=7.0 Hz), 7.18 (d, 1 H, *J*=1.8 Hz), 7.23-7.66 (m, 9H), 8.55 (d, 1 H, *J*=6.8 Hz), 9.89 (s, 1H), 10.42 (s, 1 H). Yield 96%. MS (ESI): [M+1]⁺= 478.31.

**Dimethyl 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}dipropanoate (11d)** was synthesized according to the procedure described for compound (**11a**)._¹H-NMR 200 MHz (DMSO-*d*₆) δ: 1.29 (d, 3H, *J*=7.4 Hz), 3.44 (s, 2H), 3.61 (s, 3H), 3.87 (s, 3H), 4.22-4.28 (m, 1 H), 6.95 (d, 1H, *J*=8.0 Hz), 7.17-7.23 (m, 2H), 7.39-7.47 (m, 2H), 7.54-7.67 (m, 2H), 7.67 (s, 2H), 8.01 (s, 1 H), 8.53 (d, 1 H, *J*=6.8 Hz), 9.89 (s, 1 H), 10.36 (s, 1H). Yield 21%._MS (ESI): [M+1]⁺= 981.70.

**2**,**2**'-{**carbonylbis**[**imino**-**3**,**1**-**phenylenecarbonylimino**(**1**-**methyl**-**1*H*-pyrrole-4,2-diyl)carbonylimino-3,1-phenylene(1-oxoethane-2,1-diyl)imino]}dipropanoic acid SST0883AA1 (12d)** was synthesized according to the procedure described for compound (**12a**). ¹H-NMR 400 MHz (DMSO-*d*₆) δ: 1.28 (d, 6H, *J*=7.4 Hz), 3.45 (s, 4H), 3.85 (s, 6H), 4.15-4.19 (m, 2 H), 6.94 (d, 2H, *J*=7.6 Hz), 7.15-7.22 (m, 4H), 7.38-7.43 (m, 4H), 7.53-7.55(m, 4H), 7.65-7.67 (m, 4H), 7.98 (s, 2H),8.39 (d, 2H, *J*=7.2 Hz), 8.97 (s, 2H), 9.88 (s, 2H), 10.35 (s, 2H), 12.49 (s, 2H). Quantitative yield. ¹³C-NMR 400 MHz (DMSO-*d*₆) δ: 17.21, 36.18, 41.88, 47.48, 105.64, 117.53, 118.16, 119.42, 120.62, 120.83, 122.04, 122.79, 123.75, 128.12, 128.72, 135.40, 136.40, 139.14, 139.72, 152.50, 159.68, 163.73, 169.73, 174.11. MS (ESI): [M+1]⁺= 953.74. HPLC: 12.68 min.

### Example 5

### Preparation of compound SST0881AA1 (12e)

**Methyl *N*-[(3-nitrophenyl)acetyl]phenylalaninate (3e)** was synthesized according to the procedure described for compound (3a), using L-phenylalanine methyl ester hydrochloride (**1e**) as aminoacid. ¹H-NMR 200 MHz (DMSO-*d*₆) δ: 2.89-3.04 (m, 2H), 3.59 (s, 2H), 3.61 (s, 3H), 4.47-4.51 (m, 1H), 7.14-7.23 (m, 5H), 7.55-7.58 (m, 2H), 8.06-8.11 (m, 2H), 8.69 (d, 1H, *J*=7.8 Hz). Yield 90%. MS (ESI): [M+1]⁺= 343.27.

**Methyl *N*-[(3-aminophenyl)acetyl]phenylalaninate (4e)** was synthesized according to the procedure described for compound (**4a**). ¹H-NMR 200 MHz (DMSO-*d*₆) δ: 2.90-2.99 (m, 2H), 3.24 (s, 2H), 3.58 (s, 3H), 4.41-4.46 (m, 1 H), 4.94 (s, 2H), 6.26-6.40 (m, 3H), 6.83-6.91 (m, 1H), 7.16-7.27 (m, 5H), 8.42 (d, 1H, *J*=7.6 Hz). Yield 96%. MS (ESI): [M+1]⁺= 313.25.

**Methyl *N*-[(3-{[(1-methyl-4-nitro-1*H*-pyrrol-2-yl)carbonyl]amino}phenyl)acetyl]phenylalaninate (6e)** was synthesized according to the procedure described for compound (**6a**). ¹H-NMR 200 MHz (DMSO-*d*₆) δ: 2.91-3.01 (m, 2H), 3.41 (s, 2H), 3.59 (s, 3H), 3.95 (s, 3H), 4.43-4.48 (m, 1H), 6.87 (d, 1H, *J*=7.8 Hz), 7.16-7.25 (m, 6H), 7.52-7.59 (m, 2H), 7.72 (d, 1H, *J*=2.0 Hz), 8.22 (d, 1 H, *J*=2.0 Hz), 8.57 (d, 1H, *J*=7.8 Hz), 10.10 (s, 1H). Yield 79%. MS (ESI): [M+1]⁺= 465.08.

**5-{[(3-{2-[(1-benzyl-2-methoxy-2-oxoethyl)amino]-2-oxoethyl}phenyl)amino]carbonyl}-1-methyl-1*H*-pyrrol-3-aminium chloride (7e)** was synthesized according to the procedure described for compound (**7a**). ¹H-NMR 200 MHz (DMSO-*d*₆) δ: 2.93-3.01 (m, 2H), 3.41 (s, 2H), 3.59 (s, 3H), 3.89 (s, 3H), 4.44-4.46 (m, 1 H), 6.85 (d, 1 H, *J*=7.6 Hz), 7.07-7.24 (m, 8H), 7.52-7.56 (m, 2H), 8.58 (d, 1H, *J*=7.6 Hz), 9.92 (s, 1H), 10.00 (s, 3H). Quantitative yield. MS (ESI): [M+1]⁺= 435.44.

**Methyl *N*-({3-[({1-methyl-4-[(3-nitrobenzoyl)amino]-1*H*-pyrrol-2-yl}carbonyl)amino]phenyl}acetyl)phenylalaninate (9e)** was synthesized according to the procedure described for compound (**9a**). ¹H-NMR 200 MHz (DMSO-*d*₆) δ: 2.92-3.02 (m, 2H), 3.41 (s, 2H), 3.60 (s, 3H), 3.89 (s, 3H), 4.44-4.49 (m, 1H), 6.83 (d, 1H, *J*=7.8 Hz), 7.15-7.25 (m, 7H), 7.43-7.63 (m, 3H), 7.79-7.88 (m, 1 H), 8.38-8.45 (m, 2H), 8.47 (d, 1 H, *J*=8.0 Hz), 8.77-8.81 (m, 1 H), 9.90 (s, 1 H), 10.75 (s, 1 H). Yield 48%. MS (ESI): [M+1]⁺= 584.58.

**3-{[(5-{[(3-{2-[(1-benzyl-2-methoxy-2-oxoethyl)amino]-2-oxoethyl} phenyl)amino]carbonyl}-1-methyl-1*H*-pyrrol-3-yl)amino]carbonyl}benzenaminium chloride (10e)** was synthesized according to the procedure described for compound (**10a**). ¹H-NMR 200 MHz (DMSO-*d*₆) δ: 2.92-3.02 (m, 2H), 3.40 (s, 2H), 3.59 (s, 3H), 3.87 (s, 3H), 4.44-4.48 (m, 1H), 6.83 (d, 1H, *J*=8.0 Hz), 7.18-7.77 (m, 14 H), 8.56 (d, 1H, *J*=7.8 Hz), 9.88 (s, 1 H), 10.48 (s, 1 H). Quantitative yield. MS (ESI): [M+1]⁺= 554.60.

**Dimethyl 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}bis(3-**phenylpropanoate) **(11e)** was synthesized according to the procedure described for compound (**11a**). ¹H-NMR 200 MHz (DMSO-*d*₆) δ: 2.92-3.02 (m, 2H), 3.40 (s, 2H), 3.59 (s, 3H), 3.87 (s, 3H9, 4.43-4.49 (m, 1H), 6.83 (d, 1H, J=7.6 Hz), 7.18-7.25 (m, 6H), 7.39-7.69 (m, 7H), 8.00 (s, 1 H), 8.54 (d, 1 H, J=7.8 Hz), 9.01 (s, 1 H), 9.87 (s, 1 H), 10.35 (s, 1 H). Yield 15%. MS (ESI): [M+1]⁺= 1133.82.

**2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}bis(3-phenylpropanoic acid) SST0881AA1 (12e)** was synthesized according to the procedure described for compound (**12a**). ¹H-NMR 400 MHz (DMSO-*d*₆) δ: 2.87-3.04 (m, 4H), 3.40 (s, 4H), 3.87 (s, 6H), 4.39-4.42 (m, 2H), 6.82 (d, 2H, *J*=7.6 Hz), 7.17-7.24 (m, 12H), 7.40-7.69 (m, 14H), 7.99 (s, 2H), 8.38 (d, 2H, *J*=8.4 Hz), 8.97 (s, 2H), 9.88 (s, 2H), 10.37 (s, 2H), 12.75 (s, 2H). Quantitative yield. ¹³C-NMR 400 MHz (DMSO-*d*₆) δ: 36.17, 36.67, 41.99, 53.24, 105.61, 117.53, 118.16, 119.40, 120.63, 120.95 (2), 122.05, 122.81, 123.67, 126.29 (2), 128.06 (2), 128.71, 129.06 (2), 135.40, 136.24, 137.40, 139.07, 139.70, 152.49, 159.65, 163.73, 169.86, 172.90. MS (ESI): [M+1]⁺= 1105.93. HPLC 14.30 min.

### Example 6

### Preparation of compound SST0882AA1 (12f)

**Dimethyl *N*-[(3-nitrophenyl)acetyl]glutamate (3f)** was synthesized according to the procedure described for compound (**3a**), using L-glutamic acid di methyl ester hydrochloride (**1f**) as aminoacid. ¹H-NMR 200 MHz (DMSO-*d*₆) δ: 1.79-2.05 (m, 2H), 2.34-2.48 (m, 2H), 3.57 (s, 3H), 3.61 (s, 3H), 3.66 (s, 2H), 4.21-4.36 (m, 1H), 7.61-7.69 (m, 2H), 8.15-8.16 (m, 2H), 8.65 (d, 1H, *J*=7.6 Hz). Yield 79%. MS (ESI): [M+1]⁺ = 339.18.

**Dimethyl *N*-[(3-aminophenyl)acetyl]glutamate (4f)** was synthesized according to the procedure described for compound (**4a**). ¹H-NMR 200 MHz (DMSO-*d*₆) δ: 1.72-2.01 (m, 2H), 2.37-2.47 (m, 2H), 3.55 (s, 3H), 3.64 (s, 3H), 3.67 (s, 2H), 4.21-4.36 (m, 1 H), 4.89 (b, 2H), 7.61-7.69 (m, 2H), 8.12-8.19 (m, 2H), 8.69 (d, 1H, *J*=7.6 Hz). Yield 95%. MS (ESI): [M+1]⁺ = 309.33.

**Dimethyl *N*-[(3-{[(1-methyl-4-nitro-1*H*-pyrrol-2-yl)carbonyl]amino}phenyl)acetyl]glutamate (6f)** was synthesized according to the procedure described for compound (**6a**). ¹H-NMR 200 MHz (DMSO-*d*₆) δ: 1.85-2.02 (m, 2H), 2.39 (t, 2H, *J*=7.6 Hz), 3.46 (s, 2H), 3.57 (s, 3H), 3.61 (s, 3H), 3.95 (s, 3H), 4.24-4.28 (m, 1 H), 6.99 (d, 1H, *J*=7.8 Hz), 7.26 (t, 1 H, *J*=7.8 Hz), 7.56 (d, 1H, *J*=8.0 Hz), 7.66 (s, 1 H), 7.71 (d, 1H, *J*=2.0 Hz), 8.22 (d, 1H, *J*=1.8 Hz), 8.54 (d, 1H, *J*=9.6 Hz), 10.12 (s, 1H). Yield 60%. MS (ESI): [M+1]⁺ = 461.24.

**5-((3-(2-((1,5-dimethoxy-1,5-dioxopentan-2-yl)amino)-2-oxoethyl)phenyl)carbamoyl)-1-methyl-1H-pyrrol-3-aminium chloride (7f)** was synthesized according to the procedure described for compound (**7a**). ¹H-NMR 200 MHz (DMSO-*d*₆) δ: 1.85-2.02 (m, 2H), 2.40 (t, 2H, *J*=7.6 Hz), 3.46 (s, 2H), 3.57 (s, 3H), 3.61 (s, 3H), 3.89 (s, 3H), 4.24-4.28 (m, 1 H), 6.99 (d, 1H, *J*=7.8 Hz), 7.11-7.24 (m, 3H), 7.54 (d, 1H, *J*=8.0 Hz), 7.65 (s, 1H), 8.53 (d, 1H, *J=*2.0 Hz), 9.94 (s, 1H), 10.07 (b, 3H). Yield 72%. MS (ESI): [M+1]⁺ = 431.41.

**Dimethyl *N*-({3-[({1-methyl-4-[(3-nitrobenzoyl)amino]-1*H*-pyrrol-2-yl}carbonyl)amino]phenyl}acetyl)glutamate (9f)** was synthesized according to the procedure described for compound (**9a**). ¹H-NMR 200 MHz (DMSO-*d*₆) δ: 1.85-2.02 (m, 2H), 2.36-2.48 (m, 2H), 3.46 (s, 2H), 3.57 (s, 3H), 3.62 (s, 3H), 3.89 (s, 3H), 4.26-4.28 (m, 1 H), 6.96 (d, 1H, *J*=7.8 Hz), 7.21-7.23 (m, 2H), 7.43 (d, 1H, *J*=1.6 Hz), 7.54 (d, 1H, *J*=8.6 Hz), 7.70 (s, 1 H), 7.80-7.88 (m, 1 H), 8.38-8.45 (m, 2H), 8.53 (d, 1 H, *J*=7.6 Hz), 8.80 (d, 1H, *J*=3.8 Hz), 9.92 (s, 1H), 10.75 (s, 1H). Yield 76%. MS (ESI): [M+1]⁺ = 580.19.

**3-((5-((3-(2-((1,5-dimethoxy-1,5-dioxopentan-2-yl)amino)-2-oxoethyl)phenyl)carbamoyl)-1-methyl-1H-pyrrol-3-yl)carbamoyl)benzenaminium chloride (10f)** was synthesized according to the procedure described for compound (**10a**). ¹H-NMR 200 MHz (DMSO-*d*₆) δ: 1.85-2.02 (m, 2H), 2.36-2.48 (m, 2H), 3.46 (s, 2H), 3.57 (s, 3H), 3.62 (s, 3H), 3.89 (s, 3H), 4.26-4.28 (m, 1H), 6.96 (d, 1H, *J*=7.8 Hz), 7.21-7.23 (m, 2H), 7.43 (d, 1H, *J*=1.6 Hz), 7.54 (d, 1H, *J*=8.6 Hz), 7.70 (s, 1H), 7.80-7.88 (m, 1 H), 8.38-8.45 (m, 2H), 8.53 (d, 1H, *J*=7.6 Hz), 8.80 (d, 1H, *J*=3.8 Hz), 9.10 (b, 3H), 9.92 (s, 1 H), 10.75 (s, 1 H). Yield 82%. MS (ESI): [M+1]⁺ = 550.55.

**Tetramethyl 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}dipentandioate (11f)** was synthesized according to the procedure described for compound (**11a**). ¹H-NMR 200 MHz (DMSO-*d*₆) δ: 1.79-2.01 (m, 2H), 2.36-2.50 (m, 2H), 3.45 (s, 2H), 3.57 (s, 3H), 3.61 (s, 3H), 3.87 (s, 3H), 4.26-4.28 (m, 1H), 6.93-7.02 (m, 2H), 7.18-7.26 (m, 1H), 7.39 (s, 1H), 7.53-7.57 (m, 2H), 7.65-7.70 (m, 3H), 8.00 (s, 1H), 8.53 (d, 1H, *J*=7.4 Hz), 9.06 (s, 1H), 9.89 (s, 1H), 10.36 (s, 1H). Yield 12%. MS (ESI): [M+1]⁺ = 1125.84.

**2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}dipentandioic acid SST0882AA1 (12f)** was synthesized according to the procedure described for compound (**12a**). ¹H-NMR 200 MHz (DMSO-*d*₆) δ: 1.79-1.97 (m, 4H), 2.27-2.31 (m, 4H), 3.45 (s, 4H), 3.87 (s, 6H), 4.19-4.21 (m, 2H), 6.96 (d, 2H, *J*=3.8 Hz), 7.17 (m, 4H), 7.40-7.45 (m, 4H), 7.54-7.56 (m, 4H), 7.68 (m, 4H), 7.99 (s, 2H), 8.39 (d, 2H, *J*=4.0 Hz), 9.17 (s, 2H), 9.89 (s, 2H), 10.37 (s, 2H), 12.46 (b, 4H). Yield 71%. ¹³C-NMR (400 MHz, DMSO-*d*₆) δ: 26.36, 29.96, 41.94, 51.16, 54.85, 105.63, 117.44, 118.14, 119.42, 120.58, 120.85 (2), 122.05, 122.79, 123.65, 128.12, 128.70, 135.43, 136.39, 139.17, 139.77, 152.55, 159.68, 163.76, 170.12, 173.24, 173.63. MS (ESI): [M+1]⁺ = 1069.37. HPLC: 12.20 min.

### Example 7

### Preparation of compound 12i

**Methyl *N*-[(3-{[(1-methyl-4-{[3-nitro-5-(trifluoromethyl)benzoyl]amino}-1*H*-pyrrol-2-yl)carbonyl]amino}phenyl)acetyl]glycinate (9i)**. To a solution of 5-({[3-(2-{[1-(methoxycarbonyl)-3-methylbutyl]amino}-2-oxoethyl)phenyl]amino}carbonyl)-1-methyl-1*H-*pyrrol-3-aminium chloride (**7a**) (304 mg, 0.69 mmol) and TEA (0.20 mL, 1.39 mmol) in CH₂Cl₂ (35 mL), was added slowly to a cold solution (5 °C) of 3-nitro-5-(trifluoromethyl)benzoyl chloride (**8b**) (194 mg, 1.05 mmol) in CH₂Cl₂ (5 mL). After stirring over night at 25 °C, the reaction mixture was diluted with CH₂Cl₂ and water. The organic phase was washed for three times with 1 N HCl, saturated aqueous NaHCO₃, brine, dried over Na₂SO₄ and concentrated under reduced pressure. The residue was purified by column chromatography on silica gel column using EtOAc:light petroleum (50:50 v/v) as eluent to furnish the desired product methyl *N*-[(3-{[(1-methyl-4-{[3-nitro-5-(trifluoromethyl)benzoyl]amino}-1*H*-pyrrol-2-yl)carbonyl]amino}phenyl)acetyl]glycinate (9i) (310 mg, 82%). ¹H-NMR 200 MHz (DMSO-*d*₆) δ: 3.47 (s, 2H), 3.62 (s, 3H), 3.84-3.90 (m, 5H), 6.98 (d, 1H, *J*=6.8 Hz), 7.22-7.28 (m, 2H), 7.45 (d, 1 H, *J*=1.6 Hz), 7.55-7.67 (m, 2H), 8.51 (s, 1 H), 8.71 (d, 2H, *J*=9.0 Hz), 9.05 (s, 1 H), 9.94 (s, 1 H), 10.96 (s, 1 H). Yield 86%. MS (ESI): [M+1]⁺= 562.38.

**3-{[(5-{[(3-{2-[(2-methoxy-2-oxoethyl)amino]-2-oxoethyl}phenyl)amino]carbonyl}-1-methyl-1*H*-pyrrol-3-yl)amino]carbonyl}-5-(trifluoromethyl)benzenaminium chloride (10i)** was synthesized according to the procedure described for compound (**10a**). ¹H-NMR 200 MHz (DMSO-*d*₆) δ: 3.47 (s, 2H), 3.62 (s, 3H), 3.83-3.87 (m, 5H), 6.99-7.03 (m, 2H), 7.17-7.23 (m, 3H), 7.37 (s, 2H), 7.55-7.67 (m, 2H), 8.52 (s, 1 H), 9.90 (s, 1 H), 10.42 (s, 1 H). Quantitative yield. MS (ESI): [M+1]⁺= 532.43.

**Dimethyl 2,2'-{carbonylbis[imino[5-(trifluoromethyl)-3,1-phenylene]carbonylimino(1-methyl-1*H*-pyrrole-4,2-diyl)carbonylimino-3,1-phenylene(1-oxoethane-2,1-diyl)imino]}diethanoate** (**11i**) was synthesized according to the procedure described for compound (**11a**). ¹H-NMR 200 MHz (DMSO-*d*₆) δ: 3.47 (s, 2H), 3.62 (s, 3H), 3.84-3.89 (m, 5H), 6.97 (d, 1H, *J*=6.8 Hz), 7.20-7.23 (m, 2H), 7.41 (s, 1 H), 7.55-7.68 (m, 3H), 7.94 (s, 1 H), 8.20 (s, 1 H), 8.50 (s, 1 H), 9.56 (s, 1 H), 9.92 (s, 1 H), 10.62 (s, 1 H). Yield 11%. MS (ESI): [M+1]⁺= 1090.0.

**2,2'-{carbonylbis[imino[5-(trifluoromethyl)-3,1-phenylene]carbonylimino(1-methyl-1*H-*pyrrole-4,2-diyl)carbonylimino-3,1-phenylene(1-oxoethane-2,1-diyl)imino]}diethanoic acid (12i)** was synthesized according to the procedure described for compound (**12a**). ¹H-NMR 400 MHz (DMSO-*d*₆) δ: 3.46 (s, 4H), 3.76 (s, 4H), 3.88 (s, 6H), 6.98 (d, 2H, *J*=7.6 Hz), 7.19-7.25 (m, 4H), 7.42 (d, 2H, *J*=1.6 Hz), 7.56-7.67 (m, 6H), 7.92 (s, 2H), 8.18 (s, 2H), 8.21 (s, 2H), 8.38 (s, 2H), 9.92 (s, 2H), 10.64 (s, 2H). Quantitative Yield. ¹³C-NMR 400 MHz (DMSO-*d*₆) δ: 36.21, 40.66, 42.01, 105.65, 116.65, 118.21, 119.54, 120.82, 121.19, 121.67, 122.49, 122.95, 123.85, 128.16, 129.43, 129.74, 136.29, 139.13, 140.57, 152.59, 159.62, 162.04, 170.35, 171.20. MS (ESI): [M+1]⁺= 1061.79. HPLC: 19.52 min.

### Example 8

### Preparation of compound SST0899NA1 (13c)

**Disodium 2,2'-{carbonylbis[imino[5-(trifluoromethyl)-3,1-phenylene]carbonylimino(1-methyl-1*H*-pyrrole-4,2-diyl)carbonylimino-3,1-phenylene(1-oxoethane-2,1-diyl)imino]}diethanoate salt SST0899NA1 (13c)** was synthesized according to the procedure described for compound (**13a**). HPLC: 19.57 min.

### Example 9

### Preparation of compound 12g

**Methyl *N*-[(3-nitrophenyl)acetyl]glycylglycinate (3g)** was synthesized according to the procedure described for compound (**3a**), using L-glycin-glycine methyl ester hydrochloride (**1g**) as aminoacid. ¹H-NMR 200 MHz (DMSO-*d*₆) δ: 3.62 (s, 3H), 3.68 (s, 2H), 3.76 (d, 2H, *J*=6.0 Hz), 3.85 (d, 2H, *J*=5.8 Hz), 7.60-7.72 (m, 2H), 8.09 (m, 2H), 8.37 (b, 1 H), 8.48 (b, 1 H). Yield 94%. MS (ESI): [M+1]⁺= 310.49.

**Methyl *N*-[(3-aminophenyl)acetyl]glycylglycinate (4g)** was synthesized according to the procedure described for compound (**4a**). ¹H-NMR 200 MHz (DMSO-*d*₆) δ: 3.62 (s, 3H), 3.68 (s, 2H), 3.76 (d, 2H, *J*=6.0 Hz), 3.85 (d, 2H, *J*=5.8 Hz),5,15 (s, 2H), 7.60-7.72 (m, 2H), 8.09 (m, 2H), 8.37 (b, 1H), 8.48 (b, 1 H). Quantitative yield. MS (ESI): [M+1]⁺= 280.49.

**Methyl *N*-[(3-{[(1-methyl-4-nitro-1*H*-pyrrol-2-yl)carbonyl]amino}phenyl)acetyl]glycylglycinate (6g)** was synthesized according to the procedure described for compound (**6a**). ¹H-NMR 200 MHz (DMSO-*d*₆) δ: 3.48 (s, 2H), 3.62 (s, 3H), 3.75 (d, 2H, *J*=5.8 Hz), 3.86 (d, 2H, *J*=5.8 Hz), 3.95 (s, 3H), 7.04-7.26 (m, 2H), 7.63-7.72 (m, 3H), 8.22 (d, 1H, *J*=1.6 Hz), 8.33 (b, 2H), 10.12 (s, 1H). Yield 51%. MS (ESI): [M+1]⁺= 432.16.

**Methyl *N*-[(3-{[(4-ammonio-1-methyl-1*H*-pyrrol-2-yl)carbonyl]amino}phenyl)acetyl]glycylglycinate chloride (7g)** was synthesized according to the procedure described for compound (**7a**). ¹H-NMR 200 MHz (DMSO-*d*₆) δ: 3.49 (s, 2H), 3.64 (s, 3H), 3.76 (d, 2H, *J*=5.8 Hz), 3.85 (d, 2H, *J*=5.8 Hz), 3.94 (s, 3H), 7.04-7.26 (m, 2H), 7.63-7.72 (m, 3H), 8.22 (d, 1H, *J*=1.6 Hz), 8.33 (b, 2H), 10.01 (s, 3H), 10.13 (s, 1 H). Quantitative yield. MS (ESI): [M+1]⁺= 402.35.

**Methyl *N*-({3-[({1-methyl-4-[(3-nitrobenzoyl)amino]-1*H*-pyrrol-2-yl}carbonyl)amino]phenyl}acetyl)glycylglycinate (9g)** was synthesized according to the procedure described for compound (**9a**). ¹H-NMR 200 MHz (DMSO-*d*₆) δ: 3.47 (s, 2H), 3.62 (s, 3H), 3.75 (d, 2H, *J*=5.8 Hz), 3.84-3.89 (m, 5H), 6.96 (d, 1H, *J*=8.0 Hz), 7.21-7.23 (m, 2H), 7.43 (d, 1H, *J*=1.6 Hz), 7.54-7.67 (m, 2H), 7.80-7.84 (m, 1H), 8.34-8.39 (m, 4H), 8.80 (b, 1H), 9.92 (s, 1 H), 10.76 (s, 1 H). Yield 63%. MS (ESI): [M+1]⁺= 551.39.

**Methyl N-({3-[({4-[(3-ammoniobenzoyl)amino]-1-methyl-1H-pyrrol-2-yl}carbonyl)amino]phenyl}acetyl)glycylglycinate chloride (10g)** was synthesized according to the procedure described for compound (**10a**). ¹H-NMR 200 MHz (DMSO-*d*₆) δ: 3.45 (s, 2H), 3.59 (s, 3H), 3.72 (d, 2H, *J*=5.8 Hz),3.79-3.84 (m, 5H), 6.95 (d, 1H, *J*=7.6 Hz), 7.17-7.22 (m, 2H), 7.37-7.39 (m, 2H), 7.49-7.56 (m, 2H), 7.64-7.82 (m, 3H), 8.32-8.36 (m, 2H), 9.89 (s, 1 H), 10.27 (s, 3H), 10.51 (s, 1 H). Quantitative yield. MS (ESI): [M+1]⁺= 521.44.

**Dimethyl 2**,**2**'-{**carbonylbis**[**imino**-**3**,**1**-**phenylenecarbonylimino**(**1**-**methyl**-**1*H***-**pyrrole**-**4**,**2-diyl)carbonylimino-3,1-phenylene(1-oxoethane-2,1-diyl)imino(1-oxoethane-2,1-diyl)imino]}diethanoate (11g)** was synthesized according to the procedure described for compound (**11a**). ¹H-NMR 200 MHz (DMSO-*d*₆) δ: 3.47 (s, 2H), 3.62 (s, 3H), 3.75 (d, 2H, *J*=5.8 Hz), 3.84-3.87 (m, 5H), 6.97 (d, 1H, *J*=7.8 Hz), 7.18-7.26 (m, 2H), 7.39-7.47 (m, 2H), 7.54-7.67 (m, 4H), 7.99 (b, 1H), 8.32 (b, 2H), 9.03 (s, 1H), 9.89 (s, 1H), 10.36 (s, 1H). Yield 18%. MS (ESI): [M+1]⁺= 1067.90.

**2**,**2**'-{**carbonylbis**[**imino**-**3**,**1**-**phenylenecarbonylimino**(**1**-**methyl**-**1*H*-pyrrole-4,2-diyl)carbonylimino-3,1-phenylene(1-oxoethane-2,1-diyl)imino(1-oxoethane-2,1-diyl)imino]}diethanoic acid (12g)** was synthesized according to the procedure described for compound (**12a**). ¹H-NMR 400 MHz (DMSO-*d*₆) δ: 3.47 (s, 4H), 3.73-3.77 (m, 8H), 3.87 (s, 6H), 6.97 (d, 2H, *J*=7.6 Hz), 7.18-7.22 (m, 4H), 7.40-7.43 (m, 4H), 7.56 (b, 2H), 7.67 (b,2H), 7.99 (s, 2H), 8.20 (b, 2H), 8.32 (b, 2H), 9.03 (s, 2H), 9.90 (s, 2H), 10.37 (s, 2H), 12.55 (s, 2H). Quantitative yield. ¹³C-NMR 400 MHz (DMSO-*d*₆) δ: 36.16, 40.47, 41.78, 42.14, 105.65, 117.50, 118.19, 119.42, 120.60, 120.83, 122.04, 122.78, 123.85, 128.13, 128.69, 135.40, 136.36, 139.14, 139.72, 152.49, 159.67, 163.73, 169.14, 170.30, 171.06. MS (ESI): [M+1]⁺= 1039.94.

### Example 10

### Preparation of compound SST0898NA1 (13b)

**Disodium 2,2'-{carbonylbis[imino-3,1-phenylenecarbonylimino(1-methyl-1H-pyrrole-4,2-diyl)carbonylimino-3,1-phenylene(1-oxoethane-2,1-diyl)imino(1-oxoethane-2,1-diyl)imino]}diethanoate salt SST0898NA1 (13b)** was synthesized according to the procedure described for compound (**13a**). HPLC: 16.22 min.

### Example 11

### Preparation of compound SST0754AA1 (12h)

**Ethyl 2-(3-aminophenyl)acetate (4h).** A solution of ethyl 2-(3-nitrophenyl)acetate (3h) (1.05 g, 3.40 mmol) in MeOH (25 mL) was hydrogenated over 10% Pd/C (catalytic amount) for 4h. The catalyst was filtered off and the resulting solution was concentrated to afford ethyl 2-(3-aminophenyl)acetate (**4h**). ¹H-NMR 200 MHz (CDCl₃) δ: 1.25 (t, 3H, *J*=7.2 Hz ), 3.51 (s, 2H), 4.14 (q, 2H, *J*=7.2 Hz), 6.59-6.71 (m, 3H), 7.11 (t, 1 H, *J*=7.8 Hz). Quantitative yield.

**Ethyl (3-{[(1-methyl-4-nitro-1*H*-pyrrol-2-yl)carbonyl]amino}phenyl)acetate (6h)** was synthesized according to the procedure described for compound (*6a*). ¹H-NMR 200 MHz (CDCl₃) δ: 1.27 (t, 3H, *J*=7.2 Hz), 3.63 (s, 2H), 4.02 (s, 3H), 4.17 (q, 2H, *J*=7.2 Hz), 7.06 (d, 1 H, *J*=7.6 Hz), 7.22-7.34 (m, 2H), 7.45-7.51 (m, 2H), 7.61 (d, 1 H, *J*=1.8 Hz), 7.73 (s, 1 H). Yield 66%.

**5-({[3-(2-ethoxy-2-oxoethyl)phenyl]amino}carbonyl)-1-methyl-1*H*-pyrrol-3-aminium chloride (7h)** was synthesized according to the procedure described for compound (7a). ¹H-NMR 200 MHz (DMSO-*d*₆) δ: 1.19 (t, 3H, *J*=7.0 Hz), 3.61 (s, 1 H), 3.72 (s, 3H), 4.08 (q, 2H, *J*=7.0 Hz), 6.32 (d, 1 H, *J*=2.0 Hz), 6.47 (d, 1 H, *J*=2.0 Hz), 6.90 (d, 1 H, *J*=7.6 Hz), 7.22 (t, 1 H, *J*=7.8 Hz), 7.56-7.64 (m, 2H), 9.52 (s, 1 H). Quantitative yield.

**Ethyl {3-[({1-methyl-4-[(3-nitrobenzoyl)amino]-1*H*-pyrrol-2-yl}carbonyl) amino] phenyl}acetate (9h)** was synthesized according to the procedure described for compound (**9a**). ¹H-NMR 200 MHz (DMSO-*d*₆) δ: 1.19 (t, 3H, *J*=7.2 Hz), 3.63 (s, 2H), 3.89 (s, 3H), 4.09 (q, 2H, *J*=7.2 Hz), 6.95 (d, 1H, *J*=8.0 Hz), 7.22-7.28 (m, 2H), 7.43 (d, 1H, *J*=2.0 Hz), 7.60-7.69 (m, 2H), 7.84 (t, 1 H, *J*=8.0 Hz), 8.39-8.44 (m, 2H), 9.94 (s, 1 H), 10.75 (s, 1 H). Yield 78%.

**3-({[5-({[3-(2-ethoxy-2-oxoethyl)phenyl]amino}carbonyl)-1-methyl-1*H*-pyrrol-3-yl]amino} carbonyl)benzenaminium chloride (10h)** was synthesized according to the procedure described for compound (**10a**). ¹H-NMR 200 MHz (DMSO-*d*₆) δ: 1.19 (t, 3H, J=6.8 Hz), 3.62 (s, 2H), 3.86 (s, 3H), 4.09 (q, 2H, J=6.8 Hz), 5.29 (s, 2H), 6.70-6.73 (m, 1 H), 6.94 (d, 1 H, *J*=7.6 Hz), 7.04-7.25 (m, 5H), 7.35 (d, 1 H, *J*=1.6 Hz), 7.59-7.68 (m, 2H), 9.89 (s, 1 H), 10.16 (s, 1 H). Quantitative yield.

**Diethyl 2,2'-{carbonylbis[imino-3,1-phenylenecarbonylimino(1-methyl-1*H*-pyrrole-4,2-diyl)carbonylimino-4,1-phenylene]}diacetate (11h)** was synthesized according to the procedure described for compound (**11a**). ¹H-NMR 400 MHz (DMSO-*d*₆) δ: 1.17 (t, 3H, J=6.8 Hz), 3.63 (s, 2H), 3.85(s, 3H), 4.06 (q, 2H, *J*=6.8 Hz), 6.92 (d, 1 H, *J*=7.2 Hz), 7.16 (d, 1 H, *J*=1.6 Hz), 721-7.25 (m, 1H), 7.38-7.44 (m, 2H), 7.53-7.67 (m, 4 H), 7.99 (s, 1H), 8.92 (s, 1 H), 9.89 (s, 1 H), 10.35 (s, 1 H). Yield 17%.

**2,2'-{carbonylbis[imino-3,1-phenylenecarbonylimino(1-methyl-1H-pyrrole-4,2-diyl)carbonylimino-4,1-phenylene]}diacetic acid SST0754AA1 (12h)** was synthesized according to the procedure described for compound (**12a**). ¹H-NMR 400 MHz (DMSO-*d*₆) δ: 3.54 (s, 4H), 3.87 (s, 6H), 6.93 (d, 2H, *J*=7.6 Hz), 7.18-7.26 (m, 4H), 7.39-7.69 (m, 12H), 8.01 (s, 2H), 9.79 (s, 2H), 9.92 (s, 2H), 10.40 (s, 2H), 12.32 (s, 2H). Quantitative yield. ¹³C-NMR 400 MHz (DMSO-*d*₆) δ: 36.16, 40.89, 105.71, 117.27, 118.32, 119.46, 120.51, 120.63, 120.79, 122.07, 122.72, 123.99, 128.26, 128.67, 135.18, 135.45, 139.31, 139.85, 152.64, 159.71, 163.82, 172.56. MS (ESI): [M+1]⁺= 812.04. HPLC: 15.30 min.

### Example 12

### Preparation of compound SST0756AA1 (12j)

**Ethyl 2-(4-aminophenyl)acetate (4j).** A solution of ethyl 2-(4-nitrophenyl)acetate (**3j**) (1.05 g, 3.40 mmol) in MeOH (25 mL) was hydrogenated over 10% Pd/C (catalytic amount) for 4h. The catalyst was filtered off and the resulting solution was concentrated to afford ethyl 2-(4-aminophenyl)acetate (**4j**). ¹H-NMR 200 MHz (CDCl₃) δ: 1.29 (t, 3H, *J*=7.2 Hz ), 3.72 (s, 2H), 4.13 (q, 2H, *J*=7.2 Hz), 6.51 (d, 2H, *J*= 7.2 Hz), 6.98 (d, 2H, *J*= 7.2 Hz). Quantitative yield.

**Ethyl 2-(4-(1-methyl-4-nitro-1H-pyrrole-2-carboxamido)phenyl)acetate (6j)** was synthesized according to the procedure described for compound (**6a**). ¹H-NMR 200 MHz (CDCl₃) δ: 1.27 (t, 3H, *J*=7.2 Hz), 3.63 (s, 2H), 4.02 (s, 3H), 4.17 (q, 2H, *J*=7.2 Hz), 7.21 (d, 2H, *J*=7.6 Hz), 7.50-7.56 (m, 3H), 8.02 (s, 1 H), 7.73 (s, 1 H). Yield 69%.

**5-((4-(2-ethoxy-2-oxoethyl)phenyl)carbamoyl)-1-methyl-1H-pyrrol-3-aminium chloride (7j)** was synthesized according to the procedure described for compound (7a). ¹H-NMR 200 MHz (DMSO-*d*₆) δ: 1.19 (t, 3H, *J*=7.0 Hz), 3.61 (s, 1 H), 3.72 (s, 3H), 4.08 (q, 2H, *J*=7.0 Hz), 7.13 (S, 1H), 7.21 (d, 2H, *J*=2.0 Hz), 7.56-7.64 (m, 3H), 8.02 (b, 1H), 9.15 (s, 1H). Quantitative yield.

Ethyl **2-(4-(1-methyl-4-(3-nitrobenzamido)-1H-pyrrole-2-carboxamido)phenyl)acetate** (9j) was synthesized according to the procedure described for compound (**9a**). ¹H-NMR 200 MHz (DMSO-*d*₆) δ: 1.21 (t, 3H, *J*=7.2 Hz), 3.63 (s, 2H), 3.89 (s, 3H), 4.09 (q, 2H, *J*=7.2 Hz), 7.13 (s, 1 H), 7.21 (d, 2H, *J*=7.2 Hz), 7.52-7.64 (m, 3H), 7.89-7.92 (m, 3H), 8.01 (s, 1 H), 9.94 (s, 1 H), 10.75 (s, 1 H). Yield 78%.

**3-((5-((4-(2-ethoxy-2-oxoethyl)phenyl)carbamoyl)-1-methyl-1 H-pyrrol-3-yl)carbamoyl)benzenaminium chloride (10j)** was synthesized according to the procedure described for compound (**10a**). ¹H-NMR 200 MHz (DMSO-*d*₆) δ: 1.19 (t, 3H, *J*=6.8 Hz), 3.62 (s, 2H), 3.86 (s, 3H), 4.09 (q, 2H, *J*=6.8 Hz), 5.29 (s, 2H), 7.13 (s, 1 H), 7.21 (d, 2H, *J*=7.2 Hz), 7.52-7.69 (m, 5H), 7.90-8.11 (m, 2H), 9.89 (s, 1 H), 10.16 (s, 1 H). Quantitative yield.

**Diethyl 2,2'-(((4,4'-((3,3'-(carbonylbis(azanediyl))bis(benzoyl))bis(azanediyl))bis(1-methyl-1H-pyrrole-2,2'-carbonyl))bis(azanediyl))bis(4,1-phenylene))diacetate (11j)** was synthesized according to the procedure described for compound (**11a**). ¹H-NMR 200 MHz (DMSO-*d*₆) δ: 1.19 (t, 6H, *J*=6.8 Hz), 3.62 (s, 4H), 3.86 (s, 6H), 4.09 (q, 4H, *J*=6.8 Hz), 5.29 (s, 2H), 7.13 (s, 2H), 7.21 (d, 4H, *J*=7.2 Hz), 7.52-7.69 (m, 10H), 7.90-8.11 (m, 4H), 9.89 (s, 2H), 10.16 (s, 2H). Yield 17%.

**2,2'-(((4,4'-((3,3'-(carbonylbis(azanediyl))bis(benzoyl))bis(azanediyl))bis(1-methyl-1H-pyrrole-2,2'-carbonyl))bis(azanediyl))bis(4,1-phenylene))diacetic** acid **SST0756AA1 (12j)** was synthesized according to the procedure described for compound (**12a**). ¹H-NMR 400 MHz (DMSO-*d*₆) δ: 3.62 (s, 4H), 3.86 (s, 6H), 7.18-7.26 (m, 4H), 7.39-7.69 (m, 12H), 8.01 (s, 2H), 9.79 (s, 2H), 9.92 (s, 2H), 10.40 (s, 2H), 12.32 (s, 2H). Quantitative yield. ¹³C-NMR 400 MHz (DMSO-*d*₆) δ: 36.16, 40.89, 105.71, 117.27, 118.32, 119.46, 120.51, 120.63, 120.79, 122.07, 122.72, 123.99, 128.26, 128.67, 135.18, 135.45, 139.31, 139.85, 152.64, 159.71, 163.82, 172.56. MS (ESI): [M+1]⁺= 812.04.

### EXAMPLES 13-18

### Synthesis of dipyrrole ureas

The preparation of new compounds (Scheme 14) entails the condensation of aminoacids **1a,b,d,e,f** with 2-(3-nitrophenyl)acetyl chloride **2**, to afford the intermediate **3a,b,d,e,f,h** in good yields. The latter nitro derivatives **3a,b,d,e,f,h** were transformed into the corresponding amine analogues **4a,b,d,e,f,h** in quantitative yield by catalytic hydrogenation (Pd on charcoal, 10%); the latter amines were condensed with **5** affording in good yields (70-89%)the nitro derivate **6a,b,d,e,f,h** in turn reduced to **7a,b,d,e,f,h** by catalytic hydrogenation; the resulting amino derivates **7a,b,d,e,f,h** were coupled with 1-methyl-4-nitro-1H-pyrrole-2-carbonyl chloride (**5**) to afford the nitro compounds **14a,b,d,e,f,h** subsequently reduced by catalytic hydrogenation to furnish the amino analogues **15a,b,d,e,f,h.** The subsequent reaction with CDI allowed the desired ureidic compounds **16a,b,d,e,f,h** in moderate yield (11%). Compounds **17a,b,d,e,f,h** were obtained after hydrolysis of esters **16a,b,d,e,f,h** with LiOH in THF.

Compound **18** was obtained by treatment of **17b** with an aqueous solution of NaOH (0.1 N).

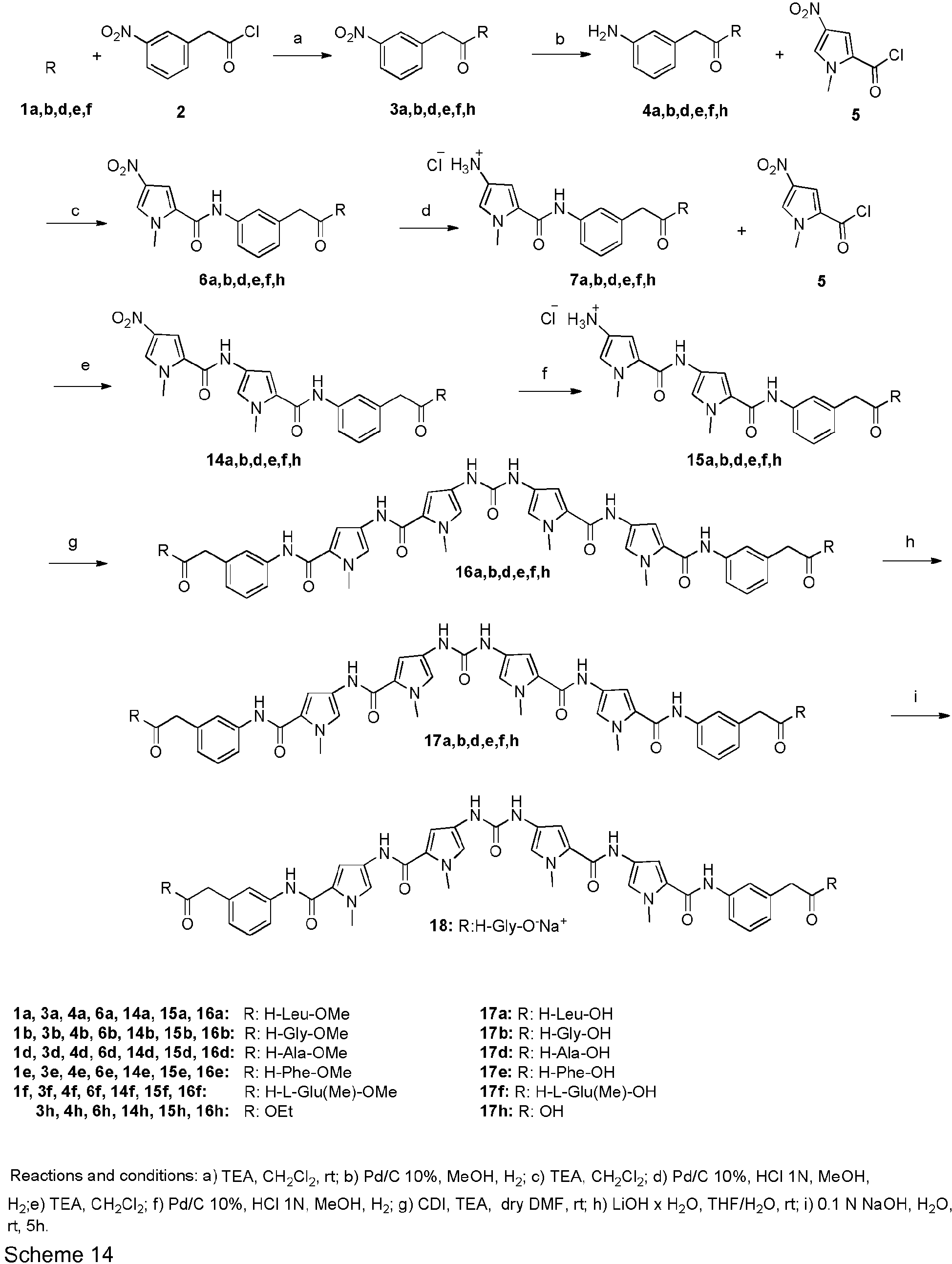

Preparation of each compound is disclosed more in details in the following.

### Example 13

### Preparation of compound SST0936AA1 (17a).

**Methyl *N*-[(3-{[(1-methyl-4-{[(1-methyl-4-nitro-1*H*-pyrrol-2-yl)carbonyl]amino}-1*H*-pyrrol-2-yl)carbonyl]amino}phenyl)acetyl]leucinate (14a).** To a solution of 5-({[3-(2-{[1-(methoxycarbonyl)-3-methylbutyl]amino}-2-oxoethyl)phenyl]amino}carbonyl)-1-methyl-1*H-*pyrrol-3-aminium chloride (**7a**) (486 mg, 1.12 mmol) and TEA (0.33 mL, 2.23 mmol) in CH₂Cl₂ (30 mL), was added slowly to a cold solution (5 °C) of 1-methyl-4-nitro-1 H-pyrrole-2-carbonyl chloride (**5**) (338 mg, 1.68 mmol) in CH₂Cl₂ (5 mL). After stirring over night at 25 °C, the reaction mixture was diluted with CH₂Cl₂ and water. The organic phase was washed for three times with 1 N HCl, saturated aqueous NaHCO₃, brine, dried over Na₂SO₄ and concentrated under reduced pressure. The residue was purified by column chromatography on silica gel column using EtOAc:light petroleum (40:60 v/v) as eluent to furnish the desired product methyl *N*-[(3-{[(1-methyl-4-{[(1-methyl-4-nitro-1*H*-pyrrol-2-yl)carbonyl]amino}-1*H*-pyrrol-2-yl)carbonyl]amino}phenyl)acetyl]leucinate (**14a**) (530 mg, 79%)._¹H-NMR 200 MHz (DMSO-*d*₆) δ: 0.82 (d, 3H, *J*=6.2 Hz), 0.89 (d, 3H, *J*=6.2 Hz), 1.52-1.63 (m, 3H), 3.44 (s, 2H), 3.61 (s, 3H), 3.86 (s, 3H), 3.97 (s, 3H), 4.22-4.27 (m, 1 H), 6.95 (d, 1 H, *J*=7.2 Hz), 7.13-7.33 (m, 3H), 7.51-7.68 (m, 3H),8.20 (d, 1H, *J*=1.6 Hz), 8.48 (d, 1H, *J*=7.6 Hz), 9.88 (s, 1H), 10.31 (s, 1H). MS (ESI): [M+1]⁺ = 553.39.

**5-({[5-({[3-(2-{[1-(methoxycarbonyl)-3-methylbutyl]amino}-2-oxoethyl)phenyl]amino}carbonyl)-1-methyl-1*H*-pyrrol-3-yl]amino}carbonyl)-1-methyl-1*H*-pyrrol-3-aminium chloride (15a).** To a solution of methyl *N*-[(3-{[(1-methyl-4-{[(1-methyl-4-nitro-1*H*-pyrrol-2-yl)carbonyl]amino}-1*H*-pyrrol-2-yl)carbonyl]amino}phenyl)acetyl]leucinate (**14a**) (265 mg, 0.65 mmol) in MeOH (110 mL) and DMF (15mL) was added 1 N HCl (0.65 mL, 0.65 mmol). The mixture of reaction was hydrogenated over 10% Pd/C (catalytic amount) for 4h. The catalyst was filtered off and the resulting solution was concentrated to afford 5-({[5-({[3-(2-{[1-(methoxycarbonyl)-3-methylbutyl]amino}-2-oxoethyl)phenyl]amino}carbonyl)-1-methyl-1*H*-pyrrol-3-yl]amino}carbonyl)-1-methyl-1*H-*pyrrol-3-aminium (**15a**) (363 mg, quantitative yield).
¹H-NMR 200 MHz (DMSO-*d*₆) δ: 0.81 (d, 3H, *J*=6.2 Hz), 0.87 (d, 3H, *J*=6.2 Hz), 1.53-1.65 (m, 3H), 3.42 (s, 2H), 3.65 (s, 3H), 3.86 (s, 3H), 3.97 (s, 3H), 4.22-4.27 (m, 1H), 6.95 (d, 1H, *J*=7.2 Hz), 7.13-7.33 (m, 3H), 7.51-7.68 (m, 3H),8.20 (d, 1H, *J*=1.6 Hz), 8.48 (d, 1H, *J*=7.6 Hz), 9.88 (s, 1 H), 10.22 (s, 3H), 10.31 (s, 1 H). MS (ESI): [M+1]⁺ = 523.49.

**Dimethyl 2,2'-{carbonylbis[imino(1-methyl-1*H*-pyrrole-4,2-diyl)carbonylimino(1-methyl-1*H*-pyrrole-4,2-diyl)carbonylimino-3,1-phenylene(1-oxoethane-2,1-diyl)imino]}bis(4-methylpentanoate) (16a).** To a solution of 5-({[5-({[3-(2-{[1-(methoxycarbonyl)-3-methylbutyl]amino}-2-oxoethyl)phenyl]amino}carbonyl)-1-methyl-1*H*-pyrrol-3-yl]amino}carbonyl)-1-methyl-1*H*-pyrrol-3-aminium chloride (**15a**) (157 mg, 0.28 mmol) in dry DMF (2 mL) was added TEA (0.08 mL, 0.56 mmol) and 0.25 equiv. of CDI (11.35 mg, 0.07 mmol) for 4 times every 30 min. The reaction mixture was stirred for 4 h at room temperature and then heated at 70 °C under Ar for 36 h. After being cooled at room temperature, the solvent was evaporated under vacuum and the residue was purified by column chromatography on silica gel column EtOAc:MeOH (98:2 v/v) as eluent to furnish the desired product dimethyl 2,2'-{carbonylbis[imino(1-methyl-1*H*-pyrrole-4,2-diyl)carbonylimino(1-methyl-1*H*-pyrrole-4,2-diyl)carbonylimino-3,1-phenylene(1-oxoethane-2,1-diyl)imino]}bis(4-methylpentanoate) (**16a**) (36 mg, 12%). ¹H-NMR 200 MHz (DMSO-*d*₆) δ: 0.82 (d, 3H, *J*=6.2 Hz), 0.89 (d, 3H, *J*=6.2 Hz), 1.53-1.65 (m, 3H), 3.42 (s, 2H), 3.61 (s, 3H), 3.84 (s, 6H), 4.01-4.04 (m, 1 H), 6.82-7.29 (m, 6 H), 7.42-7.49 (m, 2H), 7.69 (s, 1 H), 8.03 (s, 1 H), 8.45 (m, 1 H), 9.84 (s, 1H). MS (ESI): [M+1]⁺ = 1072.37.

**2,2'-{carbonylbis[imino(1-methyl-1*H*-pyrrole-4,2-diyl)carbonylimino(1-methyl-1*H-*pyrrole-4,2-diyl)carbonylimino-3,1-phenylene(1-oxoethane-2,1-diyl)imino]}bis(4-methylpentanoic acid) SST0936AA1 (17a).** To a suspension of dimethyl 2,2'-{carbonylbis[imino(1-methyl-1*H*-pyrrole-4,2-diyl)carbonylimino(1-methyl-1*H*-pyrrole-4,2-diyl)carbonylimino-3,1-phenylene(1-oxoethane-2,1-diyl)imino]}bis(4-methylpentanoate) (**16a**) (15 mg, 0.01 mmol) in THF (5 mL), was added LiOH·H₂O (2.35 mg, 0.06 mmol) and water (1.6 mL). After stirring over night at 25 °C, the solvent was concentrated under reduced pressure, the reaction mixture was diluted with water and then acidified with 1N HCI. The precipitate was filtered and wash with water to give 2,2'-{carbonylbis[imino(1-methyl-1*H-*pyrrole-4,2-diyl)carbonylimino(1-methyl-1*H*-pyrrole-4,2-diyl)carbonylimino-3,1-phenylene(1-oxoethane-2,1-diyl)imino]}bis(4-methylpentanoic acid) SST0936AA1 (**17a**) (8 mg, 57%). ¹H-NMR 400 MHz (DMSO-*d*₆) δ: 0.82 (d, 6H, *J*=6.4 Hz), 0.89 (d, 6H, *J*=6.4 Hz), 1.53-1.65 (m, 6H), 3.42 (s, 4H), 3.82- 3.85 (m, 12H), 4.20-4.21 (m, 2H), 6.82 (s, 2H), 6.95 (d, 2H, *J*=7.6 Hz), 7.03 (s, 2H), 7.13 (s, 2H), 7.19-7.23 (m, 2H), 7.30 (s, 2H), 7.53 (d, 2H, *J*=7.2 Hz), 7.69 (s, 2H), 8.13 (s, 2H), 8.35 (d, 2H, *J*=8 Hz), 9.84 (s, 2H), 9.85 (s, 2H), 12.43 (b, 2H).
¹³C-NMR (400 MHz, DMSO-*d*₆) δ: 21.22 (2), 22.76, 24.20, 36.11, 41.93, 50.19, 54.84, 103.84, 105.47, 117.25, 118.10, 119.02, 120.81, 122.10, 122.53 (2), 123.58, 128.10 (2), 136.47, 139.19, 152.48, 158.36, 159.68, 170.00, 174.08. MS (ESI): [M+1]⁺ = 1044.73. HPLC: 13.42 min.

### Example 14

### Preparation of compound SST0820NA1 (18)

**Methyl *N*-[(3-{[(1-methyl-4-{[(1-methyl-4-nitro-1*H*-pyrrol-2-yl)carbonyl]amino}-1*H***-pyrrol-**2-yl)carbonyl]amino}phenyl)acetyl]glycinate (14b)** was synthesized according to the procedure described for compound (**14a**). ¹H-NMR 200 MHz (DMSO-*d*₆) δ: 3.46 (s, 2H), 3.62 (s, 3H), 3.86 (m, 5H), 3.97 (s, 3H), 6.97 (d, 1H, J=7.0 Hz), 7.15-7.32 (m, 3H), 7.55-7.67 (m, 3H), 8.20 (s, 1 H), 8.50 (b, 1 H), 9.90 (s, 1 H), 10.32 (s, 1 H). Yield 75%. MS (ESI): [M+1]⁺ = 497.17.

**5-{[(5-{[(3-{2-[(2-methoxy-2-oxoethyl)amino]-2-oxoethyl}phenyl)amino]carbonyl}-1-methyl-1*H*-pyrrol-3-yl)amino]carbonyl}-1-methyl-1*H*-pyrrol-3-aminium chloride (15b)** was synthesized according to the procedure described for compound (**15a**). ¹H-NMR 200 MHz (DMSO-*d*₆) δ: 3.46 (s, 2H), 3.62 (s, 3H), 3.83-3.85 (m, 6H), 3.90 (s, 2H), 7.02-7.32 (m, 6H), 7.57-7.65 (m, 2H), 8.55 (b, 1H), 8.87 (s, 1H), 10.04 (s, 3H), 10.12 (s, 1H). Yield 91%. MS (ESI): [M+1]⁺ = 467.31.

**Dimethyl 2,2'-{carbonylbis[imino(1-methyl-1*H*-pyrrole-4,2-diyl)carbonylimino(1-methyl-1*H*-pyrrole-4,2-diyl)carbonylimino-3,1-phenylene(1-oxoethane-2,1-diyl)imino]}diethanoate (16b)** was synthesized according to the procedure described for compound (**16a**). ¹H-NMR 200 MHz (DMSO-*d*₆) δ: 3.46 (s, 2H), 3.66 (s, 3H), 3.85 (m, 8H), 6.83-7.30 (m, 7H), 7.54 (m, 1 H), 7.68 (s, 1 H), 8.21 (s, 1 H), 8.53 (s, 1 H), 9.85 (s, 1 H). Yield 10%. MS (ESI): [M+1]⁺ = 959.53.

**2,2'-{carbonylbis[imino(1-methyl-1*H*-pyrrole-4,2-diyl)carbonylimino(1-methyl-1*H-*pyrrole-4,2-diyl)carbonylimino-3,1-phenylene(1-oxoethane-2,1-diyl)imino]}diethanoic acid (17b)** was synthesized according to the procedure described for compound (**17a**). ¹H-NMR 400 MHz (DMSO-*d*₆) δ: 3.45 (s, 4H), 3.76 (d, 4H, *J*=6.0 Hz), 3.85 (s, 12H), 6.83 (s, 2H), 7.00 (d, 2H, *J*=7.2 Hz), 7.04 (s, 2H), 7.14 (s, 2H), 7.22 (m, 2H), 7.30 (s, 2H), 7.57 (d, 2H, *J*=8.4 Hz), 7.67 (s, 2H), 8.19 (s, 2H), 8.38 (s, 2H), 9.86 (m, 4H), 12.46 (b, 2H). Quantitative Yield. ¹³C-NMR (400 MHz, DMSO-*d*₆) δ: 28.94, 36.07, 36.14, 40.66, 42.03, 103.81, 105.49, 117.24, 118.16, 119.05, 120.05, 120.80, 122.13, 122.48 (2), 122.56, 123.75, 128.15, 136.28, 139.24, 152.49, 158.36, 159.70, 170.37, 171.25. MS (ESI): [M+1]⁺ = 931.78.

**Disodium 2,2'-{carbonylbis[imino(1-methyl-1*H*-pyrrole-4,2-diyl)carbonylimino(1-methyl-1*H*-pyrrole-4,2-diyl)carbonylimino-3,1-phenylene(1-oxoethane-2,1-diyl)imino]}diethanoate salt SST0820NA1 (18).** To a suspension of 2,2'-{carbonylbis[imino(1-methyl-1*H*-pyrrole-4,2-diyl)carbonylimino(1-methyl-1*H*-pyrrole-4,2-diyl)carbonylimino-3,1-phenylene(1-oxoethane-2,1-diyl)imino]}diethanoic acid (**17b**) (14 mg, 0.015 mmol) in water (2 mL) was added dropwise 0.1 N NaOH (0.30 mL, 0.03 mmol) at 0 °C. After stirring 5 h at 25 °C, the solvent was concentrated under reduced pressure to give disodium 2,2'-{carbonylbis[imino(1-methyl-1*H*-pyrrole-4,2-diyl)carbonylimino(1-methyl-1*H-*pyrrole-4,2-diyl)carbonylimino-3,1-phenylene(1-oxoethane-2,1-diyl)imino]}diethanoate salt (13 mg, quantitative yield) SST0820NA1 (**18**)._HPLC: 10.81 min.

### Example 15

### Preparation of compound SST0886AA1 (17d)

**Methyl *N*-[(3-{[(1-methyl-4-{[(1-methyl-4-nitro-1*H*-pyrrol-2-yl)carbonyl]amino}-1*H*-pyrrol-2-yl)carbonyl]amino}phenyl)acetyl]alaninate (14d)** was synthesized according to the procedure described for compound (**14a**). ¹H-NMR 200 MHz (DMSO-*d*₆) δ: 1.28 (d, 3H, *J*=7.4 Hz), 3.44 (s, 2H), 3.61 (s, 3H), 3.86 (s, 3H), 3.97 (s, 3H), 4.25-4.28 (m, 1H), 6.95 (d, 1H, *J*=7.6 Hz), 7.14-7.32 (m, 3H), 7.54-7.66 (m, 4H), 8.19 (s, 1H), 8.54 (d, 1H, *J*=7.0 Hz), 9.89 (s, 1 H), 10.31 (s, 1 H). Yield 88%.

**5-{[(5-{[(3-{2-[(2-methoxy-1-methyl-2-oxoethyl)amino]-2-oxoethyl}phenyl)amino]carbonyl}-1-methyl-1*H*-pyrrol-3-yl)amino]carbonyl}-1-methyl-1*H*-pyrrol-3-aminium chloride (15d)** was synthesized according to the procedure described for compound (**15a**). ¹H-NMR 200 MHz (DMSO-*d*₆) δ: 1.29 (d, 3H, *J*=7.4 Hz), 3.44 (s, 2H), 3.61 (s, 3H), 3.85 (s, 3H), 3.91 (s, 3H), 4.24-4.27 (m, 1H), 6.97-7.02 (m, 2H), 7.11-7.32 (m, 4H), 7.61-7.67 (m, 1H), 8.59 (m, 1H), 9.86 (s, 1H), 10.01 (s, 1H), 10.11 (s, 1H). Yield 92%. MS (ESI): [M+1]⁺ = 481.33.

**Dimethyl 2,2'-{carbonylbis[imino(1-methyl-1*H*-pyrrole-4,2-diyl)carbonylimino(1-methyl-1*H*-pyrrole-4,2-diyl)carbonylimino-3,1-phenylene(1-oxoethane-2,1-diyl)imino]}dipropanoate (16d)** was synthesized according to the procedure described for compound (**16a**). ¹H-NMR 200 MHz (DMSO-*d*₆) δ: 1.29 (d, 3H, J=7.4 Hz), 3.44 (s, 2H), 3.61 (s, 3H), 3.84 (s, 6H), 4.24-4.26 (m, 1 H), 6.84 (s, 1 H), 6.95 (d, 1 H, *J*=7.4 Hz), 7.03 (s, 1 H), 7.14-7.29 (m, 3H), 7.55 (d, 1H, *J*=8.4 Hz), 7.68 (s, 1H), 8.16 (s, 1H), 8.53 (d, 1H, *J*=6.8 Hz), 9.85 (s, 2H). Yield 45%. MS (ESI): [M+1]⁺ = 987.93.

**2,2'-{carbonylbis[imino(1-methyl-1*H*-pyrrole-4,2-diyl)carbonylimino(1-methyl-1*H-*pyrrole-4,2-diyl)carbonylimino-3,1-phenylene(1-oxoethane-2,1-diyl)imino]}dipropanoic acid SST0886AA1 (17d)** was synthesized according to the procedure described for compound (**17a**). ¹H-NMR 400 MHz (DMSO-*d*₆) δ: 1.27 (d, 6H, *J*=7.4 Hz), 3.44 (s, 4H), 3.61 (s, 6H), 3.84 (s, 6H), 4.24-4.26 (m, 2H), 6.82-7.30 (m, 12H), 7.55-7.66 (m, 4H), 8.17 (s, 2H), 8.41 (d, 2H, *J*=3.6 Hz), 9.85 (s, 2H), 9.86 (s, 2H), 12.61 (b, 2H). Quantitative Yield. ¹³C-NMR (400 MHz, DMSO-*d*₆) δ: 17.22, 30.33, 30.56, 36.07, 41.89, 47.48, 103.82, 105.49, 117.24, 118.13, 119.04, 120.80, 122.12, 122.50, 122.56, 123.69, 128.10, 136.39, 139.19, 152.50, 158.36, 159.71, 169.74, 174.11, 206.50. MS (ESI): [M+1]⁺ = 959.85. HPLC: 11.94 min.

### Example 16

### Preparation of compound SST0837AA1 (17e)

**Methyl *N*-[(3-{[(1-methyl-4-{[(1-methyl-4-nitro-1*H*-pyrrol-2-yl)carbonyl]amino}-1*H*-pyrrol-2-yl)carbonyl]amino}phenyl)acetyl]phenylalaninate (14e)** was synthesized according to the procedure described for compound (**14a**). ¹H-NMR 200 MHz (DMSO-*d*₆) δ: 2.91-3.32 (m, 2H), 3.40 (s, 2H), 3.59 (s, 3H), 3.86 (s, 3H), 3.97 (s, 3H), 4.38-4.54 (m, 1H), 6.83 (d, 1H, *J*=7.6 Hz), 7.13-7.25 (m, 7 H), 7.32 (d, 1H, *J*=1.6 Hz), 7.51-7.62 (m, 3 H), 8.20 (d, 1H, *J*=1.6 Hz), 8.55 (d, 1 H, *J*=7.8 Hz), 9.87 (s, 1 H), 10.31 (s, 1 H). Yield 71%. MS (ESI): [M+1]⁺= 587.64

**5-{[(5-{[(3-{2-[(1-benzyl-2-methoxy-2-oxoethyl)amino]-2-oxoethyl}phenyl)amino]carbonyl}-1-methyl-1*H*-pyrrol-3-yl)amino]carbonyl}-1-methyl-1*H*-pyrrol-3-aminium chloride (15e)** was synthesized according to the procedure described for compound (**15a**). ¹H-NMR 200 MHz (DMSO-*d*₆) δ: 2.87-3.03 (m, 2H), 3.41 (s, 2H), 3.85 (s, 3H), 3.86 (s, 3H), 4.41-4.46 (m, 1H), 6.80 (d, 1H, *J*=7.6 Hz), 6.99 (d, 1H, *J*=1.6 Hz), 7.10-7.30 (m, 9H), 7.50 (d, 1 H, *J*=8.4 Hz), 7.61 (s, 1 H), 8.56 (d, 1 H, *J*=7.6 Hz), 9.83 (s, 1 H), 10.02 (s, 3H), 10.11 (s, 1 H). Quantitative yield. MS (ESI): [M+1]⁺= 557.62

**Dimethyl 2,2'-{carbonylbis[imino(1-methyl-1*H*-pyrrole-4,2-diyl)carbonylimino(1-methyl-1*H*-pyrrole-4,2-diyl)carbonylimino-3,1-phenylene(1-oxoethane-2,1-diyl)imino]}bis(3-phenylpropanoate) (16e)** was synthesized according to the procedure described for compound (**16a**). ¹H-NMR 400 MHz (DMSO-*d*₆) δ: 2.91-3.02 (m, 2H), 3.40 (s, 2H), 3.59 (s, 3H), 3.83 (s, 3H) 3.85 (s, 3H), 4.43-4.48 (m, 1 H), 6.83 (b, 1 H), 7.03 (d, 1 H, *J*=1.6 Hz), 7.14-7.29 (m, 9H), 7.51-7.63 (m, 2H), 8.14 (s, 1H), 8.54 (d, 1H, *J*=7.8 Hz), 9.82 (s, 1H), 9.84 (s, 1 H). Yield 22%. MS (ESI): [M+1]⁺= 1140.12.

**2,2'-{carbonylbis[imino(1-methyl-1*H*-pyrrole-4,2-diyl)carbonylimino(1-methyl-1*H-*pyrrole-4,2-diyl)carbonylimino-3,1-phenylene(1-oxoethane-2,1-diyl)imino]}bis(3-phenylpropanoic acid) SST0837AA1 (17e)** was synthesized according to the procedure described for compound (**17a**). ¹H-NMR 400 MHz (DMSO-*d*₆) δ: 2.87-3.06 (m, 4H), 3.41 (s, 4H), 3.81 (s, 6H), 3.85 (s, 6H), 6.81-6.83 (m, 4H), 7.03 (d, 2H, *J*=1.6 Hz), 7.13-7.26 (m, 14H), 7.30 (d, 2H, *J*=1.6 Hz), 7.52-7.62 (m, 4H), 8.14 (s, 2H), 8.38 (d, 2H, *J*=8.0 Hz), 9.83 (s, 2H), 9.86 (s, 2H), 12.86 (s, 2H). Quantitative yield. ¹³C-NMR 400 MHz (DMSO-*d*₆) δ: 36.03, 36.11, 36.66, 41.99, 103.82, 105.45, 117.24, 118.10, 119.02, 120.91, 122.10, 122.49, 122.59, 123.60, 126.29(2), 128.05 (2), 129.05 (2), 136.21 (2), 137.39 (2), 139.12, 152.47, 158.34, 159.66, 169.85, 172.89. MS (ESI): [M+1]⁺= 1111.85. HPLC: 13.64 min.

### Example 17

### Preparation of compound SST0885AA1 (17f)

**Dimethyl *N*-[(3-{[(1-methyl-4-{[(1-methyl-4-nitro-1*H*-pyrrol-2-yl)carbonyl]amino}-1*H-*pyrrol-2-yl)carbonyl]amino}phenyl)acetyl]glutamate (14f)** was synthesized according to the procedure described for compound (**14a**). ¹H-NMR 200 MHz (DMSO-*d*₆) δ: 1.86-1.96 (m, 2), 2.36-2.43 (m, 2H), 3.45 (s, 2H), 3.57 (s, 3H), 3.62 (s, 3H), 3.86 (s, 3H), 3.94 (s, 3H), 4.26-4.28 (m, 1 H), 6.95 (d, 1 H, *J*=7.6 Hz), 7.14-7.33 (m, 3H), 7.52-7.61 (m, 2H), 7.69 (s, 1 H), 8.20 (d, 1 H, *J*=1.6 Hz), 8.53 (d, 1 H, *J*=7.6 Hz), 9.89 (s, 1 H), 10.32 (s, 1 H). Yield 60%. MS (ESI): [M+1]⁺ = 583.51.

**5-((5-((3-(2-((1,5-dimethoxy-1,5-dioxopentan-2-yl)amino)-2-oxoethyl)phenyl)carbamoyl)-1-methyl-1*H*-pyrrol-3-yl)carbamoyl)-1-methyl-1*H*-pyrrol-3-aminium chloride (15f)** was synthesized according to the procedure described for compound (**15a**). ¹H-NMR 200 MHz (DMSO-*d*₆) δ: 1.79-2.12 (m, 2H), 2.35-2.50 (m, 2H), 3.45 (s, 2H), 3.57 (s, 3H), 3.61 (s, 3H), 3.85 (s, 3H), 3.90 (s, 3H), 4.25-4.28 (m, 1H), 6.93-7.01 (m, 2H), 7.11-7.31 (m, 2H), 7.54 (d, 1H, *J*=7.8 Hz), 7.69 (s, 1H), 8.54 (7.6 Hz), 9.86 (s, 1H), 9.97 (b, 3H), 10.10 (s, 1H). Yield 92%. MS (ESI): [M+1]⁺ = 553.52.

**Tetramethyl 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}dipentandioate (16f)** was synthesized according to the procedure described for compound (**16a**). ¹H-NMR 200 MHz (DMSO-*d*₆) δ: 1.79-2.12 (m, 2H), 2.36-2.39 (m, 2H), 3.45 (s, 2H), 3.57 (s, 3H), 3.62 (s, 3H), 3.84 (s, 6H), 4.25-4.28 (m, 1 H), 6.82-7.29 (m, 6H), 7.52 (m, 2H), 7.70 (s, 1 H), 8.21 (s, 1H), 8.51 (d, 1H, *J*=7.8 Hz), 9.85 (s, 1H). Yield 18%. MS (ESI): [M+1]⁺ = 1131.56. HPLC: 12.20 min.

**2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}dipentandioic acid SST0885AA1 (17f)** was synthesized according to the procedure described for compound **(17a).** ¹H-NMR 200 MHz (DMSO-*d*₆) δ: 1.80-1.96 (m, 4H), 2.27-2.30 (m, 4H), 3.45 (s, 4H), 3.83 (s, 12H), 4.19-4.21 (m, 2H), 6.83 (s, 2H), 6.96 (d, 2H, *J*=8.0 Hz), 7.04 (s, 2H), 7.13 (s, 2H), 7.21-7.30 (m, 4H), 7.55 (d, 2H, *J*=8.4 Hz), 7.69 (s, 2H), 8.40 (d, 2H, *J*=8.0 Hz), 8.51 (s, 2H), 9.87 (d, 2H, *J*=12.4 Hz), 12.13 (b, 4H). Quantitative Yield. ¹³C-NMR (400 MHz, DMSO-*d*₆) δ: 26.36, 29.97, 36.13, 41.94, 51.18, 54.86, 103.64, 105.47, 117.11, 118.10, 119.02, 120.83, 122.16, 122.36, 122.57, 122.68, 123.60, 128.10, 136.39, 139.23, 152.58, 158.37, 159.72, 170.14, 173.24, 173.63. MS (ESI): [M+1]⁺ = 1075.83. HPLC: 11.89 min.

### Example 18

### Preparation of compound SST0760AA1 (17h)

**Ethyl (3-{[(1-methyl-4-{[(1-methyl-4-nitro-1*H*-pyrrol-2-yl)carbonyl]amino}-1*H*-pyrrol-2-yl)carbonyl]amino}phenyl)acetate (14h)** was synthesized according to the procedure described for compound (**14a**). ¹H-NMR 200 MHz (DMSO-*d*₆) δ: 1.19 (t, 3H, *J*=7.2 Hz), 3.62 (s, 2H), 3.86 (s, 3H), 3.97 (s, 3H), 4.08 (q, 2H, *J*=7.2 Hz), 6.94 (d, 1 H, *J*=7.6 Hz), 7.15 (d, 1 H, *J*=1.6 Hz), 7.25 (t, 1H, *J*=8.0 Hz), 7.32 (d, 1H, *J*=1.6 Hz), 7.59-7.62 (m, 2H), 7.68 (b, 1H), 8.19 (s, 1 H), 9.91 (s, 1 H), 10.31 (s, 1 H). Yield 49%.

**5-({[5-({[3-(2-ethoxy-2-oxoethyl)phenyl]amino}carbonyl)-1-methyl-1*H*-pyrrol-3-yl**]**amino**}**carbonyl**)-**1**-**methyl**-**1*H***-**pyrrol**-**3**-**aminium chloride (15h)** was synthesized according to the procedure described for compound (**15a**). ¹H-NMR 200 MHz (DMSO-*d*₆) δ: 1.16 (t, 3H, *J*=6.8 Hz), 3.61 (s, 2H), 3.82 (s, 3H), 3.87 (s, 3H), 4.05 (q, 2H, *J*=6.8 Hz), 6.90 (d, 1H, *J*=6.4 Hz), 7.05-7.30 (m, 5H), 7.57-7.66 (m, 2H), 9.88 (s, 1H), 10.18 (s, 1H), 10.28 (s, 3H). Quantitative yield.

**Diethyl 2,2'-{carbonylbis[imino(1-methyl-1*H*-pyrrole-4,2-diyl)carbonylimino(1-methyl-1*H*-pyrrole-4,2-diyl)carbonylimino-3,1-phenylene]}diacetate (16h)** was synthesized according to the procedure described for compound (**16a**). ¹H-NMR 200 MHz (DMSO-*d*₆) δ: 1.19 (t, 3H, *J*=7.2 Hz), 3.62 (s, 2H), 3.84 (s, 3H), 3.85 (s, 3H), 4.09 (q, 2H, *J*=7.2 Hz), 6.83(d, 1H, *J*=1.6 Hz), 6.94 (d, 1H, *J*=7.6 Hz), 7.03 (d, 1H, *J*=1.6 Hz), 7.15 (b, 1H), 7.23-7.30 (m, 2H), 7.60 (d,1H, *J*=8.4 Hz), 7.69 (b, 1 H), 8.12 (s, 1 H), 9.86 (s, 1 H), 9.87 (s, 1 H). Yield 26%.

**2,2'-{carbonylbis[imino(1-methyl-1*H*-pyrrole-4,2-diyl)carbonylimino(1-methyl-1*H-*pyrrole-4,2-diyl)carbonylimino-3,1-phenylene]}diacetic acid SST0760AA1 (17h)** was synthesized according to the procedure described for compound (**17a**). ¹H-NMR 400 MHz (DMSO-*d*₆) δ: 3.62 (s, 4H), 3.84 (s, 12H), 6.83 (d, 2H, *J*=1.6 Hz), 6.93 (d, 2H, *J*=7.6 Hz), 7.04 (d, 2H, *J*=2.0 Hz), 7.15(d, 2H, *J*=2.0 Hz), 7.23 (t, 2H, *J*=8.0 Hz), 7.30 (d, 2H, *J*=1.6 Hz), 7.58-7.69 (m, 4H), 8.56 (s, 2H), 9.87 (s, 2H), 9.89 (s, 2H), 12.25 (s, 2H). Quantitative yield. ¹³C-NMR 400 MHz (DMSO-*d*₆) δ: 22.80, 36.07, 40.93, 103.75, 105.62, 117.08, 118.34, 119.07, 120.80, 122.19, 122.49, 122.59, 122.67, 123.91, 128.23, 135.16, 139.37, 152.58, 158.41, 159.75, 172.46, 172.56.
MS (ESI): [M+1]⁺= 817.43. HPLC: 14.37 min.

### EXAMPLES 19-39

### Synthesis of diphenyl ethers, diphenylureas (alternative approach), dipyrrole ureas (alternative approach) and boronic compounds

The synthesis of new compounds is described in Schemes 15-20.

Key intermediates are the dicarboxylic acid derivatives **17h, 28,** and **12h** that were prepared as depicted in Schemes 16 and 17. Briefly, coupling of 3-aminopyrrole intermediate **22** [obtained by condensation of *tert*-butyl 2-(3-aminophenyl)acetate (**19**) with 1-methyl-4-nitropyrrole-2-carboxylic acid (**20**) (Ong et al., 2012) and subsequent hydrogenation of the nitro group, Scheme 15] with 1-methyl-4-nitropyrrole-2-carboxylic acid (**20**) in the presence of 1-ethyl-3-(3-dimethylaminopropyl)carbodiimide hydrochloride (EDC·HCI), hydroxybenzotriazole (HOBt), and triethylamine (TEA) gave the 3-nitropyrrole derivative **23** that was reduced to the corresponding amine **24** by catalytic hydrogenation.

Condensation of two equivalents of the 3-amino derivative **24** with carbonyldiimidazole, followed by *tert*-butyl ester deprotection with HCI in dioxane gave the key dicarboxylic acid **17h** (Scheme 16).

Compounds **28,** and **12h** were obtained by coupling reactions between the corresponding simmetrical dicarboxylic acids **26** or **29²** and the 3-aminopyrrole intermediate **22,** in the presence of EDC·HCl, HOBt, and TEA, and subsequent *tert*-butyl ester deprotection with HCl in dioxane (Scheme 17). Coupling reactions between the suitable *tert*-butyl ester aminoacids (**31a-f**) with properly activated dicarboxylic acids **17h, 28** and **12h,** followed by *tert*-butyl ester deprotection gave the desired target compounds **17a,b,d,e,f, 35c, 36a-f** and **12b** (scheme 18).

The dicarboxylic acids **17h, 28** and **12h** were also coupled with pinanediol leucine boronate (**37**) in the presence of HOBt, EDC and DIPEA, to provide the pinanediol ester derivatives **38-40.** Finally, the pinanediol intermediates underwent a trans-esterification reaction with phenylboronic acid under acidic conditions, to afford the desired boronic acid derivatives **41-43** (Scheme 19). In the same way were prepared the peptide boronates **46-47,** starting from the corresponding glycine-dicarboxylic acid derivative **17b** or **12b** instead of the dicarboxylic acids **17h, 28** and **12h** (Scheme 20). 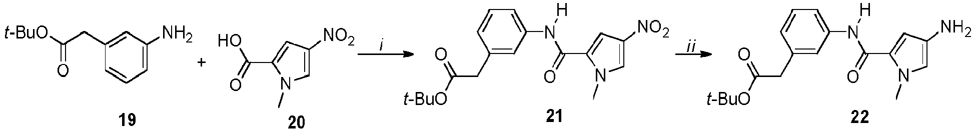 *Reagents and conditions: (i)* EDC, HOBt, TEA, dry DMF, r.t. 24 h; (*ii*) H₂ (4 atm), 10% Pd/C, EtOH, EtOAc, r.t., 4 h. 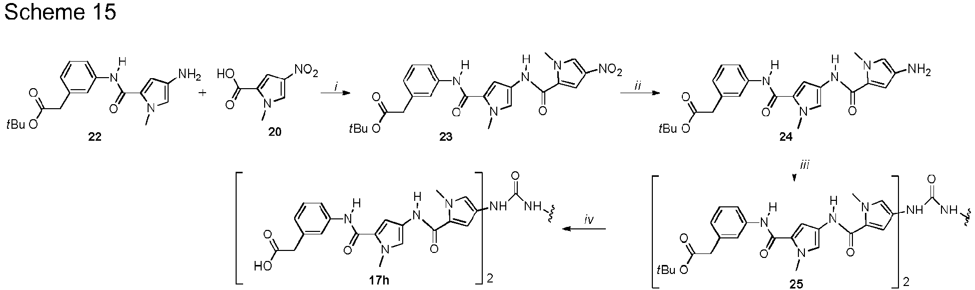 *Reagents and conditions:* (*i*) EDC, HOBt, TEA, dry DMF, r.t. 24 h; (*ii*) H₂ (4 atm), 10% Pd/C, MeOH, EtOAc, r.t. 5 h; (*iii*) CDI, THF, reflux 20 h; (*iv*) 4M HCl in dioxane, dioxane, r.t. 16 h. 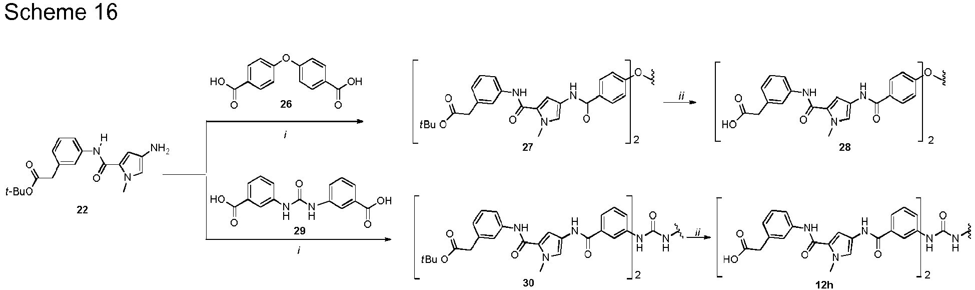 *Reagents and conditions:* (*i*) EDC, HOBt, TEA, dry DMF, r.t. 24 h; (*ii*) 4M HCl in dioxane, dioxane, r.t. 16 h. 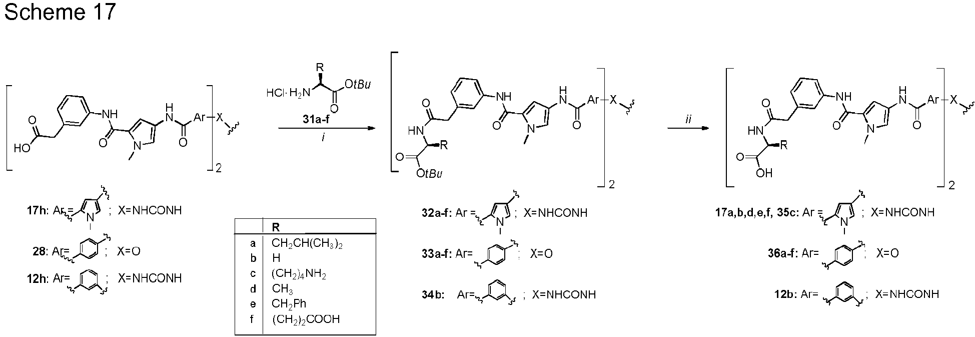 *Reagents and conditions: (i)* EDC, HOBt, TEA, dry DMF, r.t. 24 h (48 h for 32b and 33b); (*ii*) 4M HCl in dioxane, dioxane, r.t. 16 h. 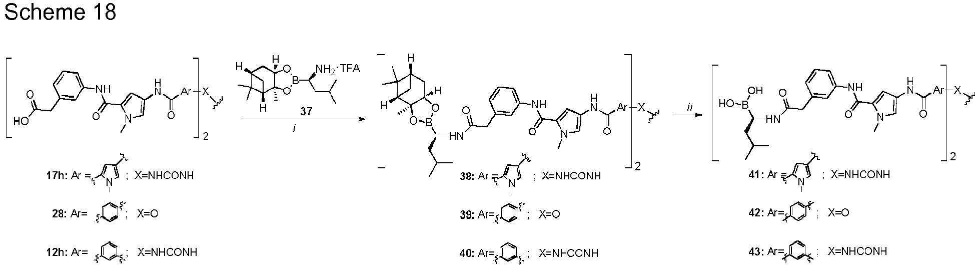 *Reagents and conditions: (i)* EDC, HOBt, DIPEA, dry DMF, r.t. 48 h; (*ii*) PhB(OH)₂, 1 M HCl, MeOH, *n*-hexane, r.t. 3 h. 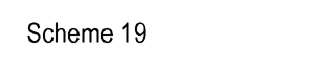 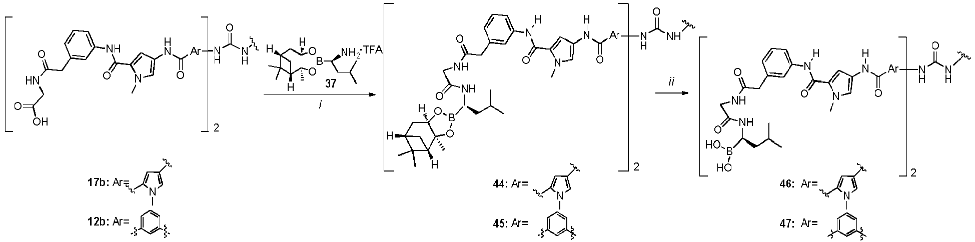 *Reagents and conditions*: (*i*) EDC, HOBt, DIPEA, dry DMF, r.t. 48 h; (*ii*) PhB(OH)₂, 1 M HCl, MeOH, n-hexane, r.t. 3 h.

### Scheme 20

**General procedure for the synthesis of amide substrates.** 1-Hydroxybenzotriazole hydrate (HOBt) (149 mg, 1.1 mmol), *N*-(3-Dimethylaminopropyl)-*N*'-ethylcarbodiimide hydrochloride (EDC) (230 mg, 1.2 mmol) and TEA (0.28 mL, 2 mmol) were added to a solution of the appropriate carboxylic acid **20, 17h, 26, 28-29** or **12h** (0.5 mmol) in dry DMF (3 mL), and the resulting mixture was stirred at room temperature for 30 minutes. Thereafter, the appropriate amine derivative **19, 22** or the amino ester derivative **31a-f** (1.2 mmol) was added and the reaction mixture was stirred at room temperature for 24 h (48 h for glycine *tert*-butyl ester hydrochloride (**31b**). EtOAc (15 mL) was added to the reaction mixture, the solution was washed in sequence with H₂O (1 x 15 mL), 5% citric acid (1 x 15 mL), brine (1 x 15 mL), 5% NaHCO₃ (1 x 15 mL), and brine (1 x 15 mL). The organic layer was dried over Na₂SO₄ and concentrated under reduced pressure.

**General procedure for *tert*-butyl ester deprotection.** A solution of 4 M HCl in dioxane (5 mL) was added dropwise to a solution of the appropriate *tert*-butyl ester derivatives **25, 27, 30, 32a-f, 33a-f** or **34b** (0.5 mmol), in dry dioxane (5 mL), and the resulting mixture was stirred at room temperature for 16 h. The solvent was removed by distillation under reduced pressure and the crude residue was triturated using a mixture of n-hexane/diethyl ether.

### Preparation of compound 22

***tert*-Butyl 2-(3-aminophenyl)acetate (19).** Boc₂O (4.82 mmol, 22.2 mmol) and DMAP (400 mg, 3.3 mmol) were added to a solution of 2-(3-nitrophenyl)acetic acid (2g, 11 mmol) in *tert-*butanol (50 mL) and the reaction mixture was stirred at room temperature for 1 h. After removal of the solvent by distillation under reduced pressure, the crude residue was purified by column flash chromatography (cyclohexane/ EtOAc 9:1) to give *tert*-butyl 2-(3-nitrophenyl)acetate (2.11 g, 81% yield) as yellowish oil.

A solution of *tert*-butyl 2-(3-nitrophenyl)acetate (2.11 g, 8.9 mmol) in EtOH (70 mL) was hydrogenated over 10% Pd/C (410 mg) at 4 atm of H₂ for 3 h at room temperature. The catalyst was filtered off over a celite plug and washed with ethanol. The filtrate was concentrated under reduced pressure to afford the crude desired amine (**19**), which was used in the next step without any further purification. ¹H NMR (400 MHz, CDCl₃): δ 1.45 (s, 9H), 3.44 (s, 2H), 3.55 (br s, 2H), 6.58 (dd, *J*₁ = 8.0, *J*₂ = 2.0 Hz, 1 H), 6.62 (s, 1 H), 6.67 (d, *J* = 7.5 Hz, 1H), 7.10 (t, *J* = 7.5 Hz, 1H). The physical-chemical properties were in agreement with those previously reported (Hama, T.; Ge, S.; Hartwig, J, F. J. Org. Chem. 2013, 78, 8250-8266).

***tert*-Butyl 2-(3-(1-methyl-4-nitro-1*H*-pyrrole-2-carboxamido)phenyl)acetate (21).** According to the above-described general procedure compound **19** was treated with the carboxylic acid derivative **20** to afford the title compound **21** (yield, 32%), that was used for the subsequent reaction without any further purification. MS (ESI): 377 [M + 18]⁺; ¹H NMR (400 MHz, CDCl₃): δ 1.47 (s, 9H), 3.56 (s, 2H), 4.04 (s, 3H), 7.05 (t, *J* = 7.5 Hz, 1 H), 7.22 (s, 1 H), 7.27-7.31 (m, 2H), 7.48 (s, 1 H), 7.61 (s, 1 H), 7.83 (br s, 1 H); ¹³C NMR (100 MHz, CDCl₃): δ 28.0, 38.0, 42.3, 81.4, 107.7, 118.8, 121.2, 125.6, 126.3, 127.2, 129.0, 134.9, 135.4, 137.6, 158.4, 171.3.

***tert*-Butyl 2-(3-(4-amino-1-methyl-1*H*-pyrrole-2-carboxamido)phenyl)acetate (22).** A solution of **21** (780 mg, 2.17 mmol) in EtOH (25 mL) and EtOAc (25 mL) was hydrogenated over 10% Pd/C (130 mg) at 4 atm of H₂ for 4 h at room temperature. The catalyst was filtered off over a celite plug and washed with ethanol. The filtrate was concentrated under reduced pressure to afford a crude residue, that was purified by column flash chromatography (EtOAc as eluent) to give **22** (73% yield) as yellowish solid. MS (ESI): 330 [M + 1]⁺; ¹H NMR (400 MHz, CDCl₃): δ 1.45 (s, 9H), 3.53 (s, 2H), 3.85 (s, 3H), 6.28 (s, 1 H), 6.36 (s, 1 H), 7.00 (d, *J* = 8.0 Hz, 1 H), 7.28 (t, *J* = 7.5 Hz, 1 H), 7.47 (s, 1 H), 7.48 (s, 1 H), 7.57 (br s, 1 H).

### Example 19

### Preparation of compound SST0760AA1 (17h)

***tert*-Butyl 2-(3-(1-methyl-4-(1-methyl-4-nitro-1*H*-pyrrole-2-carboxamido)-1*H*-pyrrole-2-carboxamido)phenyl)acetate (23).** According to the above-described general procedure the amine derivative **22** was treated with compound **20.** The title compound **23** was obtained as a yellow solid (85% yield) after purification by column flash chromatography (silica gel, cyclohexane/EtOAc 7:3 as eluent). MS (ESI): 482 [M + 1]⁺; ¹H NMR (400 MHz, acetone-*d*₆): δ 1.45 (s, 8H), 3.54 (s, 2H), 3.98 (s, 3H), 4.11 (s, 3H), 7.01 (ddd, *J*₁ = 1.0, *J*₂ = 1.5, *J*₃ = 7.5 Hz, 1 H), 7.11 (d, *J* = 2.0 Hz, 1 H), 7.28 (dd, *J*₁ = *J*₂ = 7.5 Hz, 1 H), 7.34 (d, *J* = 1.5 Hz, 1 H), 7.40 (dd, *J* = 2.0 Hz, 1 H), 7.69 (ddd, *J*₁ = 1.0, *J*₂ = 1.5, *J*₃ = 7.5 Hz, 1 H), 7.77 (dd, *J*₁ = *J*₂ = 1.5 Hz, 1 H), 7.97 (d, *J* = 1.5 Hz, 1 H), 9.21 (s, 1 H), 9.55 (s, 1H); ¹³C NMR (100 MHz, acetone-*d*₆): δ 27.3, 35.9, 37.2, 42.2, 79.9, 104.6, 106.9, 118.2, 119.2, 120.6, 122.0, 123.6, 124.0, 126.6, 127.4, 128.5, 135.6, 139.7, 157.3, 159.8, 170.2.

***tert*-Butyl 2-(3-(4-(4-amino-1-methyl-1*H*-pyrrole-2-carboxamido)-1-methyl-1*H*-pyrrole-2-carboxamido)phenyl)acetate (24).** A solution of **23** (240 mg, 0.5 mmol) in MeOH (5.8 mL) and EtOAc (2.6 mL) was hydrogenated over 10% Pd/C (24 mg) at 4 atm of H₂ for 4 h at room temperature. The catalyst was filtered off over a celite plug and washed with EtOAc. The filtrate was concentrated under reduced pressure to afford a crude residue, that was purified by column flash chromatography (CH₂Cl₂/MeOH 98 : 2) to give **24** (yield, 93%) as a red solid. MS (ESI): 452 [M + 1]⁺; ¹H NMR (400 MHz, acetone-*d*₆): δ 1.44 (s, 9H), 3.53 (s, 2H), 3.95 (s, 3H), 3.96 (s, 3H), 6.32 (dd, *J*₁ = 2.0, *J*₂ = 4.5 Hz, 2H), 6.56 (d, *J* = 2.0 Hz, 1 H), 6.62 (d, *J =* 2.0 Hz, 1H), 7.00 (ddd, *J*₁ = 1.0, *J*₂ = 1.5, *J*₃ = 8.0 Hz, 1H), 7.04 (dd, *J*₁ = 2.0, *J*₂ = 4.5 Hz, 1 H), 7.27 (dd, J₁ ≈ J₂ ≈ 8.0 Hz, 1 H), 7.28 (dd, *J*₁ = 2.0, *J*₂ = 4.5 Hz, 1 H), 7.69 (ddd, *J*₁ = 1.0, *J*₂ = 1.5, *J*₃ = 8.0 Hz, 1 H), 7.79 (dd, J₁ ≈ J₂ ≈ 1.5 Hz, 1 H), 9.16 (s, 1 H), 9.20 (s, 1 H); ¹³C NMR (100 MHz, acetone-*d*₆): δ 27.3, 35.8, 35.9, 42.2, 79.9, 104.7, 106.6, 118.2, 119.1, 120.6, 120.7, 122.7, 123.2, 123.9, 124.2, 128.5, 133.9, 135.6, 139.7, 158.9, 159.9, 170.3.

**Di-*tert*-butyl 2,2'-(((4,4'-((4,4'-(carbonylbis(azanediyl))bis(1-methyl-1*H*-pyrrole-4,2-diyl-2-carbonyl)) bis(azanediyl))bis(1-methyl-1*H*-pyrrole-4,2-diyl-2-carbonyl))bis(azanediyl))bis(3,1-phenylene))diacetate (25).** Carbonyldiimidazole (CDI) (154 mg, 0.95 mmol) was added to a solution of **24** (712 mg, 1.58 mmol) in dry THF (20 mL) and the resulting mixture was stirred under reflux for 20 h. The solvent was removed by distillation under reduced pressure and the crude residue was purified by by column flash chromatography (silica gel, CH₂Cl₂/MeOH 97 : 3 as eluent). (CH₂Cl₂/MeOH 97 : 3) to give **25** (yield, 85%) as an orange solid. MS (ESI): 929 [M + 1]⁺; ¹H NMR (400 MHz, acetone-*d*₆): δ 1.45 (s, 16H), 3.53 (s, 4H), 3.94 (s, 6H), 3.95 (s, 6H), 6.73 (d, *J* = 2.0 Hz, 2H), 7.00 (ddd, *J*₁ = 1.0, *J*₂ = 1.5, *J*₃ = 8.0 Hz, 2H), 7.04 (d, *J* = 2.0 Hz, 2H), 7.07 (d, *J* = 2.0 Hz, 2H), 7.27 (dd, J₁ ≈ J₂ = 8.0 Hz, 2H), 7.30 (d, *J* = 2.0 Hz, 2H), 7.60 (br s, 2H), 7.69 (ddd, *J*₁ = 1.0, *J*₂ = 1.5, *J*₃ = 8.0 Hz, 2H), 7.78 (dd, *J*₁ ≈ *J*₂ ≈ 1.5 Hz, 2H), 9.18 (s, 2H), 9.21 (s, 2H); ¹³C NMR(100 MHz, acetone-*d*₆): δ 27.3, 35.75; 35.8, 35.9, 42.2, 79.9, 104.1, 104.6, 118.2, 119.0, 120.6, 122.9, 123.1, 123.2, 123.3, 123.9, 128.4, 135.6, 139.7 158.7, 159.9, 170.2.

**2,2'-(((4,4'-((4,4'-(Carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))diacetic acid SST0760AA1 (17h).** According to the above-described ester deprotection general procedure the title compound **17h** was obtained as a brown solid (nearly quantitative yield) starting from compound **25,** and it was used for the subsequent reaction without any further purification.

### Example 20

### Preparation of compound 28

**Di-*tert*-butyl 2,2'-(((4,4'-((4,4'-oxybis(benzoyl))bis(azanediyl))bis(1-methyl-1*H*-pyrrole-2,2'-carbonyl))bis(azanediyl))bis(3,1-phenylene))diacetate (27).** According to the above-described general procedure the amine derivative **22** was treated with compound **26.** The title compound **27** was obtained as a white solid (75% yield) after purification by column flash chromatography (silica gel, cyclohexane/EtOAc 1:1 as eluent). MS (ESI): 881 [M + 1]⁺; ¹H NMR (400 MHz, MeOH-*d₄*): δ 1.46 (s, 18H), 3.54 (s, 4H), 3.92 (s, 6H), 7.02 (ddd, *J*₁ = 1.0, *J*₂ = 2.0, *J*₃=7.5 Hz, 2H), 7.08 (d, *J* = 2.0 Hz, 2H), 7.12-7.16 (m, 4H), 7.29 (dd, *J*₁ = *J*₂ = 7.5 Hz, 2H), 7.35 (d, *J* = 2.0 Hz, 2H), 7.52-7.55 (m, 2H), 7.58-7.59 (m, 2H), 7.95-7.98 (m, 4H); ¹³C NMR (100 MHz, MeOH-*d₄*): δ 26.9, 35.5, 42.1, 80.7, 105.7, 118.4, 119.1, 120.2, 121.3, 122.0, 123.4, 124.5, 128.4, 129.3, 129.9, 135.4, 138.7, 159.3, 160.9, 165.1, 171.4.

**2,2'-(((4,4'-((4,4'-Oxybis(benzoyl))bis(azanediyl))bis(1-methyl-1*H*-pyrrole-2,2'-carbonyl))bis(azanediyl))bis(3,1-phenylene))diacetic acid (28).**
According to the above-described ester deprotection general procedure the title compound **28** was obtained as a brown solid (nearly quantitative yield) starting from compound **27,** and it was used for the subsequent reaction without any further purification. MS (ESI): 769 [M+1]⁺; ¹H NMR (400 MHz, DMSO-d₆): δ 3.54 (s, 4H), 3.89 (s, 6H), 6.95 (d, *J =* 7.5 Hz, 2H), 7.19-7.27 (m, 8H), 7.39 (d, *J* = 2.0 Hz, 2H), 7.59-7.62 (m, 2H), 7.69-7.70 (m, 2H), 8.05 (d, *J* = 9.0 Hz, 4H), 9.87 (s, 2H), 10.33 (s, 2H).

### Example 21

### Preparation of compound SST0754AA1 (12h)

**Di-*tert*-butyl 2,2'-(((4,4'-((3,3'-(carbonylbis(azanediyl))bis(benzoyl))bis(azanediyl))bis(1-methyl-1*H*-pyrrole-4,2-diyl-2-carbonyl))bis(azanediyl))bis(3,1-phenylene))diacetate (30).** According to the above-described general procedure the amine derivative **22** was treated with compound **29.** The title compound **30** was obtained as an orange solid (84% yield) after purification by column flash chromatography (silica gel, cyclohexane/EtOAc 2:8 as eluent). MS (ESI): 923 [M + 1]⁺; ¹H NMR (400 MHz, DMSO-*d₆*): δ 1.41 (s, 18H), 3.52 (s, 4H), 3.89 (s, 6H), 6.94 (d, *J* = 7.5 Hz, 2H), 7.19 (t, *J* = 7.5 Hz, 2H), 7.25 (t, *J* = 7.5 Hz, 2H), 7.38 (d, *J =* 2.0 Hz, 2H), 7.44 (7, *J* = 7.5 Hz, 2H), 7.56 - 7.69 (m, 8H), 8.02 - 8.03 (m, 1 H), 8.95 (s, 2H), 9.85 (s, 2H), 10.29 (s, 2H); ¹³C NMR (100 MHz, DMSO-*d₆*): δ 28.2, 36.6, 42.4, 80.6, 106.4, 119.0, 120.0, 121.2, 121.3, 121.5, 122.7, 123.4, 124.3, 128.8, 129.2, 135.5, 136.0, 139.9, 140.1, 153.1, 160.3, 164.4, 170.8.

**2,2'-(((4,4'-((3,3'-(Carbonylbis(azanediyl))bis(benzoyl))bis(azanediyl))bis(1-methyl-1*H-*pyrrole-4,2-diyl-2-carbonyl))bis(azanediyl))bis(3,1-phenylene))diacetic acid SST0754AA1 (12h).** According to the above-described ester deprotection general procedure the title compound **12h** was obtained as a brown solid (nearly quantitative yield) starting from compound **30,** and it was used for the subsequent reaction without any further purification.

### Example 22

### Preparation of compound 17b

**Di-*tert*-butyl 2,2'-((2,2'-(((4,4'-((4,4'-(carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))diacetate (32b).** According to the above-described general procedure compound **17h** was treated with glycine *tert*-butyl ester hydrochloride (**31b**). The title compound **32b** was obtained as a pink solid (38% yield) after purification by column flash chromatography (silica gel, CH₂Cl₂/MeOH 95:5 as eluent). MS (ESI): 932 [M + 1]⁺; ¹H NMR (400 MHz, acetone-*d*₆): δ 1.41 (s, 18H), 3.56 (s, 4H), 3.85 (d, *J* = 5.5 Hz, 4H), 3.89 (s, 6H), 3.92 (s, 6H), 6.75 (br s, 2H), 6.98 (br s, 2H), 7.03-7.05 (m, 4H), 7.24 (dd, *J*₁ = *J*₂ = 8.0 Hz, 2H), 7.29 (br s, 2H), 7.42 (dd, *J*₁ = *J*₂ = 5.5 Hz, 2H), 7.67-7.71 (m, 6H), 9.19 (s, 4H); ¹³C NMR (100 MHz, acetone-*d*₆): δ 14.4, 25.5, 31.4, 36.6, 41.2, 104.4, 106.1, 117.8, 118.8, 119.6, 121.4, 122.7, 123.1, 123.2, 124.3, 128.7, 136.8, 139.8, 153.1, 158.9, 160.3, 170.9, 171.7.

**2,2'-((2,2'-(((4,4'-((4,4'-(Carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))diacetic acid (17b).** According to the above-described ester deprotection general procedure the title compound **17b** was obtained as a beige solid (nearly quantitative yield) starting from compound **32b.**

### Example 23

### Preparation of compound SST0886AA1 (17d).

**Di-*tert*-butyl 2,2'-((2,2'-(((4,4'-((4,4'-(carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))(2S,2'S)-dipropionate (32d).** According to the above-described general procedure compound **17h** was treated with L-alanine *tert*-butyl ester hydrochloride **(31d).** The title compound **32d** was obtained as a pink solid (41% yield) after purification by column flash chromatography (silica gel, CH₂Cl₂/MeOH 95:5 as eluent). MS (ESI): 960 [M + 1]⁺; ¹H NMR (400 MHz, acetone-*d*₆): δ 1.17 (d, *J* = 7.0 Hz, 6H), 1.25 (s, 18H), 3.38 (s, 4H), 3.71 (s, 6H), 3.74 (s, 6H), 4.14-4.19 (m, 2H), 6.61 (br s, 2H), 6.77 (br s, 2H), 6.86-6.89 (m, 4H), 7.06 (dd, *J*₁ = *J*₂ = 8.0 Hz, 2H), 7.13 (br s, 2H), 7.36 (d, *J* = 7.0 Hz, 2H), 7.51 (d, *J* = 8.0 Hz, 2H), 7.57 (br s, 2H), 9.02 (s, 4H); ¹³C NMR (100 MHz, acetone-*d*₆): δ 17.2, 27.2, 35.8, 35.9, 42.6, 48.8, 80.6, 104.8, 118.2, 119.2, 120.9, 122.6, 123.2, 123.3, 124.0, 136.4, 139.6, 158.8, 159.9, 170.1, 171.9.

**(2*S*,2'*S*)-2,2'-((2,2'-(((4,4'-((4,4'-(Carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))dipropionic acid SST0886AA1 (17d).** According to the above-described ester deprotection general procedure the title compound **17d** was obtained as a beige solid (nearly quantitative yield) starting from compound **32d.**

### Example 24

### Preparation of compound SST0936AA1 (17a)

**Di-*tert*-butyl 2,2'-((2,2'-(((4,4'-((4,4'-(carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))(2*S*,2'*S*)-bis(4-methylpentanoate) (32a).** According to the above-described general procedure compound **17h** was treated with L-leucine *tert*-butyl ester hydrochloride (**31a**). The title compound **32a** was obtained as a pink solid (31% yield) after purification by column flash chromatography (silica gel, CH₂Cl₂/MeOH 95:5 as eluent). MS (ESI): 1156 [M + 1]⁺; ¹H NMR (400 MHz, acetone-*d*₆): δ 0.73 (d, *J* = 6.5 Hz, 6H), 0.75 (d, *J* = 6.5 Hz, 6H), 1.25 (s, 18H), 1.40-1.44 (m, 4H), 1.52-1.62 (m, 2H), 3.39 (br s, 4H), 3.70 (s, 6H), 3.73 (s, 6H), 4.21-4.27 (m, 2H), 6.61 (br s, 2H), 6.76 (br s, 2H), 6.87-6.89 (m, 4H), 7.06 (dd, *J*₁ ≈ *J*₂ = 8.0 Hz, 2H), 7.13 (br s, 2H), 7.33 (d, *J* = 8.0 Hz, 2H), 7.48 (d, *J* = 8.0 Hz, 2H), 7.55 (br s, 2H), 7.60 (br s, 2H), 9.03 (br s, 4H); ¹³C NMR (100 MHz, acetone-*d*₆): δ 21.2, 22.3, 24.6, 27.3, 35.8 35.9, 40.8, 42.7, 51.6, 80.6, 104.8, 118.2, 119.3, 120.9, 122.6, 123.2, 123.3, 123.9, 128.4, 136.5, 139.6, 158.9, 159.9, 170.3, 171.8.

**(2*S*,2'*S*)-2,2'-((2,2'-(((4,4'-((4,4'-(Carbonylbis(azanediyl))bis(1-methyl-1*H*-pyrrole-4,2-diyl-2**-**carbonyl**))**bis**(**azanediyl**))**bis**(**1**-**methyl**-**1*H***-**pyrrole**-**4**,**2**-**diyl**-**2-carbonyl))bis(azanediyl))bis(3,1-phenylene))bis(acetyl))bis(azanediyl))bis(4-methylpentanoic acid) SST0936AA1 (17a).** According to the above-described ester deprotection general procedure the title compound **17a** was obtained as a beige solid (nearly quantitative yield) starting from compound **32a.**

### Example 25

### Preparation of compound SST0837AA1 (17e)

**Di-*tert*-butyl 2,2'-((2,2'-(((4,4'-((4,4'-(carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))(2*S*,2'*S*)-bis(3-phenylpropanoate) (32e).** According to the above-described general procedure compound **17h** was treated with L-phenylalanine *tert*-butyl ester hydrochloride (**31e**). The title compound **32e** was obtained as a pink solid (52% yield) after purification by column flash chromatography (silica gel, CH₂Cl₂/MeOH 95:5 as eluent). MS (ESI): 1225 [M + 1]⁺; ¹H NMR (400 MHz, acetone-*d*₆): δ 1.23 (s, 18H), 2.83-2.94 (m, 4H), 3.39 (dd, *J*₁ = 14.0, *J*₂ = 19.0 Hz, 4H), 3.78 (s, 6H), 3.81 (s, 6H), 4.46 (dd, *J*₁ = 7.5, *J*₂ = 14.0 Hz, 2H), 6.60 (d, *J* = 1.5 Hz, 2H), 6.84 (d, *J* = 7.5 Hz, 2H), 6.87 (d, *J* = 7.5 Hz, 2H), 6.93 (d, *J* = 1.5 Hz, 2H), 7.01-7.13 (m, 14H), 7.16 (d, *J =* 1.5 Hz, 2H), 7.47 (br s, 2H), 7.55 (d, *J* = 7.5 Hz, 2H), 7.59 (br s, 2H), 9.04 (s, 2H), 9.06 (s, 2H), ¹³C NMR (100 MHz, acetone-*d*₆): δ 27.2, 35.8; 35.9, 37.5, 42.8, 54.1, 80.9, 104.6, 118.2, 119.0, 120.73, 120.74, 122.8, 123.2, 124.0, 126.5, 128.2, 128.5, 129.4, 136.4, 137.1, 169.7, 170.4.

**(2S,2'*S*)-2,2'-((2,2'-(((4,4'-((4,4'-(Carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))bis(3-phenylpropanoic acid) SST0837AA1 (17e).** According to the above-described ester deprotection general procedure the title compound **17e** was obtained as a beige solid (nearly quantitative yield) starting from compound **32e.**

### Example 26

### Preparation of compound SST0885AA1 (17f)

**Tetra-*tert*-butyl 2,2'-((2,2'-(((4,4'-((4,4'-(carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))(2*S*,2'*S*)-diglutarate (32f).** According to the above-described general procedure compound **17h** was treated with L-glutamic acid di-*tert*-butyl ester hydrochloride (**31f**). The title compound **32f** was obtained as a pink solid (44% yield) after purification by column flash chromatography (silica gel, CH₂Cl₂/MeOH 95:5 as eluent). MS (ESI): 1300 [M + 1]⁺; ¹H NMR (400 MHz, acetone-*d*₆): δ 1.41 (s, 36H), 1.85-1.90 (m, 4H), 2.30-2.35 (m, 4H), 3.56 (br s, 4H), 3.85 (s, 6H), 3.88 (s, 6H), 4.37-4.41 (m, 2H), 6.76 (br s, 2H), 6.91 (br s, 2H), 7.03 (d, *J* = 8.0 Hz, 2H), 7.03 (br s, 2H), 7.22 (dd, *J*₁ = *J*₂ = 8.0 Hz, 2H), 7.28 (br s, 2H), 7.56 (d, *J* = 7.5 Hz, 2H), 7.64 (d, *J* = 8.0 Hz, 2H), 7.71 (br s, 2H), 7.74 (br s, 2H), 9.17 (s, 4H); ¹³C NMR (100 MHz, acetone-*d*₆): δ 27.1, 27.3, 27.4, 31.2, 35.86, 35.92, 42.7, 52.4, 54.1, 79.7, 81.0, 104.9, 118.3, 119.3, 120.9, 122.6, 123.2, 123.3, 123.9, 128.5, 136.4, 139.6, 158.9, 159.9, 170.5, 171.0, 171.6.

**(2*S*,2'*S*)-2,2'-((2,2'-(((4,4'-((4,4'-(Carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))bis(5-(tert-butoxy)-5-oxopentanoic acid) SST0885AA1 (17f).** According to the above-described ester deprotection general procedure the title compound **17f** was obtained as a yellowish solid (nearly quantitative yield) starting from compound **32f.**

### Example 27

### Preparation of compound SST0902AA1 (35c)

**Tetra-*tert*-butyl 2,2'-((2,2'-(((4,4'-((4,4'-(carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))(2*S*,2'*S*)-diglutarate (32c).** According to the above-described general procedure compound **17h** was treated with Nepsilon-Boc-L-lysine *tert*-butyl ester hydrochloride (**31c**). The title compound **32c** was obtained as a pink solid (45% yield) after purification by column flash chromatography (silica gel, CH₂Cl₂/MeOH 95:5 as eluent). MS (ESI): 1075 [M + 1]⁺; ¹H NMR (400 MHz, acetone-*d*₆): δ 1.40 (s, 18H), 1.41 (s, 18H), 1.63-1.71 (m, 2H), 1.75-1.83 (m, 2H), 2.95-3.04 (m, 4H), 3.56 (s, 4H), 3.86 (s, 6H), 3.89 (s, 6H), 4.30-4.35 (m, 2H), 5.99 (dd, *J*₁ = *J*₂ = 5.5 Hz, 2H), 6.76 (br s, 2H), 6.92 (br s, 2H), 7.02 (br s, 2H), 7.04 (d, *J* = 8.0 Hz, 2H), 7.23 (dd, *J*₁ = *J*₂ = 8.0 Hz, 2H), 7.29 (br s, 2H), 7.48 (d, *J* = 8.0 Hz, 2H), 7.64 (d, *J* = 7.5 Hz, 2H), 7.70 (br s, 2H), 7.74 (br s, 2H), 9.18 (s, 4H); ¹³C NMR (100 MHz, acetone-*d*₆): δ 22.6, 27.3, 27.8, 31.5, 35.8, 35.9, 39.9, 42.7, 53.0, 77.5, 80.7, 104.8, 118.3, 119.3, 120.9, 122.6, 123.2, 123.3, 124.0, 128.5, 136.5, 139.6, 155.9, 158.8, 160.0, 170.3, 171.3.

**(2*S*,2'*S*)-2,2'-((2,2'-(((4,4'-((4,4'-(carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))bis(6-aminohexanoic acid) dihydrochloride SST0902AA1 (35c).** According to the above-described ester deprotection general procedure the title compound **35c** was obtained as a beige solid (nearly quantitative yield) starting from compound **32c.** MS (ESI): 1073 [M+1]⁺; ¹H NMR (400 MHz, DMSO-*d₆*): δ 1.30-1.41 (m, 4H), 1.48-1.57 (m, 4H), 1.60-1.66 (m, 2H), 1.69-1.78 (m, 2H), 2.67-2.79 (m, 4H), 3.47 (dd, *J*₁ = 12.0, *J*₂ = 19.0 Hz, 4H), 3.85 (s, 6H), 3.86 (s, 6H), 3.97-4.08 (m, 4H), 4.16-4.22 (m, 2H), 3.83 (d, J = 1.5 Hz, 2H), 6.97 (d, *J* = 7.5 Hz, 2H), 7.04 (d, *J=* 1.5 Hz, 2H), 7.17 (d, *J=* 1.5 Hz, 2H), 7.22 (dd, *J*₁ = *J*₂ = 7.5 Hz, 2H), 7.28 (d, *J =* 1.5 Hz, 2H), 7.52 (d, *J* = 7.5 Hz, 2H), 7.73 (br s, 2H), 8.22 (br s, 2H), 8.35 (d, *J* = 8.0 Hz, 2H), 9.83 (s, 2H), 9.84 (s, 2H), 12.59 (br s, 2H); ¹³C NMR (100 MHz, DMSO-*d₆*): δ 15.6, 22.8, 27.0, 31.0, 42.5, 52.2, 65.4, 104.3, 106.1, 117.7, 118.7, 119.6, 121.4, 122.8, 123.0, 123.2,123.3, 124.2, 128.6, 137.0, 139.8, 153.2, 159.0, 160.3, 170.6, 174.0.

### Example 28

### Preparation of compound SST0907AA1 (36b)

**Di-*tert*-butyl 2,2'-((2,2'-(((4,4'-((4,4'-oxybis(benzoyl))bis(azanediyl))bis(1-methyl-1*H-*pyrrole-2,2'-carbonyl))bis(azanediyl))bis(3,1-phenylene))bis(acetyl))bis(azanediyl))diacetate (33b).** According to the above-described general procedure compound **28** was treated with glycine *tert*-butyl ester hydrochloride (**31b**). The title compound **33b** was obtained as off-white solid (23% yield) after purification by column flash chromatography (silica gel, gradient from CH₂Cl₂/MeOH 98:2 to CH₂Cl₂/MeOH 95:5 as eluent). MS (ESI): 995 [M + 1]⁺; ¹H NMR (400 MHz, DMSO-*d₆*): δ 1.40 (s, 18H), 3.46 (s, 4H), 3.74 (d, *J* = 6.0 Hz, 4H), 3.89 (s, 6H), 6.97-7.00 (m, 2H), 7.19-7.26 (m, 8H), 7.58 (ddd, *J*₁ = 1.0, *J*₂ = 2.0, *J*₃ = 8.0 Hz, 2H), 7.68-7.69 (m, 2H), 8.03-8.06 (m, 4H), 8.32 (t, *J* = 6.0 Hz, 2H), 9.86 (s, 2H), 10.32 (s, 2H); ¹³C NMR (100 MHz, DMSO-*d₆*): δ 28.2, 36.7, 42.0, 42.6, 81.0, 106.2, 118.9, 120.0, 121.4, 122.6, 123.37, 123.41, 124.3, 128.7, 130.2, 130.6, 136.8, 139.7, 158.9, 160.2, 163.4, 169.4, 170.9.

**2,2'-((2,2'-(((4,4'-((4,4'-Oxybis(benzoyl))bis(azanediyl))bis(1-methyl-1*H*-pyrrole-2,2'-carbonyl))bis(azanediyl))bis(3,1-phenylene))bis(acetyl))bis(azanediyl))diacetic acid SST0907AA1 (36b).** According to the above-described ester deprotection general procedure the title compound **36b** was obtained as a off-white solid (nearly quantitative yield) starting from compound **33b.** MS (ESI): 883 [M+1]⁺; ¹H NMR (400 MHz, DMSO-*d₆*): δ 3.47 (s, 4H), 3.77 (s, 4H), 3.88 (s, 6H), 6.97-7.00 (m, 2H), 7.19-7.24 (m, 8H), 7.39 (d, *J* = 4.0 Hz, 2H), 7.57-7.60 (m, 2H), 7.67 (s, 2H), 8.03-8.06 (m, 4H), 8.32 (d, *J* = 6.0 Hz, 2H), 9.86 (s, 2H), 10.34 (s, 2H); ¹³C NMR (100 MHz, DMSO-*d₆*): δ 26.8, 36.7, 42.6, 118.8, 118.9, 120.0, 121.4, 122.6, 123.4, 124.4, 128.7, 130.2, 130.6, 136.8, 139.7, 158.9, 160.2, 163.4, 170.9, 171.7.

### Example 29

### Preparation of compound SST0830AA1 (36d).

**(2*S*,2'*S*)-Di-tert-butyl 2,2'-((2,2'-(((4,4'-((4,4'-oxybis(benzoyl))bis(azanediyl))bis(1-methyl-1*H*-pyrrole-2,2'-carbonyl))bis(azanediyl))bis(3,1-phenylene))bis(acetyl))bis(azanediyl))dipropanoate (33d).** According to the above-described general procedure compound **28** was treated with L-alanine *tert*-butyl ester hydrochloride (**31d**). The title compound **33d** was obtained as off-white solid (30% yield) after purification by column flash chromatography (silica gel, gradient from cyclohexane/EtOAc 1:9 to EtOAc as eluent). MS (ESI): 1023 [M + 1]⁺; ¹H NMR (400 MHz, MeOH-*d₄*): δ 1.31 (d, *J =* 7.0 Hz, 6H), 1.42 (s, 18H), 3.49 (s, 4H), 3.93 (s, 6H), 4.18 (t, *J* = 7.0 Hz, 2H), 7.02 (d, *J =* 7.5 Hz, 2H), 7.23-7.29 (m, 8H), 7.43 (d, *J* = 2.0 Hz, 2H), 8.08-8.10 (m, 4H), 8.38 (d, *J* = 7.0 Hz, 2H), 9.90 (s, 2H), 10.36 (s, 2H); ¹³C NMR (100 MHz, MeOH-*d₄*): δ 17.6, 28.1, 36.7, 42.5, 48.9, 80.7, 106.2, 118.8, 118.9, 120.0, 121.4, 122.6, 123.4, 124.3, 128.6, 130.2, 130.6, 136.9, 139.7, 158.9, 160.2, 163.4, 170.2, 172.3.

**(2*S*,2'*S*)-2,2'-((2,2'-(((4,4'-((4,4'-Oxybis(benzoyl))bis(azanediyl))bis(1-methyl-1*H*-pyrrole-2,2'-carbonyl))bis(azanediyl))bis(3,1-phenylene))bis(acetyl))bis(azanediyl))dipropanoic acid SST0830AA1 (36d).** According to the above-described ester deprotection general procedure the title compound **36d** was obtained as a beige solid (nearly quantitative yield) starting from compound **33d.** MS (ESI): 911 [M+1]⁺; ¹H NMR (400 MHz, DMSO-*d₆*): δ 1.29 (d, *J* = 7.0 Hz, 6H), 3.46 (s, 4H), 3.88 (s, 6H), 4.20-4.25 (m, 2H), 6.96-6.99 (m, 2H), 7.19-7.25 (m, 8H), 7.39 (d, *J* = 2.0 Hz, 2H), 7.56-7.59 (m, 2H), 7.66-7.67 (m, 2H), 8.04 (d, *J* = 9.0 Hz, 4H), 8.34 (d, *J =* 7.5 Hz, 2H), 9.85 (s, 2H), 10.32 (s, 2H); ¹³C NMR (100 MHz, DMSO-*d₆*): δ 17.8, 36.7, 42.5, 48.0, 106.2, 118.8, 118.9, 120.0, 121.4, 122.6, 123.4, 124.3, 128.6, 130.2, 130.6, 136.9, 139.7, 158.9, 160.2, 163.4, 170.2, 174.5.

### Example 30

### Preparation of compound SST0831AA1 (36a).

**(2*S*,2'*S*)-Di-tert-butyl 2,2'-((2,2'-(((4,4'-((4,4'-oxybis(benzoyl))bis(azanediyl))bis(1-methyl-1*H*-pyrrole-2,2'-carbonyl))bis(azanediyl))bis(3,1-phenylene))bis(acetyl))bis(azanediyl))bis(4-methylpentanoate) (33a).** According to the above-described general procedure compound **28** was treated with L-leucine *tert*-butyl ester hydrochloride (**31a**). The title compound **33a** was obtained as off-white solid (52% yield) after purification by column flash chromatography (silica gel, cyclohexane/EtOAc 2:8 as eluent). MS (ESI): 1107 [M + 1]⁺; ¹H NMR (400 MHz, MeOH-*d₄*): δ 0.87 (d, *J =* 6.5 Hz, 6H), 0.92 (d, *J* = 6.5 Hz, 6H), 1.40 (s, 18H), 1.55-1.63 (m, 4H), 1.64-1.71 (m, 2H), 3.54 (s, 4H), 3.83 (s, 6H), 4.30-4.34 (m, 2H), 7.02-7.05 (m, 8H), 7.22 (dd, *J*₁ = *J*₂ = 8.0 Hz, 2H), 7.32 (d, *J* = 2.0 Hz, 2H), 7.47-7.49 (m, 2H), 7.59-7.60 (m, 2H), 7.89-7.91 (m, 4H); ¹³C NMR (100 MHz, MeOH-*d₄*): δ 20.7, 21.9, 24.7, 26.9, 35.7, 40.2, 42.2, 51.9, 81.3, 105.7, 118.4, 119.1, 120.3, 121.4, 122.0, 123.3, 124.3, 128.5, 129.3, 129.8, 136.0, 138.7, 159.1, 160.7, 165.0, 172.1, 172.4.

**(2*S*,2'*S*)-2,2'-((2,2'-(((4,4'-((4,4'-Oxybis(benzoyl))bis(azanediyl))bis(1-methyl-1*H*-pyrrole-2,2'-carbonyl))bis(azanediyl))bis(3,1-phenylene))bis(acetyl))bis(azanediyl))bis(4-methylpentanoic acid) SST0831AA1 (36a).** According to the above-described ester deprotection general procedure the title compound **36a** was obtained as a beige solid (nearly quantitative yield) starting from compound **33a.** MS (ESI): 995 [M+1]⁺; ¹H NMR (400 MHz, MeOH-*d₄*): δ 0.87 (d, *J* = 6.0 Hz, 6H), 0.92 (d, *J* = 6.0 Hz, 6H), 1.60-1.69 (m, 6H), 3.56 (s, 4H), 3.83 (s, 6H), 4.43-4.46 (m, 2H), 7.02-7.05 (m, 10H), 7.21 (dd, *J*₁ = *J*₂ = 8.0 Hz, 2H), 7.46-7.49 (m, 2H), 7.56-7.58 (m, 2H), 7.90 (d, *J* = 8.0 Hz, 4H); ¹³C NMR (100 MHz, MeOH-*d₄*): δ 20.5, 21.9, 24.6, 35.6, 39.9, 42.1, 51.0, 105.7, 118.3, 119.1, 121.4, 121.8, 123.2, 124.3, 128.5, 129.3, 129.7, 135.9, 136.0, 138.6, 159.1, 160.7, 165.0, 172.6, 174.6.

### Example 31

### Preparation of compound SST0832AA1 (36e).

**(2*S*,2'*S*)-Di-tert-butyl 2,2'-((2,2'-(((4,4'-((4,4'-oxybis(benzoyl))bis(azanediyl))bis(1-methyl-1*H*-pyrrole-2,2'-carbonyl))bis(azanediyl))bis(3,1-phenylene))bis(acetyl))bis(azanediyl))bis(3-phenylpropanoate) (33e).** According to the above-described general procedure compound **28** was treated with L-phenylalanine *tert*-butyl ester hydrochloride (**31e**). The title compound **33e** was obtained as off-white solid (46% yield) after purification by column flash chromatography (silica gel, cyclohexane/EtOAc 2:8 as eluent). MS (ESI): 1175 [M + 1]⁺; ¹H NMR (400 MHz, MeOH-*d₄*): δ 1.38 (s, 18H), 2.94-3.01 (m, 2H), 3.07-3.34 (m, 2H), 3.51 (s, 4H), 3.91 (s, 6H), 4.54-4.58 (m, 2H), 6.93-6.96 (m, 2H), 7.08 (d, *J* = 2.0 Hz, 2H), 7.11-7.20 (m, 10H), 7.22-7-26 (m, 6H), 7.36 (d, J=2.0 Hz, 2H), 7.50-7.54 (m, 4H), 7.95-7.98 (m, 4H); ¹³C NMR (100 MHz, MeOH-*d₄*): δ 26.8, 35.6, 37.0, 42.2, 54.5, 81.6, 105.7, 118.4, 119.2, 120.2, 121.5, 122.1, 123.4, 124.4, 126.4, 128.0, 128.5, 129.0, 129.3, 129.9, 135.8, 136.7, 138.7, 159.2, 160.8, 165.1, 170.7, 172.1.

**(2*S*,2'*S*)-2,2'-((2,2'-(((4,4'-((4,4'-Oxybis(benzoyl))bis(azanediyl))bis(1-methyl-1*H*-pyrrole-2,2'-carbonyl))bis(azanediyl))bis(3,1-phenylene))bis(acetyl))bis(azanediyl))bis(3-phenylpropanoic acid) SST0832AA1 (36e).** According to the above-described ester deprotection general procedure the title compound **36e** was obtained as a yellowish solid (nearly quantitative yield) starting from compound **33e.** MS (ESI): 1063 [M+1]⁺; ¹H NMR (400 MHz, DMSO-*d₆*): δ 2.87-2.93 (m, 2H), 3.04-3.09 (m, 2H), 3.43 (s, 4H), 3.89 (s, 6H), 4.44 (ddd, *J*₁ = *J*₂ = 9.0, *J*₃ = 5.0 Hz, 2H), 6.84 (d, *J* = 7.5 Hz, 2H), 7.16-7.22 (m, 14H), 7.23-7.25 (m, 4H), 7.39 (d, *J* = 2.0 Hz, 2H), 7.55 (dd, *J*₁ = 2.0, *J*₂ = 8.0 Hz, 2H), 7.62-7.63 (m, 2H), 9.84 (s, 2H), 10.33 (s, 2H); ¹³C NMR (100 MHz, DMSO-*d₆*): δ 36.7, 37.3, 42.6, 54.0, 106.2, 118.8, 118.9, 120.0, 121.6, 122.5, 122.6, 123.4, 124.2, 126.8, 128.6, 129.6, 130.2, 130.6, 136.8, 138.0, 139.6, 158.9, 160.2, 163.4, 170.4, 173.3.

### Example 32

### Preparation of compound SST0833AA1 (36f).

**(2*S*,2'*S*)-Tetra-tert-butyl 2,2'-((2,2'-(((4,4'-((4,4'-oxybis(benzoyl))bis(azanediyl))bis(1-methyl-1*H*-pyrrole-2,2'-carbonyl))bis(azanediyl))bis(3,1-phenylene))bis(acetyl))bis(azanediyl))dipentanedioate (33f).** According to the above-described general procedure compound **28** was treated with L-glutamic acid di-*tert*-butyl ester hydrochloride (**31f**). The title compound **33f** was obtained as off-white solid (44% yield) after purification by column flash chromatography (silica gel, cyclohexane/EtOAc 2:8 as eluent). MS (ESI): 1251 [M + 1]⁺; ¹H NMR (400 MHz, MeOH-*d₄*): δ 1.43 (s, 36H), 1.84-1.93 (m, 2H), 2.05-2.14 (m, 2H), 2.32 (t, *J* = 7.0 Hz, 4H), 3.57 (s, 4H), 3.90 (s, 6H), 4.31-4.34 (m, 2H), 7.06-7.12 (m, 8H), 7.27 (dd, *J*₁ = *J*₂ = 8.0 Hz, 2H), 7.35 (d, *J* = 2.0 Hz, 2H), 7.51-7.54 (m, 2H), 7.59-7.60 (m, 2H), 7.95 (d, *J* = 9.0 Hz, 4H); ¹³C NMR (100 MHz, MeOH-*d₄*): δ 26.5, 26.9, 27.0, 31.2, 42.2, 52.5, 80.4, 81.6, 105.7, 118.4, 119.1, 120.2, 121.4, 122.0, 123.3, 124.4, 128.5, 129.3, 129.8, 136.0, 138.7, 159.2, 160.8, 165.1, 171.0, 172.2, 172.4.

**(2*S*,2'*S*)-2,2'-((2,2'-(((4,4'-((4,4'-Oxybis(benzoyl))bis(azanediyl))bis(1-methyl-1*H*-pyrrole-2,2'-carbonyl))bis(azanediyl))bis(3,1-phenylene))bis(acetyl))bis(azanediyl))dipentanedioic acid SST0833AA1 (36f).** According to the above-described ester deprotection general procedure the title compound **36f** was obtained as a off-white solid (nearly quantitative yield) starting from compound **33f.** MS (ESI): 1027 [M+1]⁺; ¹H NMR (400 MHz, MeOH-*d₄*): δ 1.95-2.03 (m, 2H), 2.17-2.24 (m, 2H), 2.38-2.45 (m, 4H), 3.59 (s, 4H), 3.91 (s, 6H), 4.45-4.49 (m, 2H), 6.90-7.09 (m, 6H), 7.11-7.15 (m, 4H), 7.28 (dd, *J*₁ = *J*₂ = 7.5 Hz, 2H), 7.52-7.58 (m, 4H), 7.94-7.97 (m, 4H); ¹³C NMR (100 MHz, MeOH-*d₄*): δ 26.5, 29.8, 35.5, 42.1, 51.8, 105.7, 118.4, 119.1, 119.2, 121.4, 121.9, 123.3, 124.4, 128.5, 129.3, 129.8, 135.9, 138.6, 159.3, 160.9, 165.1, 172.6, 173.4, 174.9.

### Example 33

### Preparation of compound SST0834AA1 (36c).

**(2*S*,2'*S*)-Di-tert-butyl 2,2'-((2,2'-(((4,4'-((4,4'-oxybis(benzoyl))bis(azanediyl))bis(1-methyl-1*H*-pyrrole-2,2'-carbonyl))bis(azanediyl))bis(3,1-phenylene))bis(acetyl))bis(azanediyl))bis(6-((tert-butoxycarbonyl)amino)hexanoate) (33c).** According to the above-described general procedure compound **28** was treated with Nepsilon-Boc-L-lysine *tert*-butyl ester hydrochloride (**31c**). The title compound **33c** was obtained as off-white solid (52% yield) after purification by column flash chromatography (silica gel, cyclohexane/EtOAc 2:8 as eluent). MS (ESI): 1337 [M + 1]⁺; ¹H NMR (400 MHz, MeOH-*d₄*): δ 1.29-1.31 (m, 8H), 1.43 (s, 36H), 1.64-1.73 (m, 2H), 1.76-1.85 (m, 2H), 2.98-3.03 (m, 4H), 3.57 (s, 4H), 3.89 (s, 6H), 4.24-4.27 (m, 2H), 7.06-7.12 (m, 8H), 7.27 (dd, *J*₁ = *J*₂ = 8.0 Hz, 2H), 7.35 (d, *J* = 2.0 Hz, 2H), 7.50-7.52 (m, 2H), 7.60-7.61 (m, 2H), 7.94 (d, *J =* 9.0 Hz, 4H); ¹³C NMR (100 MHz, MeOH-*d₄*): δ 22.7, 26.9, 27.5, 29.4, 30.9, 35.6, 39.8, 42.2, 53.3, 78.4, 81.4, 105.7, 118.4, 119.1, 120.2, 121.4, 122.1, 123.3, 124.4, 128.5, 129.3, 129.8, 136.0, 138.7, 157.1, 159.2, 160.8, 165.0, 171.5, 172.4.

**(2*S*,2'*S*)-2,2'-((2,2'-(((4,4'-((4,4'-oxybis(benzoyl))bis(azanediyl))bis(1-methyl-1*H*-pyrrole-4,2-diyl-2-carbonyl))bis(azanediyl))bis(3,1-phenylene))bis(acetyl))bis(azanediyl))bis(6-aminohexanoic acid) dihydrochloride SST0834AA1 (36c).** According to the above-described ester deprotection general procedure the title compound **36c** was obtained as a off-white solid (nearly quantitative yield) starting from compound **33c.** MS (ESI): 1025 [M+1]⁺; ¹H NMR (400 MHz, MeOH-*d₄*): δ 1.40-1.49 (m, 4H), 1.62-1.67 (m, 4H), 1.75-1.79 (m, 2H), 1.87-1.93 (m, 2H), 2.83-2.86 (m, 4H), 3.59 (s, 4H), 3.86 (s, 6H), 4.40-4.43 (m, 2H), 7.06-7.14 (m, 8H), 7.26 (dd, *J*₁ = *J*₂ = 7.5 Hz, 2H), 7.31-7.40 (m, 2H), 7.49-7.51 (d, *J* = 8.0 Hz, 2H), 7.62 (s, 2H), 7.95 (d, *J* = 8.0 Hz, 4H); ¹³C NMR (100 MHz, MeOH-*d₄*): δ 22.4, 26.6, 30.7, 35.7, 39.1, 42.2, 52.0, 106.0, 118.4, 119.4, 120.4, 121.6, 121.9, 123.1, 124.6, 128.6, 129.4, 129.7, 136.1, 138.6, 159.2, 160.9, 165.0, 172.6, 173.7.

### Example 34

### Preparation of compound SST0755AA1 (12b).

**Di-*tert*-butyl 2,2'-((2,2'-(((4,4'-((3,3'-(carbonylbis(azanediyl))bis(benzoyl))bis(azanediyl))bis(1-methyl-1*H*-pyrrole-4,2-diyl-2-carbonyl))bis(azanediyl))bis(3,1-phenylene))bis(acetyl))bis(azanediyl))diacetate (34b).** According to the above-described general procedure compound **12h** was treated with glycine *tert*-butyl ester hydrochloride (**31b**). The title compound **34b** was obtained as white solid (60% yield) after precipitation from DMF/H₂O. MS (ESI): 1037 [M+1]⁺. ¹H NMR (400 MHz, DMSO-*d₆*): δ 1.42 (s, 18H), 3.46 (s, 2H), 3.73 (d, *J* = 5.5 Hz, 4H), 3.88 (s, 6H), 7.19 - 6.98 (d, *J* = 7.5 Hz, 2H), 7.23 (t, *J* = 7.5 Hz, 2H), 7.39 (s, 2H), 7.44 (t, *J* = 7.5 Hz, 2H), 7.69 - 7.56 (m, 8H), 8.01- 7.96 (m, 2H), 8.32 - 8.30 (m, 2H), 8.93 (s, 2H), 9.86 (s, 2H), 10.32 (s, 2H). ¹³C NMR (100 MHz, DMSO-*d₆*): δ 28.2, 36.6, 42.0, 42.6, 81.0, 106.3, 118.2, 118.9, 120.0, 121.2, 121.5, 122.6, 123.5, 124.3, 128.7, 129.2, 136.1, 136.8, 139.7, 140.3, 153.1, 160.3, 164.3, 169.4, 170.8.

**2,2'-((2,2'-(((4,4'-((3,3'-(Carbonylbis(azanediyl))bis(benzoyl))bis(azanediyl))bis(1-methyl-1*H*-pyrrole-4,2-diyl-2-carbonyl))bis(azanediyl))bis(3,1-phenylene))bis(acetyl))bis(azanediyl))diacetic acid SST0755AA1 (12b).** According to the above-described ester deprotection general procedure the title compound **12b** was obtained as a off-white solid (nearly quantitative yield) starting from compound **34b.**

### Examples 35-39

### Synthesis of boronic compounds

**General procedure for the synthesis of boronic acid pinanediol ester derivatives (38-40, 44-45).** HOBt (324 mg, 2.4 mmol) was added to an ice-cold solution of the appropriate carboxylic acid **17h, 28, 12h, 17b** or **12b** (0.5 mmol) in dry DMF (15 mL). After 20 min, the reaction mixture was treated with EDC·HCl (460 mg, 2.4 mmol). Finally, a solution of commercially available (*R*)-boroleucine-(+)-pinanediol trifluoroacetate (**37**) (379 mg, 1 mmol) and DIPEA (208 µL, 1.2 mmol) in dry DMF (4 mL) were added and the reaction mixture was stirred at room temperature for 48 h. EtOAc (10 mL) was added to the reaction mixture, the solution was washed in sequence with 0.1 M KHSO₄ (1 x 10 mL), 5% NaHCO₃ (1 x 10 mL) and brine (1 x 10 mL), and then dried over Na₂SO₄ and filtered. After removing the solvent by distillation under reduced pressure, the crude residue was purified by column flash chromatography or crystallization.

### General Procedure for Pinanediol Removal.

Phenylboronic acid (110 mg, 0.9 mmol), and *n*-hexane (16 mL) were sequentially added to a solution of the pinanediol esters **38-40, 44** or **45** (0.5 mmol) in MeOH (16 mL), and HCl (1 mmol of a 1 M solution in degassed H₂O). The resulting biphasic solution was vigorously stirred. After 30 min, the n-hexane solution (containing the pinanediol phenylboronate) was removed, and fresh n-hexane (16 mL) was added. This last procedure was repeated six times until a TLC analysis of the *n*-hexane layer did not reveal further phenylboronate production (total reaction time 3 h). The methanol phase was concentrated under reduced pressure and the crude residue was purified by crystallization.

### Example 35

### Preparation of compound SST0905AA1 (41)

**4,4'-(Carbonylbis(azanediyl))bis(1-methyl-*N*-(1-methyl-5-((3-(2-(((*R*)-3-methyl-1-((3a*S*,4*S*,6*S*,7a*R*)-3a,5,5-trimethylhexahydro-4,6-methanobenzo[*d*][1,3,2]dioxaborol-2-yl)butyl)amino)-2-oxoethyl)phenyl)carbamoyl)-1*H*-pyrrol-3-yl)-1*H*-pyrrole-2-carboxamide) (38).** The pinanediol ester derivative **38** was prepared according to the above general procedure starting from **17h** and **37.** Pink solid (32% yield); purification by column flash chromatography (silica gel, CH₂Cl₂/MeOH 95:5 as eluent). MS (ESI): 1311 [M + 1]⁺; ¹H NMR (400 MHz, acetone-*d*₆): δ 0.73-0.84 (m, 18H), 1.11-1.31 (m, 20H), 1.63-170 (m, 6H), 1.19-1.82 (m, 2H), 2.15 (dd, *J*₁ = *J*₂ = 11.0 Hz, 2H), 2.62 (dd, *J*₁ = *J*₂ = 7.0 Hz, 2H), 3.48 (br s, 4H), 3.75 (br s, 12H), 4.04 (d, *J* = 8.0 Hz, 2H), 6.69 (br s, 2H), 6.79 (br s, 2H), 6.89-6.95 (m, 4H), 7.10-7.15 (m, 4H), 7.50 (d, *J* = 7.0 Hz, 2H), 7.59 (br s, 2H), 7.59 (br s, 2H), 7.70 (br s, 2H), 8.37 (br s, 2H), 8.99 (br s, 2H), 9.08 (s, 2H).

**((1*R*,1'*R*)-((2,2'-(((4,4'-((4,4'-(carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))bis(3-methylbutane-1,1-diyl))diboronic acid SST0905AA1 (41).**
According to the above-described general procedure for pinanediol removal, the title compound **41** was obtained as a red solid (83% yield) starting from compound **38.** Purification by crystallization from MeOH/diethyl ether. ¹H NMR (400 MHz, MeOH-*d₄*): δ 0.80-0.82 (m, 12H), 1.18-1.30 (m, 4H), 1.51-1.63 (m, 2H), 1.54-1.62 (m, 2H), 3.66 (s, 4H), 3.78 (s, 6H), 3.79 (s, 6H), 6.67 (s, 2H), 6.88 (br s, 2H), 6.91 (s, 2H), 6.97 (d, *J* = 8.0 Hz, 2H), 7.17 (br s, 2H), 7.21 (dd, *J*₁ = *J*₂ = 8.0 Hz, 2H), 7.42 (d, *J* = 8.0 Hz, 2H), 7.58 (br s, 2H); ¹³C NMR (100 MHz, MeOH-*d₄*): δ 20.9, 22.4, 25.7, 35.4, 35.45, 36.3, 39.5, 119.7, 121.2, 121.7, 123.0, 123.1, 124.2, 128.9, 133.4, 139.2, 160.0, 160.9, 177.2.

### Example 36

### Preparation of compound SST0838AA1 (42)

**4,4'-((4,4'-oxybis(benzoyl))bis(azanediyl))bis(1-methyl-*N*-(3-(2-(((*R*)-3-methyl-1-((3a*S*,4*S*,6*S*,7a*R*)-3a,5,5-trimethylhexahydro-4,6-methanobenzo[*d*][1,3,2]dioxaborol-2-yl)butyl)amino)-2-oxoethyl)phenyl)-1*H*-pyrrole-2-carboxamide) (39).** The pinanediol ester derivative **39** was prepared according to the above general procedure starting from **28** and **37.** Orange solid (23% yield); purification by column flash chromatography (silica gel, cyclohexane/EtOAc 1:9 as eluent). MS (ESI): 1263 [M + 1]⁺; ¹H NMR (400 MHz, MeOH-*d₄*): δ 0.84 (s, 6H), 0.92 (d, *J* = 2.0 Hz, 6H), 0.94 (d, *J* = 2.0 Hz, 6H), 1.25 (s, 6H), 1.34 (s, 6H), 1.35-1.39 (m, 4H), *1.47 (d, J =* 10.0 Hz, 2H), 1.73-1.85 (m, 6H), 1.93 (t, *J* = 5.5 Hz, 2H), 2.06-2.12 (m, 2H), 2.27-2.33 (m, 2H), 2.65-2.69 (m, 2H), 3.67 (s, 4H), 3.88 (s, 6H), 4.12-4.15 (m, 2H), 7.02-7.09 (m, 8H), 7.28 (dd, *J*₁ = *J*₂ = 8.0 Hz, 2H), 7.34 (d, *J* = 2.0 Hz, 2H), 7.54-7.60 (m, 4H), 7.92-7.94 (m, 4H); ¹³C NMR (100 MHz MeOH-*d₄*): δ 21.1, 22.3, 23.3, 25.5, 26.1, 26.5, 28.5, 35.7, 36.4, 36.8, 37.9, 40.0, 40.5, 52.3, 75.6, 83.4, 105.8, 118.4, 119.6, 120.3, 121.1, 122.1, 123.3, 124.2, 128.8, 129.3, 129.8, 133.8, 139.0, 159.2, 160.7, 165.0, 176.3.

**((1*R*,1'*R*)-((2,2'-(((4,4'-((4,4'-Oxybis(benzoyl))bis(azanediyl))bis(1-methyl-1*H*-pyrrole-2,2'-carbonyl))bis(azanediyl))bis(3,1-phenylene))bis(acetyl))bis(azanediyl))bis(3-methylbutane-1,1-diyl))diboronic acid SST0838AA1 (42).** According to the above-described general procedure for pinanediol removal, the title compound **42** was obtained as off-white solid (71% yield) starting from compound **39**. ¹H NMR (400 MHz, MeOH-*d₄*): δ 0.88 (s, 6H), 0.89(s, 6H), 1.27-1.32 (m, 4H), 1.62-1.69 (m, 2H), 2.73 (t, *J* = 7.5 Hz, 2H), 3.75 (s, 4H), 3.82 (s, 6H), 7.0-7.03 (m, 8H), 7.10 (s, 2H), 7.25 (dd, *J*₁ = *J*₂ = 8.0 Hz, 2H), 7.50 (dd, *J*₁ = 2.0, *J*₂ = 8.0 Hz, 2H), 7.68 (s, 2H), 7.89-7.91 (m, 4H); ¹³C NMR (100 MHz, MeOH-*d₄*): δ 20.9, 22.4, 25.6, 35.6, 36.1, 39.3, 106.0, 118.4, 119.9, 121.3, 121.8, 121.9, 123.0, 124.2, 128.9, 129.4, 129.7, 133.1, 139.2, 159.2, 160.8, 165.0, 177.6.

### Example 37

### Preparation of compound SST0828AA1 (43)

**4,4'-((3,3'-(Carbonylbis(azanediyl))bis(benzoyl))bis(azanediyl))bis(1-methyl-*N*-(3-(2-(((R)-3-methyl-1-((3a*S*,4*S*,6*S*,7a*R*)-3a,5,5-trimethylhexahydro-4,6-methanobenzo[*d*][1,3,2]dioxaborol-2-yl)butyl)amino)-2-oxoethyl)phenyl)-1*H*-pyrrole-2-carboxamide) (40).** The pinanediol ester derivative **40** was prepared according to the above general procedure starting from **12h** and **37.** Orange solid (84% yield); purification by column flash chromatography (silica gel, gradient from EtOAc to EtOAc/MeOH 9:1 as eluent). MS (ESI): 1305 [M + 1]⁺; ¹H NMR (400 MHz, MeOH-*d₄*): δ 0.27 - 0. 81 (m, 22H), 1.17 - 1.26 (m, 10H), 1.33 - 1.35 (m, 2H), 1.60 - 1.81 (m, 8H), 1.95 - 2.00 (m, 2H), 2.15 - 2.21 (m, 2H), 2.54 (t, *J* = 7.5 Hz, 2H), 3.54 (s, 4H), 3.74 (s, 6H), 3.96 - 4.02 (m, 4H), 6.90 (d, *J* = 7.5 Hz, 2H), 6.95 (d, *J* = 1.5 Hz, 2H), 7.14 (t, *J* = 7.5 Hz, 2H), 7.20 - 7.21 (m, 2H), 7.24 (t, *J* = 7.5 Hz, 2H), 7.38 - 7.46 (m, 8H), 7.88 (s, 2H); ¹³C NMR (100 MHz, MeOH-*d₄*): δ 21.0, 22.3, 23.3, 25.5, 26.1, 26.4, 28.4, 35.6, 36.4, 36.8, 37.8, 40.0, 40.5, 42.2, 75.6, 82.4, 105.8, 118.0, 119.6, 120.4, 121.2, 121.9, 122.0, 123.2, 124.2, 128.7, 133.7, 135.2, 139.0, 139.4, 152.3, 153.6, 160.8, 165.9, 176.4,

**((1*R*,1'*R*)-((2,2'-(((4,4'-((3,3'-(Carbonylbis(azanediyl))bis(benzoyl))bis(azanediyl))bis(1-methyl-1H-pyrrole-4,2-diyl-2-carbonyl))bis(azanediyl))bis(3,1-phenylene))bis(acetyl))bis(azanediyl))bis(3-methylbutane-1,1-diyl))diboronic acid SST0828AA1 (43).** According to the above-described general procedure for pinanediol removal, the title compound **43** was obtained as off-white solid (46% yield) starting from compound **40.** Purification by crystallization from MeOH/H₂O. ¹H NMR (400 MHz, MeOH-*d₄*): δ 0.87 - 0.96 (m, 12H), 1.26 - 1.38 (m, 4H), 1.66 - 1.71 (m, 2H), 2.68 - 2.72 (m, 2H), 3.79 (s, 4H), 3.93 (s, 6H), 7.08 - 7.11 (m, 4H), 7.34 (t, *J* = 7.5 Hz, 2H), 7.39 (d, *J* = 1.5 Hz, 2H), 7.46 (t, *J* = 7.5 Hz, 2H), 7.55 - 7.68 (m, 10H), 8.05 (t, *J* = 1.5 Hz, 2H); ¹³C NMR (100 MHz, MeOH-*d₄*): δ 19.4, 20.9, 24.1, 34.0, 34.8, 38.0, 104.2, 116.5, 118.2, 119.7, 120.3, 120.4, 121.6, 121.7, 122.7, 127.3, 132.1, 133.8, 137.6, 137.9, 155.3, 159.5, 164.5, 175.4.

### Example 38

### Preparation of compound SST0906AA1 (46)

**4,4'-(Carbonylbis(azanediyl))bis(1-methyl-*N*-(1-methyl-5-((3-(2-((2-(((*R*)-3-methyl-1-((3a*S*,4*S*,6*S*,7a*R*)-3a,5,5-trimethylhexahydro-4,6-methanobenzo[*d*][1,3,2]dioxaborol-2-yl)butyl)amino)-2-oxoethyl)amino)-2-oxoethyl)phenyl)carbamoyl)-1*H*-pyrrol-3-yl)-1*H-*pyrrole-2-carboxamide) (44).** The pinanediol ester derivative **44** was prepared according to the above general procedure starting from **17b** and **37.** Brown solid (82% yield) obtained by filtration of the solid formed after adding H₂O to the reaction mixture and subsequent purification by crystallization from MeOH/diethyl ether. MS (ESI): 1425 [M + 1]⁺; ¹H NMR (400 MHz, MeOH-*d₄*): δ 0.85-0.92 (m, 18H), 1.25-1.47 (m, 18H), 1.65-1.80 (m, 6H), 1.81-1.88 (m, 2H), 1.92-1.93 (m, 2H), 2.08-2.11 (m, 2H), 2.27-2.35 (m, 2H), 2.71 (dd, *J* = 7.5 Hz, 2H), 3.60 (s, 4H), 3.92 (s, 6H), 3.93 (s, 6H), 4.07 (s, 2H), 4.15 (dd, *J*₁ = 2.0, *J*₂ = 8.5 Hz, 2H), 6.78 (br s, 2H), 6.98 (br s, 2H), 7.07 (br s, 2H), 7.08 (d, *J* = 7.5 Hz, 2H), 7.27 (br s, 2H), 7.30 (dd, *J*₁ = *J*₂ = 7.5 Hz, 2H), 7.53 (d, *J =* 7.5 Hz, 2H), 7.60 (s, 2H).

**((1*R*,1*'R*)-((2,2'-((2,2'-(((4,4'-((4,4'-(carbonylbis(azanediyl))bis(1-methyl-1*H*-pyrrole-4,2-diyl-2-carbonyl))bis(azanediyl))bis(1-methyl-1*H*-pyrrole-4,2-diyl-2-carbonyl))bis(azanediyl))bis(3,1-phenylene))bis(acetyl))bis(azanediyl))bis(acetyl))bis(azanediyl))bis(3-methylbutane-1,1-diyl))diboronic acid SST0906AA1 (46).** According to the above-described general procedure for pinanediol removal, the title compound **46** was obtained as a red solid (95% yield) starting from compound **44**. Purification by crystallization from MeOH/diethyl ether. ¹H NMR (400 MHz, MeOH-*d₄*): δ 0.90-0.92 (m, 12H), 1.27-1.34 (m, 4H), 1.57-1.66 (m, 2H), 2.66-2.72 (m, 2H), 3.61 (s, 4H), 3.92 (s, 6H), 3.93 (s, 6H), 4.10 (s, 4H), 6.78 (br s, 2H), 7.01 (br s, 2H), 7.09 (d, *J* = 8.0 Hz, 2H), 7.31 (dd, *J*₁ = *J*₂ = 8.0 Hz, 2H), 7.51 (d, *J* = 8.0 Hz, 2H), 7.62 (br s, 2H); ¹³C NMR (100 MHz, MeOH-*d₄*): δ 21.0, 22.3, 35.4, 35.5, 38.5, 39.4, 39.4, 42.1, 100.4, 119.3, 121.4, 121.4, 124.5, 127.3, 128.6, 134.3, 135.6, 138.8, 160.9, 173.2, 175.2.

### Example 39

### Preparation of compound SST0829AA1 (47)

**4,4'-((3,3'-(Carbonylbis(azanediyl))bis(benzoyl))bis(azanediyl))bis(1-methyl-*N*-(3-(2-((2-(((*R*)-3-methyl-1-((3a*S*,4*S*,6*S*,7a*R*)-3a,5,5-trimethylhexahydro-4,6-methanobenzo[*d*][1,3,2]dioxaborol-2-yl)butyl)amino)-2-oxoethyl)amino)-2-oxoethyl)phenyl)-1*H*-pyrrole-2-carboxamide) (45).** The pinanediol ester derivative **45** was prepared according to the above general procedure starting from **12b** and **37**. White solid (85% yield) obtained by filtration of the solid formed after adding H₂O to the reaction mixture. MS (ESI) 1419 [M + 1]⁺; ¹H NMR (400 MHz, DMSO-*d₆*): δ 0.81 - 0.86 (m, 22H), 1.22 - 1.33 (m, 14H), 1.63 - 2.21 (m, 14H), 2.59 (br s, 2H), 3.49 (s, 4H), 3.76 - 3.85 (m, 2H), 3.89 (s, 6H), 4.09 (d, *J* = 7.5 Hz, 2H), 6.98 (d, *J* = 7.5 Hz, 2H), 7.19 - 7.70 (m, 16H), 7.96 - 8.01 (m, 2H), 8.33 (br s, 2H), 8.68 (s, 2H), 8.93 (s, 2H), 9.86 (s, 2H), 10.32 (s, 2H); ¹³C NMR (100 MHz, DMSO-*d₆*): δ 22.7, 23.4, 24.3, 25.3, 26.4, 27.6, 29.4, 36.5, 36.7, 39.8, 40.3, 40.9, 42.7, 52.2, 76.2, 83.5, 106.3, 118.2, 118.8, 120.0, 121.2, 121.4, 122.7, 123.4, 124.3, 128.7, 129.2, 136.0, 136.8, 139.8, 140.3, 153.1, 160.3, 162.8, 164.3, 171.0, 172.2

**((1*R,*1'*R*)-((2,2'-((2,2'-(((4,4'-((3,3'-(Carbonylbis(azanediyl))bis(benzoyl))bis(azanediyl))bis(1-methyl-1*H*-pyrrole-4,2-diyl-2-carbonyl))bis(azanediyl))bis(3,1-phenylene))bis(acetyl))bis(azanediyl))bis(acetyl))bis(azanediyl))bis(3-methylbutane-1,1-diyl))diboronic acid SST0829AA1 (47).** According to the above-described general procedure for pinanediol removal the title compound **47** was obtained as an orange solid (42% yield) starting from compound **45**. Purification by crystallization from MeOH/H₂O. ¹H NMR (400 MHz, DMSO-*d₆*): δ 0.80 (s, 12H), 1.25 - 1.33 (m, 2H), 1.60 - 1.64 (m, 2H), 2.49 - 2.51 (m, 2H), 3.48 (s, 4H), 3.63 (s, 4H), 3.87 (s, 6H), 6.96 - 6.99 (m, 2H), 7.18 -7.22 (m, 4H), 7.35 - 7.45 (m, 4H), 7.50 - 7.54 (m, 8H), 7.98 - 8.01 (m, 2H), 8.44 (br s, 1 H), 8.80 (br s, 1H), 9.18 (br s, 1H), 9.89 (br s, 1H), 10.4 (br s, 1H); ¹³C NMR (100 MHz, DMSO-*d₆*): δ 22.5, 22.7, 23.3, 24.3, 25.3, 25.6, 29.4, 36.7, 106.3, 118.1, 118.8, 120.0, 121.1, 121.4, 122.6, 123.4, 124.4, 128.7, 129.2, 136.0, 136.6, 139.8, 140.3, 153.1, 160.3, 164.4, 171.2, 172.2.

### Example 40

### Preparation of compound 49b

Compound 49b has been prepared as depicted in scheme 21 below.

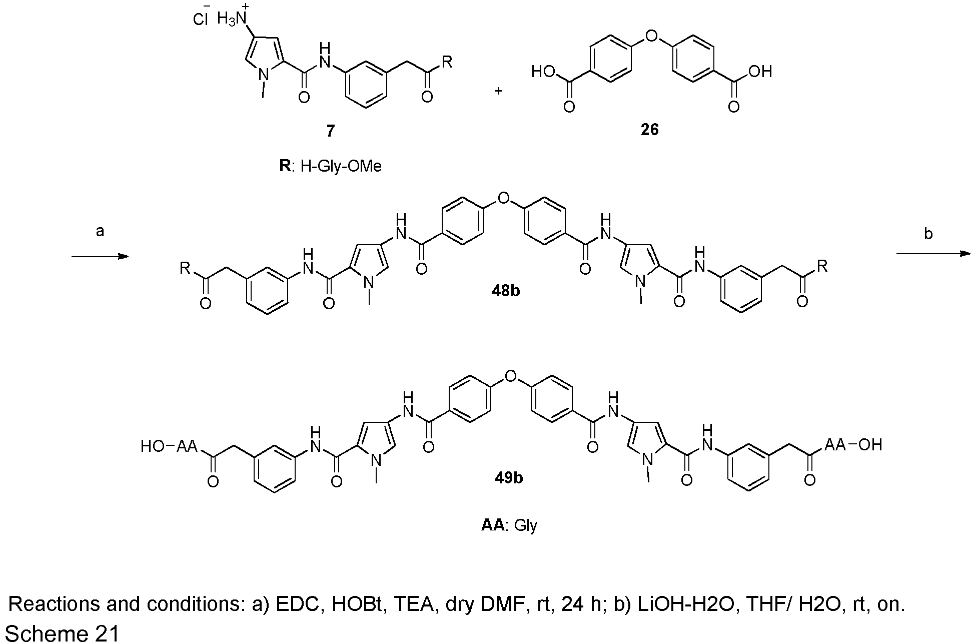

### Dimethyl 2,2'-((2,2'-(((4,4'-((4,4'-oxybis(benzoyl))bis(azanediyl))bis(1-methyl-1H-pyrrole-2,2'-carbonyl))bis(azanediyl))bis(3,1-phenylene))bis(acetyl))bis(azanediyl))diacetate

**(48b).** 1-Hydroxybenzotriazole hydrate (HOBt) (149 mg, 1.1 mmol), *N*-(3-Dimethylaminopropyl)-*N'*-ethylcarbodiimide hydrochloride (EDC) (230 mg, 1.2 mmol) and TEA (0.28 mL, 2 mmol) were added to a solution of 4,4'-oxydibenzoic acid (**26**) (0.5 mmol) in dry DMF (3 mL), and the resulting mixture was stirred at room temperature for 30 minutes. EtOAc (15 mL) was added to the reaction mixture, the solution was washed in sequence with H₂O (1 x 15 mL), 5% citric acid (1 x 15 mL), brine (1 x 15 mL), 5% NaHCO₃ (1 x 15 mL), and brine (1 x 15 mL). The organic layer was dried over Na₂SO₄ and concentrated under reduced pressure to afford dimethyl 2,2'-((2,2'-(((4,4'-((4,4'-oxybis(benzoyl))bis(azanediyl))bis(1-methyl-1 H-pyrrole-2,2'-carbonyl))bis(azanediyl))bis(3,1-phenylene))bis(acetyl))bis(azanediyl))diacetate (**48b**) (75% Yield). ¹H-NMR 200 MHz (DMSO-*d*₆) δ: 3.69 (s, 6H), 3.85 (s, 4H), 3.91 (s, 6H), 4.17 (m, 4H), 6.97 (d, 1H, *J*=7.6 Hz), 7.17-7.27 (m, 2H), 7.39-7.47 (m, 2H), 7.54-7.58 (m, 2H), 7.67 (s, 2H), 8.00 (s, 1 H), 8.07 (s, 2H), 9.15 (b, 4H). MS (ESI): [M+1]⁺ = 910.33.

**2,2'-((2,2'-(((4,4'-((4,4'-oxybis(benzoyl))bis(azanediyl))bis(1-methyl-1H-pyrrole-2,2'-carbonyl))bis(azanediyl))bis(3,1-phenylene))bis(acetyl))bis(azanediyl))diacetic acid (49b).** To a suspension of dimethyl 2,2'-((2,2'-(((4,4'-((4,4'-oxybis(benzoyl))bis(azanediyl))bis(1-methyl-1 H-pyrrole-2,2'-carbonyl))bis(azanediyl))bis(3,1-phenylene))bis(acetyl))bis(azanediyl))diacetate (**48b**) (17.10 mg, 0.02 mmol) in THF (5 mL), was added LiOH·H₂O (2.7 mg, 0.06 mmol) and water (1.6 mL). After stirring over night at 25 °C, the solvent was concentrated under reduced pressure, the reaction mixture was diluted with water and then acidified with 1 N HCl. The precipitate was filtered and wash with water to give 2,2'-((2,2'-(((4,4'-((4,4'-oxybis(benzoyl))bis(azanediyl))bis(1-methyl-1H-pyrrole-2,2'-carbonyl))bis(azanediyl))bis(3,1-phenylene))bis(acetyl))bis(azanediyl))diacetic acid (**49b**) (6.97 mg, 42%). NMR 200 MHz (DMSO-*d*₆) δ: 3.85 (s, 4H), 3.91 (s, 6H), 4.17 (m, 4H), 6.97 (d, 1 H, *J*=7.6 Hz), 7.17-7.27 (m, 2H), 7.39-7.47 (m, 2H), 7.54-7.58 (m, 2H), 7.67 (s, 2H), 8.00 (s, 1H), 8.07 (s, 2H), 9.15 (b, 4H), 12.09 (b, 2H). MS (ESI): [M+1]⁺ = 882.30.

### Example 41

### Preparation of compound 52b

Compound 52b has been prepared as depicted in scheme 22 below.

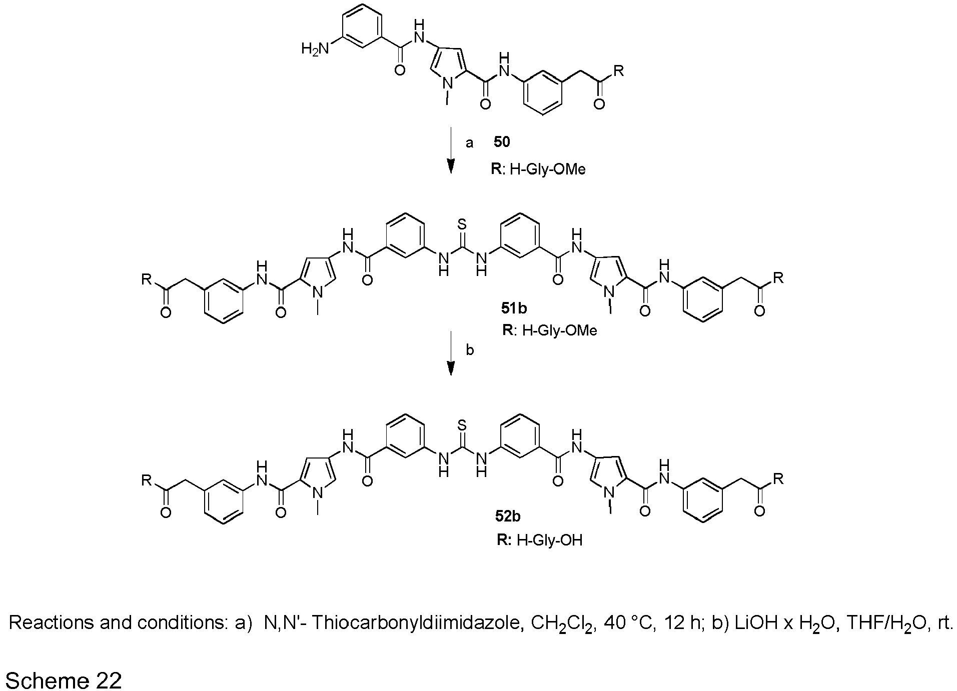

**Dimethyl 2,2'-((2,2'-(((4,4'-((3,3'-(thiocarbonylbis(azanediyl))bis(benzoyl))bis(azanediyl))bis(1-methyl-1H-pyrrole-2,2'-carbonyl))bis(azanediyl))bis(3,1-phenylene))bis(acetyl))bis(azanediyl))diacetate (51b).** To a stirred solution of methyl 2-(2-(3-(4-(3-aminobenzamido)-1-methyl-1H-pyrrole-2-carboxamido)phenyl)acetamido)acetate (**50**) (0.61 g, 2.2mmol) in CH₂Cl₂ (100mL) was added N,N'-Thiocarbonyldiimidazole (0.18g, 1.03mmol), the mixture was stirred at 40°C for 12h. The solvent was removed under vacuum and the crude was treated with distilled water, the solid formed was filtered, washed with water and dried under IR lamp. Purification of crude product was performed by chromatography (chloroform/methanol 30:1 as eluent) to give 0.385 g as pure product. R_{f} (chloroform/methanol 10:1): 0.55; 60% as a yellow solid; 195-196 °C; washed with diethyl ether. ¹H NMR (DMF *d₇*) δ 1.37 (s, 6H), 3.85 (s, 4H), 3.91 (s, 6H), 4.01 (b, 2H), 4.17 (m, 4H), 6.96 (d, 1H, *J*=7.6 Hz), 7.17-7.21 (m, 2H), 7.40-7.43 (m, 2H), 7.53 (m, 2H), 7.68 (s, 2H), 8.01 (s, 1H), 8.03 (s, 2H), 9.15 (b, 4H). MS (ESI): [M+1]⁺ = 463.19.

**2,2'-((2,2'-(((4,4'-((3,3'-(thiocarbonylbis(azanediyl))bis(benzoyl))bis(azanediyl))bis(1-methyl-1H-pyrrole-2,2'-carbonyl))bis(azanediyl))bis(3,1-phenylene))bis(acetyl))bis(azanediyl))diacetic acid.** (**52b**) A mixture of NaOH (5 mmol) and dimethyl 2,2'-((2,2'-(((4,4'-((3,3'-(thiocarbonylbis(azanediyl))bis(benzoyl))bis(azanediyl))bis(1-methyl-1H-pyrrole-2,2'-carbonyl))bis(azanediyl))bis(3,1-phenylene))bis(acetyl))bis(azanediyl))diacetate (**51b**) (0.84 g, 1.38 mmol) in THF (50 mL) and H₂O (20 mL) was stirred at room temperature overnight. The solvent was removed under vacuum and the crude was treated with 1 N HCl until pH 3-4, the solid formed was filtered, washed with water and dried under IR lamp. to give pure product (**52b**) (0.43g).; R_{f} (chloroform/methanol 10:1): 0.10; 44% as a brow solid; Carbonizes 300°C; washed with diethyl ether. ¹H NMR (DMF *d₇*) δ: 3.85 (s, 4H), 3.91 (s, 6H), 4.01 (b, 2H), 4.17 (m, 4H), 6.96 (d, 1H, *J*=7.6 Hz), 7.17-7.21 (m, 2H), 7.40-7.43 (m, 2H), 7.53 (m, 2H), 7.68 (s, 2H), 8.01 (s, 1 H), 8.03 (s, 2H), 9.15 (b, 4H), 12.55 (br s, 2H). MS (ESI): [M+1]⁺ = 940.30.

### Biological assays

### EXAMPLE 42

### In vitro screening for heparanase activity

All compounds were tested in a simple, accurate and robust microplate assay based on the cleavage of the synthetic heparin fragment, the pentasaccharide Fondaparinux (AGA*IA). This assay was originally developed by Hammond and coworkers (Hammond E, Li CP, Ferro V. Development of a colorimetric assay for heparanase activity suitable for kinetic analysis and inhibitor screening. Anal. Biochem. 396(1), 112-116 (2010)), where Fondaparinux corresponds to the methyl glycoside of the antithrombin III (ATIII)-activating pentasaccharide sequence of heparin. Fondaparinux is cleaved by heparanase to generate a reducing disaccharide, which can be quantitatively measured via a colorimetric or a fluorescence assay, the last by the fluorescent sensor polymer-H [Schiemann S, Lühn S, Alban S. Development of both colorimetric and fluorescence heparinase activity assays using fondaparinux as substrate. Anal. Biochem. 427(1), 82-89 (2012)].

### Materials and methods

To determine the activity of the newly designed heparanase inhibitors an homogenous assay based on the cleavage of the synthetic heparin oligosaccharide fondaparinux has been employed (Hammond E. et al. Anal. Biochem. 2010, 396, 112). The assay measures the appearance of the disaccharide product of heparanase-catalyzed fondaparinux cleavage colorimetrically using the tetrazolium salt WST-1. Assay was essentially performed as described with minor modifications. Briefly, NUNC ELISA 96-well plates were pre-treated with a solution of 4% BSA (bovin serum albumin) in phosphate-buffered saline containing 0.05% Tween 20 (PBST), for 2 h at 37°C, then washed three times with PBST, dried by tapping on paper towel and stored at -20°C. Before use, once again, the plates were washed with PBST.

Assay was carried out with 100 µl/well of assay solution containing 40 mM sodium acetate buffer (pH 5.0), 100 µM fondaparinux, 2.5 nM heparanase and serial dilutions of test compounds (tested in triplicate). Plates were sealed with adhesive tape and incubated, in the dark, for 3 h at 37°C, followed by development with 1.69 mM of the tetrazolium salt WST-1 solution in 0.1 M NaOH, for 1 h at 60°C. Then, the absorbance at 560 nm was measured through a microplate reader (Victor 3, Perkin Elmer).

### Results

IC₅₀ value for each compound, versus heparanase, was calculated by GraphPad software and results are summarized in the Table 1 below.

Finally, the measurements were corrected by subtracting both the reagent background and the inner absorbance value of test compound.

**Table 1. IC₅₀ value in AGA*IA assay, versus heparanase inhibition.**

| Compound | Code lab | AGAIA assay IC₅₀ |
|---|---|---|
| *Reference compound* | Roneparstat (SST0001) | ~ 5 nM |
| *Reference compound* | Suramin | ~ 26 µM |
| 12a | SST0884AA1 | + |
| 12b | SST0755AA1 | +++ |
| 12c | SST0900TF1 | + |
| 12d | SST0883AA1 | + |
| 12e | SST0881AA1 | +++ |
| 12f | SST0882AA1 | + |
| 12g | SST0898AA1 | + |
| 12h | SST0754AA1 | + |
| 12j | SST0756AA1 | + |
| 13b | SST0898NA1 | + |
| 13c | SST0899NA1 | +++ |
| 17a | SST0936AA1 | + |
| 17d | SST0886AA1 | + |
| 17e | SST0837AA1 | ++ |
| 17f | SST0885AA1 | + |
| 17h | SST0760AA1 | ++ |
| 18 | SST0820NA1 | ++ |
| 35c | SST0902AA1 | + |
| 36a | SST0831AA1 | + |
| 36b | SST0907AA1 | + |
| 36c | SST0834AA1 | + |
| 36d | SST0830AA1 | + |
| 36e | SST0832AA1 | +++ |
| 36f | SST0833AA1 | + |
| 41 | SST0905AA1 | + |
| 42 | SST0838AA1 | + |
| 43 | SST0828AA1 | + |
| 46 | SST0906AA1 | + |
| 47 | SST0829AA1 | + |

| | | |
|---|---|---|
| Legend: Range activity (IC₅₀) with AGA*IA test on Fondaparinux: +++: 0.05-0.49 µM ++ ; 0.50-0.99 µM +: 1.00-20.00 µM | | |

Results from this first screening on AGA*IA showed that compounds of the present invention are endowed with anti-heparanase activity. Some of them are active in the micromolar range (more potent than suramin), others are active in a submicromolar range. Some of them are very powerful (SST0755AA1, SST0881AA1, SST0899NA1, SST0832AA1), with an anti-heparanase activity less than 0.5 µM. Given the difference of M.W. with heparin-derivatives macromolecules currently under clinical investigation, it is noteworthy that these compounds are potent as Roneparstat, here used as reference compound. Roneparstat is taken as reference compound since it is a potent inhibitor of heparanase currently undergoing clinical trials.

Upon screening as inhibition of heparanase enzymatic activity, selected active compounds were furtherly characterized *in vitro,* through a suitable cell proliferation assay (SRB Cell Cytotoxicity Assay) for their putative cytotoxic activity against three tumor cell lines, and through specific cellular assay for their putative anti-metastatic activity. According to these data selected compounds were also tested for the effects on the expression of proangiogenic factors by real-time quantitative PCR.

### EXAMPLE 43

### Cytotoxicity assays

### Materials and methods

SRB Cell Cytotoxicity Assay is an assay based upon the quantitative staining of cellular proteins by sulforhodamine B (SRB). This assay can be used for high-throughput drug screening (Vichai & Kirkitara, Nature Protocol. 2006, 1, 112). Sulforhodamine B is an anionic aminoxanthene dye that forms an electrostatic complex with the basic amino acid residues of proteins under moderately acid conditions, which provides a sensitive linear response.

To assess a putative antiproliferative activity of the selected compounds, HT1080 (human fibrosarcoma), U87MG (human glioblastoma astrocytoma) and U2OS (human osteosarcoma) cell lines were treated for 72h, in triplicate, with increasing concentrations of heparanase inhibitors. Inhibition of cell proliferation was then measured by the classical colorimetric SRB Cell Cytotoxicity assay. Absorbance was measured by spectrophotometer at a wavelength of 510 nm and cell proliferation was determined as percent with respect to control cells (cells treated with the vehicle alone). EC₅₀ values were then determined by GraphPad Prism.

### Results

Results are summarized in the following Table 2.

**Table 2. Putative antiproliferative activity of the selected compounds on three tumor cell lines.**

| | | | Concentration (µM) of test compounds at which the cell growth is inhibited by 50 %. | | |
|---|---|---|---|---|---|
| | | AGA*IA assay | HT1080 | U87MG | U2OS |
| *Reference* | SST0001 | ~5 nM | > 10 | > 10 | > 10 |
| *Reference* | Suramin | ~ 26 µM | > 10 | > 10 | > 10 |
| 12b | SST0755AA1 | +++ | > 2.5 | > 2.5 | > 2.5 |
| 12e | SST0881AA1 | +++ | > 2.5 | > 2.5 | > 2.5 |
| 13c | SST0899NA1 | +++ | > 2.5 | > 2.5 | > 2.5 |
| 17e | SST0837AA1 | ++ | > 2.5 | > 2.5 | > 2.5 |
| 17h | SST0760AA1 | ++ | > 2.5 | > 2.5 | > 2.5 |
| 18 | SST0820NA1 | ++ | > 2.5 | > 2.5 | > 2.5 |
| 36e | SST0832AA1 | +++ | > 2.5 | > 2.5 | > 2.5 |

All selected active compounds do not affect cell proliferation (on three tumor cell lines) up to the tested concentration (2.5 µM). These data are very important because they show that compounds of the present invention, at concentrations in which they do not have any interference with cell proliferation, are selective heparanase inhibitors. This makes their use particularly advantageous in conditions wherein an anti-heparanase activity is desired but a cytotoxic effect is undesirable.

### EXAMPLE 44

### The invasion Assays

Heparanase inhibitors were then evaluated for their activity on the invasive potential of HT1080 (human fibrosarcoma), U87MG (human glioblastoma astrocytoma) and U2OS (human osteosarcoma) human cell lines, using the Matrigel cell invasion assay which allows to assess the cell invasion ability of malignant and normal cells.

### Materials and methods

Invasion assay was performed using BioCoat cell culture inserts (BioCoat 8.0µm PET Membrane 24 Well Permeable Supports, Corning). Filters were coated with 100 µl of 300 µg/ml Matrigel dissolved in coating buffer (0.01 M Tris pH 8.0, 0.7% NaCl) (Becton-Dickinson Sciences). DMEM (Dulbecco's Modified Eagle's Medium) containing 5% FBS was added to the lower chambers. Cells were pre-treated with heparanase inhibitors for 24 h, detached and added to the upper chambers in media containing the inhibitors. Cells were allowed to invade for 24 h at 37°C in a CO₂ incubator and then unmigrated cells were removed from the upper chamber with a cotton swab. The invading cells were fixed, stained with 0.1% crystal violet for 10 min at room temperature, photographed and counted under inverted microscope.

Test compounds were analyzed at fixed concentration (2.5 µM) for several fields, in duplicates. Activity of test compounds was compared to the reference compound. Activity of most interesting compound was confirmed in a second cell invasion assay. Data were expressed as the percent invasion with respect to cell migration through uncoated insert membrane.

Results are shown in Table 3 below.

### EXAMPLE 45

### Cell Adhesion Assays

Cell surface, non-integrin molecules such as heparan sulfate proteoglycans (HSPGs) may be involved in the regulation of early adhesive stages of cell-ECM interactions. A cell adhesion assay has been employed to determine if the newly designed heparanase inhibitors were able to interfere with adhesion properties of cancer cells.

### Materials and methods

HT1080 (human fibrosarcoma) and U87MG (human glioblastoma astrocytoma) cells were treated for 18-20 h with test compounds in complete medium supplemented with 10% FCS. Then cells were collected, washed in complete medium, and added to matrigel coated wells in triplicate, at previously identified optimal seeding density, and incubated at 37 °C in complete medium for times ranging from 15 minutes to 4h, according to the cell line. After incubation, the wells were washed three times with serum-free medium and the remaining firmly attached cells were stained with crystal-violet 0.1%. Optical density was measured at 560 nm by Victor 3 (Perkin-Elmer) plate analyzer. Each experiment was repeated three times.

### Results

Results are shown in the following Table 3.

**Table 3. Test compounds were analyzed at fixed concentration (2.5 µM) versus Reference compound (Suramin) at 10 µM. In Invasion assay, data were expressed as the percent invasion (range) with respect to cell migration through uncoated insert membrane. In Adhesion assay, data were expressed as the percent of adhesion with respect to the control (100%).**

| **Cpd** | **Code lab** | **Conc.** | **Invasion Assay Inhibition** | | | **Adhesion Assay (%)** | |
|---|---|---|---|---|---|---|---|
| | | | HT1080 | U87MG | U2OS | HT1080 | U87MG |
| Ref. | Suramin | 10 µM | + | - | - | 100 | 72.98 |
| 12b | SST0755AA1 | 2.5 µM | + | + | - | 97.1 | 16.7 |
| 13c | SST0899NA1 | 2.5 µM | + | + | ++ | 88.2 | 22.0 |
| 17e | SST0837AA1 | 2.5 µM | + | + | - | 93.2 | 51.6 |
| 18 | SST0820NA1 | 2.5 µM | ++ | + | + | 86.4 | 62.9 |
| 36e | SST0832AA1 | 2.5 µM | ++ | ++ | + | 91.2 | 73.0 |

| | | | | | | | |
|---|---|---|---|---|---|---|---|
| Legend: ++: 50-90% + : < 50% - : Not Active | | | | | | | |

Invasion is related to the heparanase activity unlike adhesion that is not directly related to the enzyme inhibition so, it should be considered as an adjuvant effect of the test compounds. Five compounds were selected, all with submicromolar IC₅₀ as heparanase inhibitor. They were tested in invasion and adhesion assays. On two cell lines, HT1080 and U87MG compounds were more active in inhibition of invasion; less potent on U2OS. In adhesion assay, the tumor cell line U87MG was more sensible against four on five compounds; less sensible was the other cell line, HT1080.

### EXAMPLE 46

### Real-Time Quantitative PCR (qPCR) for measuring mRNA gene-expression of FGF1/2, VEGF, MMP-9, HSPE-1 in genes tumor cell lines

### Materials and methods

To evaluate the effect of heparanase inhibitors on the tumor transcription of genes encoding for proangiogenic factors, such as FGF1/2, VEGF, MMP-9, HSPE-1, the relative mRNA levels were analyzed by quantitative Real-Time Quantitative PCR.

To perform the assay, HT1080 (human fibrosarcoma) cells were cultured in complete medium containing 10%FCS (fetal calf serum). Cells were plated in 24-well plates and treated at fixed concentration (i.e., highest non-toxic concentration) of test compounds. After 24 h, cells were detached, collected and total RNA extracted by RNeasy Mini Kit (QIAGEN). Following reverse transcription step, FGF1/2, VEGF, MMP-9, HSPE-1 mRNA levels, were quantified by qPCR using the advanced Universal SYBR Green Supermix (Bio Rad) and ABI PRISM 7900HT Thermal Cycler System (Perkin Elmer), according to the manufacturer's instructions. Appropriate no-RT and non-template controls were included in each 96-well PCR reaction, and dissociation analysis was performed at the end of each run to confirm the specificity of the reaction. Relative levels of RNA were calculated using the ddCt method. Standard curve and sample data were run in duplicate. In Table 4 a mean value is reported.

### Results

Results are reported in the following table 4.

**Table 4. Real-Time RT-PCR of pro-angiogenic genes**

| | | Gene Expression (% mRNA vs. control) | | | | |
|---|---|---|---|---|---|---|
| | Conc | FGF-1 | FGF-2 | VEGF | MMP-9 | HPSE-1 |
| Control | | ~ 100 | ~ 100 | ~ 100 | ~ 100 | ~ 100 |
| SST0832AA1 | 2.5 uM | ~ 62 | ~ 64 | ~ 51 | ~ 50 | ~ 88 |
| SST0899AA1 | 2.5 uM | ~ 53 | ~ 82 | ~ 55 | ~ 50 | ~ 63 |
| SST0001 | 10.0 uM | ~ 70 | ~ 77 | ~ 43 | ~ 42 | ~ 70 |

Data from qPCR assay, with two selected active compounds versus SST0001 (reference compound) on HT1080 (human fibrosarcoma) cells, highlighted an inhibitory effect against the tumor transcription genes encoding for all proangiogenic factors tested.

SST0832AA1 and SST0899AA1, the two selected compounds, were tested at a concentration (2.5 µM) highlighting a significant inhibition of genes expression encoding for proangiogenic factors. This effect was comparable to SST0001, used as reference compound and tested at 10 µM.

## Claims

1. A compound having the following formula (I) 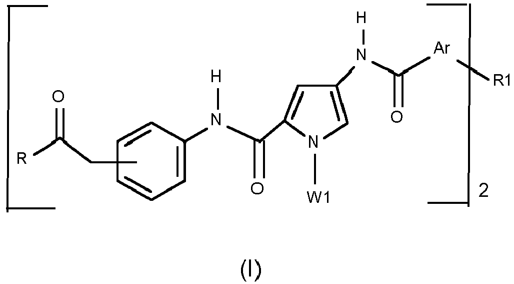
wherein R₁ is selected from the group consisting of NHCONH, O and NHCSNH;
Ar is selected from 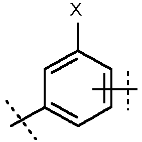 wherein X is selected from the group consisting of H, halogen and C₁-C₄alkyl, linear or branched, optionally substituted with one or more halogens,
and 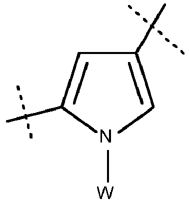 wherein W is selected from the group consisting of H, C₁-C₄ alkyl, linear or branched, optionally substituted with one or more halogens, and cyclopropyl;
W1 is selected from the group consisting of H, C₁-C₄ alkyl, linear or branched, optionally substituted with one or more halogens, and cyclopropyl,
and R is selected from the group consisting of OH, one or more amino acids, NHCH[B(OH)₂]C₁-C₄alkyl, linear or branched, and amino acid-NHCH[B(OH)₂]C₁-C₄alkyl, linear or branched,
the enantiomers, diastereoisomers, and mixtures thereof,
or a pharmaceutically acceptable salt, hydrate or solvate thereof.

2. The compound according to claim 1 wherein R is an amino acid, preferably selected from the group consisting of glycine (Gly) and phenylalanine (Phe).

3. The compound according to anyone of claims 1-2, wherein the group 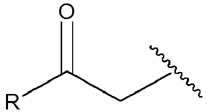 is in position 3 (meta) or in position 4 (para) of the phenyl ring.

4. The compound according to anyone of claims 1-3 wherein X is selected from the group consisting of H, F and CF₃.

5. The compound according to anyone of claims 1-4, wherein R₁ is NHCONH.

6. The compound according to claim 5 wherein Ar is 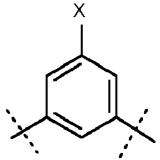 and X and R have the meanings defined in the previous claims.

7. The compound according to claim 6 wherein X is selected from H and CF₃.

8. The compound according to claim 6 or 7 wherein R is an amino acid, preferably selected from glycine and phenylalanine.

9. The compound according to claim 5 wherein Ar is 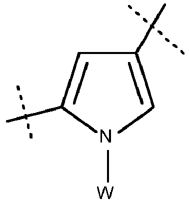 and W and R have the meanings defined in the previous claims.

10. The compound according to claim 9, wherein W is CH₃.

11. The compound according to claim 9 or 10 wherein R is selected from OH and an amino acid.

12. The compound according to claim 11 wherein R is an amino acid selected from glycine and phenylalanine.

13. The compound according to anyone of claims 1-4 wherein R₁ is O and Ar is 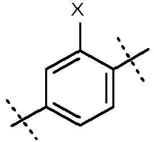 wherein X and R have the meanings defined in the previous claims.

14. The compound according to claim 13 wherein X is H.

15. The compound according to claim 13 or 14 wherein R is an amino acid, preferably it is phenylalanine.

16. The compound according to anyone of the preceding claims which is in the form of a sodium salt.

17. The compound according to anyone of the preceding claims, wherein said compound is selected from the group consisting of:
(2S,2'S)-2,2'-((2,2'-(((4,4'-((4,4'-Oxybis(benzoyl))bis(azanediyl))bis(1-methyl-1 H-pyrrole-2,2'-carbonyl))bis(azanediyl))bis(3,1-phenylene))bis(acetyl))bis(azanediyl))bis(3-phenylpropanoic acid),
disodium 2,2'-{carbonylbis[imino[5-(trifluoromethyl)-3,1-phenylene]carbonylimino(1-methyl-1 H-pyrrole-4,2-diyl)carbonylimino-3,1-phenylene(1-oxoethane-2,1-diyl)imino]}diethanoate salt,
disodium 2,2'-{carbonylbis[imino-3,1-phenylenecarbonylimino(1-methyl-1*H*-pyrrole-4,2-diyl)carbonylimino-3,1-phenylene(1-oxoethane-2,1-diyl)imino]}diethanoate salt, 2,2'-((2,2'-(((4,4'-((3,3'-(Carbonylbis(azanediyl))bis(benzoyl))bis(azanediyl))bis(1-methyl-1*H*-pyrrole-4,2-diyl-2-carbonyl))bis(azanediyl))bis(3,1-phenylene))bis(acetyl))bis(azanediyl))diacetic acid,
2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}bis(3-phenylpropanoic acid),
2,2'-{carbonylbis[imino(1-methyl-1H-pyrrole-4,2-diyl)carbonylimino(1-methyl-1H-pyrrole-4,2-diyl)carbonylimino-3,1-phenylene]}diacetic acid,
disodium 2,2'-{carbonylbis[imino(1-methyl-1H-pyrrole-4,2-diyl)carbonylimino(1-methyl-1H-pyrrole-4,2-diyl)carbonylimino-3,1-phenylene(1-oxoethane-2,1-diyl)imino]}diethanoate salt, and
2,2'-{carbonylbis[imino(1-methyl-1H-pyrrole-4,2-diyl)carbonylimino(1-methyl-1H-pyrrole-4,2-diyl)carbonylimino-3,1-phenylene(1-oxoethane-2,1-diyl)imino]}bis(3-phenylpropanoic acid).

18. The compound according to anyone of claims 1-16, wherein said compound of formula (I) is one of the followings:
2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}bis(4-methylpentanoic acid),
5,5'-{carbonylbis[imino-3,1-phenylenecarbonylimino(1-methyl-1H-pyrrole-4,2-diyl)carbonylimino-3,1-phenylene(1-oxoethane-2,1-diyl)imino]}bis(1-aminium-6-hexanoic acid) trifluoroacetate salt,
2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}dipropanoic acid, 2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}dipentandioic acid,
Disodium 2,2'-{carbonylbis[imino-3,1-phenylenecarbonylimino(1-methyl-1H-pyrrole-4,2-diyl)carbonylimino-3,1-phenylene(1-oxoethane-2,1-diyl)imino(1-oxoethane-2,1-diyl)imino]}diethanoate salt,
2,2'-{carbonylbis[imino-3,1-phenylenecarbonylimino(1-methyl-1H-pyrrole-4,2-diyl)carbonylimino-4,1-phenylene]}diacetic acid,
2,2'-{carbonylbis[imino(1-methyl-1H-pyrrole-4,2-diyl)carbonylimino(1-methyl-1H-pyrrole-4,2-diyl)carbonylimino-3,1-phenylene(1-oxoethane-2,1-diyl)imino]}bis(4-methylpentanoic acid),
2,2'-{carbonylbis[imino(1-methyl-1H-pyrrole-4,2-diyl)carbonylimino(1-methyl-1H-pyrrole-4,2-diyl)carbonylimino-3,1-phenylene(1-oxoethane-2,1-diyl)imino]}dipropanoic acid,
2,2'-{carbonylbis[imino-3,1-phenylenecarbonylimino(1-methyl-1H-pyrrole-4,2-diyl)carbonylimino-3,1-phenylene(1-oxoethane-2,1-diyl)imino]}dipentandioic acid, 2,2'-(((4,4'-((3,3'-(Carbonylbis(azanediyl))bis(benzoyl))bis(azanediyl))bis(1-methyl-1H-pyrrole-4,2-diyl-2-carbonyl))bis(azanediyl))bis(3,1-phenylene))diacetic acid, (2S,2'S)-2,2'-((2,2'-(((4,4'-((4,4'-(Carbonylbis(azanediyl))bis(1-methyl-1H-pyrrole-4,2-diyl-2-carbonyl))bis(azanediyl))bis(1-methyl-1H-pyrrole-4,2-diyl-2-carbonyl))bis(azanediyl))bis(3,1-phenylene))bis(acetyl))bis(azanediyl))bis(3-phenylpropanoic acid),
(2S,2'S)-2,2'-((2,2'-(((4,4'-((4,4'-(Carbonylbis(azanediyl))bis(1-methyl-1H-pyrrole-4,2-diyl-2-carbonyl))bis(azanediyl))bis(1-methyl-1H-pyrrole-4,2-diyl-2-carbonyl))bis(azanediyl))bis(3,1-phenylene))bis(acetyl))bis(azanediyl))bis(5-(tert-butoxy)-5-oxopentanoic acid),
(2S,2'S)-2,2'-((2,2'-(((4,4'-((4,4'-(carbonylbis(azanediyl))bis(1-methyl-1H-pyrrole-4,2-diyl-2-carbonyl))bis(azanediyl))bis(1-methyl-1H-pyrrole-4,2-diyl-2-carbonyl))bis(azanediyl))bis(3,1-phenylene))bis(acetyl))bis(azanediyl))bis(6-aminohexanoic acid) dihydrochloride,
2,2'-((2,2'-(((4,4'-((4,4'-Oxybis(benzoyl))bis(azanediyl))bis(1-methyl-1H-pyrrole-2,2'-carbonyl))bis(azanediyl))bis(3,1-phenylene))bis(acetyl))bis(azanediyl))diacetic acid, (2S,2'S)-2,2'-((2,2'-(((4,4'-((4,4'-Oxybis(benzoyl))bis(azanediyl))bis(1-methyl-1H-pyrrole-2,2'-carbonyl))bis(azanediyl))bis(3,1-phenylene))bis(acetyl))bis(azanediyl))dipropanoic acid,
(2S,2'S)-2,2'-((2,2'-(((4,4'-((4,4'-Oxybis(benzoyl))bis(azanediyl))bis(1-methyl-1 H-pyrrole-2,2'-carbonyl))bis(azanediyl))bis(3,1-phenylene))bis(acetyl))bis(azanediyl))bis(4-methylpentanoic acid), (2S,2'S)-2,2'-((2,2'-(((4,4'-((4,4'-Oxybis(benzoyl))bis(azanediyl))bis(1-methyl-1H-pyrrole-2,2'-carbonyl))bis(azanediyl))bis(3,1-phenylene))bis(acetyl))bis(azanediyl))dipentanedioic acid,
(2S,2'S)-2,2'-((2,2'-(((4,4'-((4,4'-oxybis(benzoyl))bis(azanediyl))bis(1-methyl-1H-pyrrole-4,2-diyl-2-carbonyl))bis(azanediyl))bis(3,1-phenylene))bis(acetyl))bis(azanediyl))bis(6-aminohexanoic acid) dihydrochloride, ((1R,1'R)-((2,2'-(((4,4'-((4,4'-(carbonylbis(azanediyl))bis(1-methyl-1H-pyrrole-4,2-diyl-2-carbonyl))bis(azanediyl))bis(1-methyl-1H-pyrrole-4,2-diyl-2-carbonyl))bis(azanediyl))bis(3,1-phenylene))bis(acetyl))bis(azanediyl))bis(3-methylbutane-1,1-diyl))diboronic acid,
((1R,1'R)-((2,2'-(((4,4'-((4,4'-Oxybis(benzoyl))bis(azanediyl))bis(1-methyl-1H-pyrrole-2,2'-carbonyl))bis(azanediyl))bis(3,1-phenylene))bis(acetyl))bis(azanediyl))bis(3-methylbutane-1,1-diyl))diboronic acid,
((1R,1'R)-((2,2'-(((4,4'-((3,3-(Carbonylbis(azanediyl))bis(benzoyl))bis(azanediyl))bis(1-methyl-1H-pyrrole-4,2-diyl-2-carbonyl))bis(azanediyl))bis(3,1-phenylene))bis(acetyl))bis(azanediyl))bis(3-methylbutane-1,1-diyl))diboronic acid,
((1R,1'R)-((2,2'-((2,2'-(((4,4'-((4,4'-(carbonylbis(azanediyl))bis(1-methyl-1H-pyrrole-4,2-diyl-2-carbonyl))bis(azanediyl))bis(1-methyl-1H-pyrrole-4,2-diyl-2-carbonyl))bis(azanediyl))bis(3,1-phenylene))bis(acetyl))bis(azanediyl))bis(acetyl))bis(azanediyl))bis(3-methylbutane-1,1-diyl))diboronic acid,
((1R,1'R)-((2,2'-((2,2'-(((4,4'-((3,3-(Carbonylbis(azanediyl))bis(benzoyl))bis(azanediyl))bis(1-methyl-1H-pyrrole-4,2-diyl-2-carbonyl))bis(azanediyl))bis(3,1-phenylene))bis(acetyl))bis(azanediyl))bis(acetyl))bis(azanediyl))bis(3-methylbutane-1,1-diyl))diboronic acid,
2,2'-((2,2'-(((4,4'-((4,4'-oxybis(benzoyl))bis(azanediyl))bis(1-methyl-1H-pyrrole-2,2'-carbonyl))bis(azanediyl))bis(3,1-phenylene))bis(acetyl))bis(azanediyl))diacetic acid and
2,2'-((2,2'-(((4,4'-((3,3'-(thiocarbonylbis(azanediyl))bis(benzoyl))bis(azanediyl))bis(1-methyl-1 H-pyrrole-2,2'-carbonyl))bis(azanediyl))bis(3,1-phenylene))bis(acetyl))bis(azanediyl))diacetic acid.

19. The compound of anyone of claims 1-18 for use as a medicament.

20. The compound of anyone of claims 1-18 for use in the treatment of a disease which can be improved or prevented by an anti-heparanase activity.

21. The compound for the use according to claim 20 wherein said disease is selected from the group consisting of tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis and aging.

22. A pharmaceutical composition containing as active ingredient the compound of anyone of claims 1-18 and at least one pharmaceutically acceptable vehicle and/or excipient.

23. The pharmaceutical composition according to claim 22 wherein said composition further comprises one or more further active ingredients.

24. The pharmaceutical composition according to claim 23 wherein said further active ingredient is a chemotherapeutic agent.

25. The pharmaceutical composition according to claim 24, wherein said chemotherapeutic agent is selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens.

26. The pharmaceutical composition according to claim 25 wherein said chemotherapeutic agent is a cytotoxic agent selected from the group consisting of melphalan, irinotecan, rituximab and lapatinib; or is a proteasome inhibitor selected from the group consisting of bortezomib, carfilzomib and ixazomib; or is an immunomodulatory agent selected from the group consisting of thalidomide, lenalidomide and pomalidomide.
